# EUROPÄISCHE PATENTSCHRIFT

(11) **EP 2 569 312 B1**
(45) Veröffentlichungstag und Bekanntmachung des Hinweises auf die Patenterteilung: **26.03.2014**
(21) Anmeldenummer: 11716595.1
(22) Anmeldetag: 03.05.2011
(51) Int. Cl.: C07D 413/14, A61K 31/4245, A61P 35/00

(54) **SUBSTITUIERTE HETEROCYCLYLBENZYL-PYRAZOLE UND IHRE VERWENDUNG**
SUBSTITUTED HETEROCYCLYL BENZYL PYRAZOLES, AND USE THEREOF
HÉTÉROCYCLYLBENZYLE-PYRAZOLES SUBSTITUÉES ET LEUR UTILISATION

(30) Priorität: 08.05.2010 EP 10004855
(43) Veröffentlichungstag der Anmeldung: 20.03.2013
(73) Patentinhaber: Bayer Intellectual Property GmbH, 40789 Monheim am Rhein (DE)
(72) Erfinder: HÄRTER, Michael, 51375 Leverkusen (DE); BECK, Hartmut, 42287 Wuppertal (DE); GRESCHAT-SCHADE, Susanne, 49419 Wagenfeld (DE); ELLINGHAUS, Peter, 49324 Melle (DE); UNTERSCHEMMANN, Kerstin, 45239 Essen (DE); SCHUHMACHER, Joachim, 42113 Wuppertal (DE)
(74) Vertreter: BIP Patents
(86) Internationale Anmeldenummer: PCT/EP2011/057021
(87) Internationale Veröffentlichungsnummer: WO 2011/141326

(56) Entgegenhaltungen:
- WO-A2-2008/141731

## Beschreibung

Die vorliegende Anmeldung betrifft neue, substituierte 1-[3-(Heterocyclyl)benzyl]-*1*H-Pyrazol-Derivate, Verfahren zu ihrer Herstellung, ihre Verwendung zur Herstellung von Arzneimitteln zur Behandlung und/ oder Prävention von Krankheiten, insbesondere zur Behandlung und/oder Prävention von hyperproliferativen und angiogenen Erkrankungen sowie solcher Erkrankungen, die durch eine metabolische Adaptation an hypoxische Zustände entstehen. Solche Behandlungen können als Monotherapie oder auch in Kombination mit anderen Arzneimitteln oder weiteren therapeutischen Maßnahmen erfolgen.

Krebserkrankungen sind die Folge unkontrollierten Zellwachstums verschiedenster Gewebe. In vielen Fällen dringen die neuen Zellen in bestehende Gewebe ein (invasives Wachstum), oder sie metastasieren in entfernte Organe. Krebserkrankungen treten in verschiedensten Organen auf und haben oft gewebespezifische Krankheitsverläufe. Daher beschreibt die Bezeichnung Krebserkrankung als Oberbegriff eine große Gruppe definierter Erkrankungen verschiedener Organe, Gewebe und Zelltypen.

Im Jahr 2002 wurden weltweit 4,4 Millionen Menschen mit Tumorerkrankungen der Brust, des Darms, der Eierstöcke, der Lunge oder der Prostata diagnostiziert. Für das gleiche Jahr wurden ca. 2,5 Millionen Todesfälle als Folge dieser Erkrankungen angenommen (Globocan 2002 Report). In den USA allein wurden für das Jahr 2005 über 1,25 Millionen neue Fälle und über 500.000 Todesfälle aufgrund von Krebserkrankungen prognostiziert. Die Mehrzahl dieser neuen Fälle betrifft Krebserkrankungen von Darm (∼ 100.000), Lunge (∼ 170.000), Brust (∼ 210.000) und Prostata (∼ 230.000). Es wird von einer weiteren Zunahme der Krebserkrankungen von ca. 15% über die nächsten 10 Jahre ausgegangen (American Cancer Society, Cancer Facts and Figures 2005).

Tumore früher Stadien lassen sich gegebenenfalls durch chirurgische und radiotherapeutische Maßnahmen entfernen. Metastasierte Tumore können im Regelfall durch Chemotherapeutika nur palliativ therapiert werden. Ziel hierbei ist, die optimale Kombination aus einer Verbesserung der Lebensqualität und der Verlängerung der Lebenszeit zu erreichen.

Chemotherapien setzen sich häufig aus Kombinationen von zytotoxischen Arzneimitteln zusammen. Die Mehrheit dieser Substanzen haben als Wirkmechanismus eine Bindung an Tubulin, oder es handelt sich um Verbindungen, die mit der Bildung und Prozessierung von Nukleinsäuren interagieren. In neuerer Zeit zählen dazu auch Enzym-Inhibitoren, die mit der epigenetischen DNA-Modifikation oder der Zellzyklusprogression interferieren (z.B. Histon-Deacetylase-Inhibitoren, Aurora-Kinase-Inhibitoren). Da solche Therapien toxisch sind, setzt man in neuerer Zeit vermehrt auf gezielte Therapien, bei denen spezielle Prozesse in der Zelle blockiert werden, ohne dass eine hohe toxische Belastung erfolgt. Dazu zählen insbesondere Inhibitoren von Kinasen, welche die Phosphorylierung von Rezeptoren und Signalübertragungsmolekülen hemmen. Ein Beispiel hierfür ist Imatinib, das sehr erfolgreich zur Behandlung von chronisch-myeloischer Leukämie (CML) und gastrointestinalen stromalen Tumoren (GIST) eingesetzt wird. Weitere Beispiele sind EGFR-Kinase- und HER2-blockierende Substanzen wie Erlotinib sowie VEGFR-Kinase-Inhibitoren wie Sorafenib und Sunitinib, welche bei Nierenzellkarzinomen, Leberkarzinomen bzw. fortgeschrittenen Stadien von GIST eingesetzt werden.

Mit einem gegen VEGF gerichteten Antikörper ist es gelungen, die Lebenserwartung von Kolorektalkarzinom-Patienten zu verlängern. Bevacizumab hemmt das Blutgefäßwachstum, was der schnellen Ausdehnung eines Tumors im Wege steht, da dieser für eine kontinuierlich funktionierende Ver- und Entsorgung einen Anschluß an das Blutgefäßsystem benötigt.

Ein Stimulus für die Angiogenese ist die Hypoxie, welche bei soliden Tumoren immer wieder auftritt, da die Blutversorgung aufgrund des ungeregelten Wachstums unzureichend ist. Bei Sauerstoffarmut stellen die Zellen ihren Stoffwechsel von der oxidativen Phosphorylierung auf die Glykolyse um, damit der ATP-Spiegel in der Zelle stabilisiert wird. Dieser Prozess wird durch einen Transkriptionsfaktor gesteuert, der abhängig vom Sauerstoffgehalt in der Zelle hochreguliert wird. Dieser "Hypoxie-induzierter Faktor" (HIF) genannte Transkriptionsfaktor wird normalerweise posttranslational durch einen schnellen Abbau entfernt und am Transport in den Zellkern gehindert. Dies geschieht durch die Hydroxylierung zweier Prolin-Einheiten in der sauerstoffabbaubaren Domäne (ODD) und einer Asparagin-Einheit in der Nähe des C-Terminus durch die Enzyme Prolyl-Dehydrogenase und FIH ("factor inhibiting HIF"). Nach der Modifikation der Prolin-Einheiten kann HIF vermittels des Hippel-Lindau-Proteins (Teil eines Ubiquitin-E3-Ligase-Komplexes) über den Proteasomenapparat abgebaut werden (Maxwell, Wiesener *et al.,* 1999). Bei Sauerstoffmangel unterbleibt der Abbau, das Protein wird hochreguliert und führt zur Transkription bzw. zur Blockade der Transkription zahlreicher (mehr als 100) anderer Proteine (Semenza und Wang, 1992; Wang und Semenza, 1995).

Der Transkriptionsfaktor HIF wird durch die regulierte α- und eine konstitutiv vorhandene β-Untereinheit (ARNT, aryl hydrocarbon receptor nuclear translocator) gebildet. Von der α-Untereinheit gibt es drei verschiedene Spezies 1α, 2α und 3α, wobei die letzte eher als Suppressor anzunehmen ist (Makino, Cao *et al.,* 2001). Bei den HIF-Untereinheiten handelt es sich um bHLH (basic helix loop helix)-Proteine, die über ihre HLH- und PAS (Per-Arnt-Sim)-Domäne dimerisieren, was ihre Transaktivierungsaktivität startet (Jiang, Rue *et al.,* 1996).

In den wichtigsten Tumorentitäten wird die Überexpression des HIF1α-Proteins mit zunehmender Blutgefäßdichte und verstärkter VEGF-Expression korreliert (Hirota und Semenza, 2006). Gleichzeitig wird der Glukosestoffwechsel hin zur Glykolyse verändert, und der Krebs-Zyklus wird zugunsten der Produktion von Zellbausteinen reduziert. Dies impliziert auch eine Änderung des Fettstoffwechsels. Solche Änderungen scheinen das Überleben der Tumore zu gewährleisten. Wird nun andererseits die Aktivität von HIF gehemmt, so könnte man folglich die Entwicklung von Tumoren unterdrücken. Dies wurde bereits in verschiedenen experimentellen Modellen beobachtet (Chen, Zhao *et al.,* 2003; Stoeltzing, McCarty *et al.,* 2004; Li, Lin *et al.,* 2005; Mizukami, Jo *et al.,* 2005; Li, Shi *et al.,* 2006). Spezifische Inhibitoren des von HIF gesteuerten Metabolismus sollten sich daher als Tumortherapeutika eignen.

In WO 2004/089303-A2 sind di-Aryl-substituierte Pyrazole als mGluR5-Modulatoren zur Behandlung psychiatrischer Erkrankungen beschrieben. In WO 2010/072352-A1 und WO 2010/085584-A1 werden 3-Phenyl-5-(1*H*-pyrazol-4-yl)-1,2,4-oxadiazol-Derivate als Sphingosin-1-phosphat-Agonisten zur Therapie von Autoimmun- und Gefäßerkrankungen offenbart.

In WO 2005/030121-A2 und WO 2007/065010-A2 wird die Verwendbarkeit bestimmter Pyrazol-Derivate zur Inhibition der Expression von HIF und HIF-regulierten Genen in Tumorzellen beschrieben. In WO 2008/141731-A2 werden Heteroaryl-substituierte *N*-Benzylpyrazole als Inhibitoren des HIF-Regulationsweges zur Behandlung von Krebserkrankungen offenbart. Allerdings zeigte es sich, dass viele dieser Verbindungen eine nicht hinreichende inhibitorische Aktivität aufweisen oder aber aufgrund ihrer pharmakokinetischen Eigenschaften in Tiermodellen eine so lange Halbwertszeit (>48 h) im menschlichen Körper erwarten lassen, dass eine signifikante Substanz-Akkumulation nach wiederholter einmal täglicher Gabe wahrscheinlich ist.

Es war daher Aufgabe der vorliegenden Erfindung, neue Verbindungen aufzufinden und bereitzustellen, welche einerseits als potente Inhibitoren der transaktivierenden Wirkung des Transkriptionsfaktors HIF agieren und andererseits ein pharmakokinetisches Profil aufweisen, das die wiederholte einmal tägliche Gabe ohne gleichzeitiges Auftreten einer klinisch relevanten Akkumulation erlaubt. Solche Eigenschaften könnten insgesamt auch zu einer Verbreiterung der klinischen Anwendbarkeit dieser HIF-Inhibitoren führen und insbesondere ihre Kombinierbarkeit mit anderen Wirkstoffen, wie beispielsweise klassischen Tumor-Chemotherapeutika, erleichtern.

Diese Aufgabe wird durch die im Folgenden beschriebenen erfindungsgemäßen Verbindungen gelöst. Strukturell zeichnet sich diese neue Gruppe von *N*-Benzylpyrazol-Derivaten durch einen Hydroxy- oder Cyano-substituierten Heterocyclyl-Rest in 3-Position der Benzyl-Kopfgruppe aus, welcher überraschenderweise zu einem verbesserten Eigenschaftsprofil der Verbindungen führt.

Im Einzelnen betrifft die vorliegende Erfindung Verbindungen der allgemeinen Formel (I) 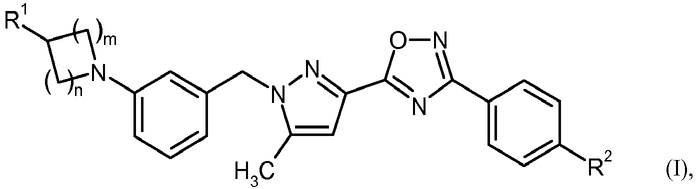 in welcher
- m: für die Zahl 1 oder 2 steht,
- n: für die Zahl 1, 2 oder 3 steht,
- R¹: für Hydroxy oder Cyano steht,
und
- R²: für Trifluormethoxy, Trifluormethylsulfanyl, Trifluormethylsulfonyl, Pentafluorsulfanyl oder eine Gruppe der Formel 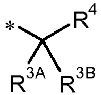 steht, worin
* die Verknüpfungsstelle mit dem Phenyl-Ring kennzeichnet,
R^{3A} und R^{3B} unabhängig voneinander Fluor oder Methyl bedeuten
oder
miteinander verknüpft sind und gemeinsam mit dem Kohlenstoffatom, an das sie gebunden sind, einen Cyclopropan-1,1-diyl-, Cyclobutan-1,1-diyl-, Cyclopentan-1,1-diyl-, Cyclohexan-1,1-diyl-, Oxetan-3,3-diyl- oder Tetrahydro-2*H*-pyran-4,4-diyl-Ring bilden, und
R⁴ Wasserstoff, Fluor, Methyl, Trifluormethyl oder Methoxy bedeutet,
sowie ihre Salze, Solvate und Solvate der Salze.

Bevorzugt im Rahmen der vorliegenden Erfindung sind Verbindungen der Formel (I), in welcher
- m und n: unabhängig voneinander für die Zahl 1 oder 2 stehen,
- R¹: für Hydroxy oder Cyano steht,
und
- R²: für Trifluormethyl, Trifluormethoxy, Trifluormethylsulfanyl oder eine Gruppe der Formel 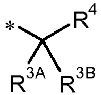 steht, worin
* die Verknüpfungsstelle mit dem Phenyl-Ring kennzeichnet,
R^{3A} und R^{3B} beide Methyl bedeuten oder miteinander verknüpft sind und gemeinsam mit dem Kohlenstoffatom, an das sie gebunden sind, einen Cyclopropan-1,1-diyl-, Cyclo-butan-1,1-diyl-, Oxetan-3,3-diyl- oder Tetrahydro-2*H-*pyran-4,4-diyl-Ring bilden, und
R⁴ Wasserstoff, Fluor, Methyl oder Trifluormethyl bedeutet,
sowie ihre Salze, Solvate und Solvate der Salze.

Besonders bevorzugt im Rahmen der vorliegenden Erfindung sind Verbindungen der Formel (I), in welcher
- m und n: beide für die Zahl 1 oder die Zahl 2 stehen,
- R¹: für Hydroxy oder Cyano steht,
und
- R²: für Trifluormethoxy oder eine Gruppe der Formel 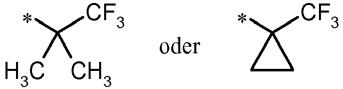 steht, worin
* die Verknüpfungsstelle mit dem Phenyl-Ring kennzeichnet,
sowie ihre Salze, Solvate und Solvate der Salze.

Eine besondere Ausführungsform der vorliegenden Erfindung betrifft Verbindungen der Formel (I), in welcher
- R¹: für Hydroxy steht
und
- m, n und R²: jeweils die oben angegebenen Bedeutungen haben,
sowie ihre Salze, Solvate und Solvate der Salze.

Diese zuletzt genannte Ausführungsform entspricht Verbindungen der Formel (I-A) 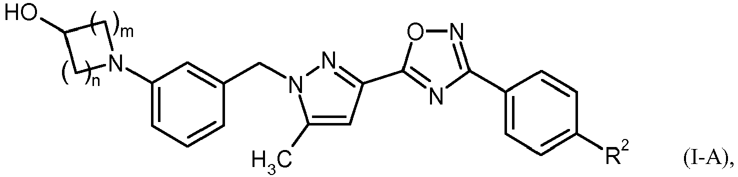 in welcher m, n und R² jeweils die oben angegebenen Bedeutungen haben,
und ihren Salzen, Solvaten und Solvaten der Salze.

In einem weiteren Aspekt umfasst die vorliegende Erfindung auch bestimmte Prodrugs von Verbindungen der Formel (I-A). Allgemein bezeichnet der Begriff "Prodrugs" hierbei kovalente Derivate der Verbindungen der Formel (I-A), welche selbst biologisch aktiv oder inaktiv sein können, jedoch während ihrer Verweilzeit im Körper auf beispielsweise metabolischem oder hydrolytischem Wege zu Verbindungen der Formel (I-A) umgesetzt werden.

Weiterer Gegenstand der vorliegenden Erfindung sind demgemäß Verbindungen der Formel (I-PD) 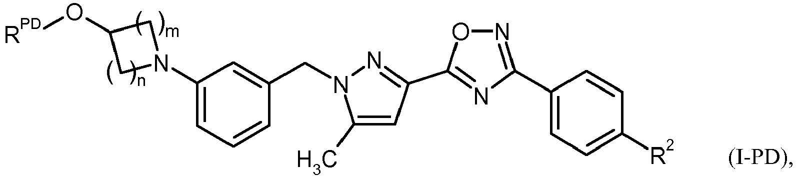 in welcher m, n und R² jeweils die oben aufgeführten Bedeutungen haben
und
- R^{PD}: für eine Prodrug-Gruppe der Formel 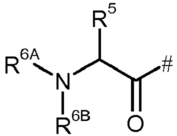 steht, worin
# die Verknüpfungsstelle mit dem Sauerstoffatom kennzeichnet,
R⁵ Wasserstoff oder (C₁-C₄)-Alkyl bedeutet, und
R^{6A} und R^{6B} unabhängig voneinander Wasserstoff oder Methyl bedeuten,
sowie ihre Salze, Solvate und Solvate der Salze.

Bevorzugt im Rahmen der vorliegenden Erfindung sind Verbindungen der Formel (I-PD), in welcher
- R^{PD}: für eine Prodrug-Gruppe der Formel 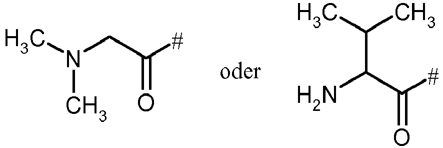 steht, worin
# die Verknüpfungsstelle mit dem Sauerstoffatom kennzeichnet,
sowie ihre Salze, Solvate und Solvate der Salze.

Die Verbindungen der Formel (I-PD) stellen Prodrugs der Verbindungen der Formel (I-A) mit einer guten Löslichkeit in wässrigen oder anderen physiologisch verträglichen Medien dar; sie bieten zudem die Möglichkeit zur Salzbildung mit entsprechenden Säuren, was zu einer weiteren Steigerung der Löslichkeit führen kann. Die Verbindungen der Formel (I-PD) und ihre Salze eignen sich daher insbesondere für intravenöse Anwendungsformen oder auch für Feststoff-Formulierungen mit modifiziertem Freisetzungsverhalten. Hierdurch könnten auch zusätzliche therapeutische Einsatzgebiete für diese Verbindungen erschlossen werden.

Erfindungsgemäße Verbindungen sind somit die Verbindungen der Formeln (I), (I-A) und (I-PD) sowie deren Salze, Solvate und Solvate der Salze, die von den Formeln (I), (I-A) und (I-PD) umfassten Verbindungen der nachfolgend genannten Formeln und deren Salze, Solvate und Solvate der Salze sowie die von den Formeln (I), (I-A) und (I-PD) umfassten, nachfolgend als Ausführungsbeispiele genannten Verbindungen und deren Salze, Solvate und Solvate der Salze, soweit es sich bei den von den Formeln (I), (I-A) und (I-PD) umfassten, nachfolgend genannten Verbindungen nicht bereits um Salze, Solvate und Solvate der Salze handelt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomeren und Diastereomeren und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise zu einer Verlängerung der Halbwertszeit im Körper oder zu einer Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach allgemein gebräuchlichen, dem Fachmann bekannten Verfahren hergestellt werden, so beispielsweise nach den weiter unten beschriebenen Methoden und den bei den Ausführungsbeispielen wiedergegebenen Vorschriften, indem hierbei entsprechende isotopische Modifikationen der jeweiligen Reagentien und/oder Ausgangsverbindungen eingesetzt werden.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung, Reinigung oder Lagerung der erfindungsgemäßen Verbindungen verwendet werden können.

Physiologisch unbedenkliche Salze der erfindungsgemäßen Verbindungen umfassen insbesondere die Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:
(C₁-C₄)-Alkyl steht im Rahmen der Erfindung für einen geradkettigen oder verzweigten Alkylrest mit 1 bis 4 Kohlenstoffatomen. Beispielhaft und vorzugsweise seien genannt: Methyl, Ethyl, *n*-Propyl, Isopropyl, *n*-Butyl, Isobutyl, sec.-Butyl, *tert.*-Butyl*.*

Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder mit zwei gleichen oder verschiedenen Substituenten ist bevorzugt. Besonders bevorzugt ist die Substitution mit einem Substituenten.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der vorliegenden Erfindung ist ein Verfahren zur Herstellung der Verbindungen der Formel (I), dadurch gekennzeichnet, dass man eine Verbindung der Formel (II) 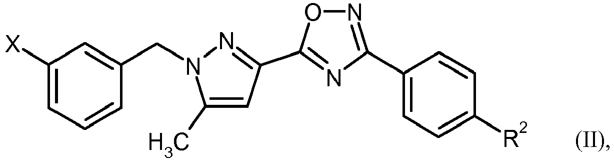 in welcher R² die oben angegebene Bedeutung hat
und
- X: für Brom oder Iod steht,
in Gegenwart eines geeigneten Palladium-Katalysators und einer Base mit einer Verbindung der Formel (III) 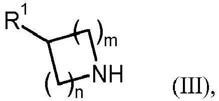 in welcher m, n und R¹ die oben angegebenen Bedeutungen haben,
kuppelt
und die so erhaltenen Verbindungen der Formel (I) gegebenenfalls in ihre Enantiomere und/oder Diastereomere trennt und/oder mit den entsprechenden (i) Lösungsmitteln und/oder (ii) Säuren in ihre Solvate, Salze und/oder Solvate der Salze überführt.

Als inerte Lösungsmittel für die Umsetzung (II) + (III) → (I) eignen sich beispielsweise aromatische Kohlenwasserstoffe wie Benzol, Toluol oder Xylol, Ether wie Diethylether, Diisopropylether, Methyl-*tert*.-butylether, 1,2-Dimethoxyethan, Bis-(2-methoxyethyl)-ether, Tetrahydrofuran oder 1,4-Dioxan, oder dipolar-aprotische Lösungsmittel wie Acetonitril, *N,N*-Dimethylformamid (DMF), *N*,*N-*Dimethylacetamid (DMA), Dimethylsulfoxid (DMSO), *N*,*N'*-Dimethylpropylenharnstoff (DMPU), *N*-Methylpyrrolidinon (NMP) oder Pyridin. Ebenso ist es möglich, Gemische dieser Lösungsmittel einzusetzen. Bevorzugt wird 1,2-Dimethoxyethan, Tetrahydrofuran, *N*,*N*-Dimethylformamid oder Toluol verwendet.

Die Kupplungsreaktion (II) + (III) → (I) wird mit Hilfe eines Übergangsmetall-Katalysators ausgeführt. Hierfür sind insbesondere Palladium(0)-Katalysatoren wie beispielsweise Bis(dibenzyliden-aceton)palladium(0), Tris(dibenzylidenaceton)dipalladium(0) oder Tetrakis(triphenylphosphino)-palladium(0) geeignet, gegebenenfalls in Kombination mit zusätzlichen Phosphanliganden wie 2-Dicyclohexylphosphino-2'-(*N*,*N-*dimethylamino)biphenyl, 2-Dicyclohexylphosphino-2',4',6'-triisopropylbiphenyl (X-Phos) oder 4,5-Bis(diphenylphosphino)-9,9-dimethylxanthen (Xantphos) [vgl. z.B. J. Hassan et al., Chem. Rev. 102, 1359-1469 (2002)]. Bevorzugt wird Tris(dibenzylidenaceton)-dipalladium(0) in Verbindung mit 2-Dicyclohexylphosphino-2',4',6'-triisopropylbiphenyl (X-Phos) eingesetzt.

Die Kupplung wird in der Regel unter Zusatz einer Base durchgeführt. Hierfür eignen sich insbesondere Alkalicarbonate, -hydrogencarbonate, -phosphate, -hydrogenphosphate oder *tert*.-butylate wie Natriumcarbonat, Kaliumcarbonat, Cäsiumcarbonat, Natriumhydrogencarbonat, Kaliumhydrogencarbonat, Trikaliumphosphat, Dinatriumhydrogenphosphat, Dikaliumhydrogenphosphat, Natrium-*tert*.-butylat oder Kalium-*tert*.-butylat. Bevorzugt wird Cäsiumcarbonat oder Natrium-tert.-butylat verwendet.

Die Reaktion (II) + (III) → (I) erfolgt im Allgemeinen bei Normaldruck in einem Temperaturbereich von +50°C bis +200°C, bevorzugt bei +100°C bis +150°C. Eine Durchführung bei vermindertem oder bei erhöhtem Druck (z.B. von 0.5 bis 5 bar) ist jedoch auch möglich. Gegebenenfalls ist es hilfreich, die Umsetzung unter gleichzeitiger Mikrowellen-Bestrahlung vorzunehmen.

Im Fall, dass der Rest R¹ für Hydroxy steht, kann es bei der oben beschriebenen Kupplungsreaktion (II) + (III) → (I) gegebenenfalls von Vorteil sein, diese Hydroxy-Gruppe temporär geschützt zu halten. Hierzu werden vorzugsweise Silylether-Derivate der Verbindung (III) eingesetzt, wie beispielsweise entsprechende Trimethylsilyl-, Triisopropylsilyl-, tert.-Butyldimethylsilyl- oder *tert.-*Butyl(diphenyl)silyl-Ether, welche aus den Verbindungen (III) nach bekannten Verfahren leicht zugänglich sind. Nach erfolgter Kupplung können diese Schutzgruppen dann nach üblichen Methoden, wie beispielsweise durch Behandlung mit Tetra-*n*-butylammoniumfluorid, wieder abgespalten werden.

Die Verbindungen der Formel (II) ihrerseits können dadurch hergestellt werden, dass man zunächst ein *N*'-Hydroxyamidin der Formel (IV) 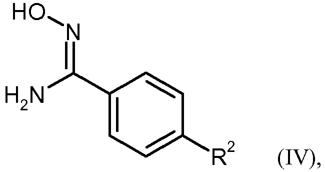 in welcher R² die oben angegebene Bedeutung hat,
mit einer Pyrazolcarbonsäure der Formel (V) 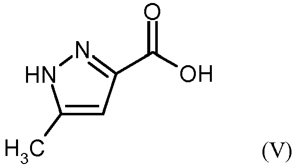 zu einem 1,2,4-Oxadiazol-Derivat der Formel (VI) 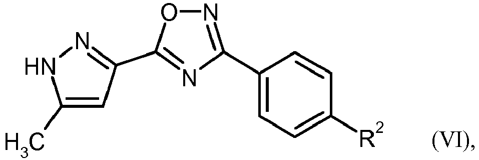 in welcher R² die oben angegebene Bedeutung hat,
kondensiert und die Verbindung (VI) dann in Gegenwart einer Base mit einer Verbindung der Formel (VII) 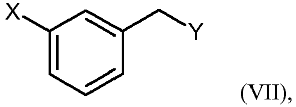 in welcher X die oben angegebene Bedeutung hat
und
- Y: für eine Abgangsgruppe wie beispielsweise Chlor, Brom, Iod, Mesylat, Triflat oder Tosylat steht,
umsetzt.

Die Kondensationsreaktion (IV) + (V) → (VI) wird bevorzugt mit Hilfe eines Carbodiimids wie *N*'-(3-Dimethylaminopropyl)-*N*-ethylcarbodiimid (EDC), in Verbindung mit 1-Hydroxy-1*H*-benzotriazol (HOBt) als Aktivester-Komponente, oder mit Hilfe eines Phosgen-Derivats wie 1,1'-Carbonyldiimidazol (CDI) in einem hochsiedenden dipolar-aprotischen Lösungsmittel wie beispielsweise *N,N*-Dimethylformamid oder Dimethylsulfoxid durchgeführt.

Der initiale Kupplungsschritt bei dieser Reaktion erfolgt im Allgemeinen in einem Temperaturbereich von 0°C bis +50°C; die Cyclisierung zum 1,2,4-Oxadiazol wird dann durch anschließendes Erhitzen der Reaktionsmischung auf Temperaturen von +100°C bis +150°C bewerkstelligt. Die Umsetzung kann bei normalem, bei erhöhtem oder bei erniedrigtem Druck durchgeführt werden (z.B. von 0.5 bis 5 bar); in der Regel arbeitet man bei Normaldruck.

Inerte Lösungsmittel für den Verfahrensschritt (VI) + (VII) → (II) sind beispielsweise Halogenkohlenwasserstoffe wie Dichlormethan, Trichlormethan, Tetrachlormethan, Trichlorethylen oder Chlorbenzol, Ether wie Diethylether, Diisopropylether, Methyl-*tert*.-butylether, Tetrahydrofuran, 1,4-Dioxan, 1,2-Dimethoxyethan oder Bis-(2-methoxyethyl)-ether, Kohlenwasserstoffe wie Benzol, Toluol, Xylol, Pentan, Hexan, Cyclohexan oder Erdölfraktionen, oder dipolar-aprotische Lösungsmittel wie Aceton, Methylethylketon, Ethylacetat, Acetonitril, *N,N*-Dimethylformamid (DMF), *N,N-*Dimethylacetamid (DMA), Dimethylsulfoxid (DMSO), *N,N'*-Dimethylpropylenharnstoff (DMPU), *N*-Methylpyrrolidinon (NMP) oder Pyridin. Ebenso ist es möglich, Gemische der genannten Lösungsmittel einzusetzen. Bevorzugt wird Tetrahydrofuran oder 1,4-Dioxan verwendet.

Als Base für die Umsetzung (VI) + (VII) → (II) eignen sich übliche anorganische oder organische Basen. Hierzu gehören bevorzugt Alkalihydroxide wie beispielsweise Lithium-, Natrium- oder Kaliumhydroxid, Alkali-Alkoholate wie Natrium- oder Kaliummethanolat, Natrium- oder Kaliumethanolat oder Natrium- oder Kalium-*tert*.-butylat, Alkalihydride wie Natrium- oder Kaliumhydrid, oder Amide wie Natriumamid, Lithium- oder Kalium-bis(trimethylsilyl)amid oder Lithiumdiisopropylamid. Bevorzugt wird Kalium-*tert*.-butylat eingesetzt. Gegebenenfalls ist der Zusatz eines Alkylierungskatalysators, wie beispielsweise Lithiumbromid, Natriumiodid, Tetra-*n*-butylammoniumbromid oder Benzyltriethylammoniumchlorid, von Vorteil.

Die Reaktion erfolgt im Allgemeinen in einem Temperaturbereich von -20°C bis +100°C, bevorzugt bei 0°C bis +60°C. Die Umsetzung kann bei normalem, bei erhöhtem oder bei erniedrigtem Druck durchgeführt werden (z.B. von 0.5 bis 5 bar); in der Regel arbeitet man bei Normaldruck.

Weiterer Gegenstand der vorliegenden Erfindung ist ein Verfahren zur Herstellung der Verbindungen der Formel (I-PD), dadurch gekennzeichnet, dass man eine Verbindung der Formel (I-A) 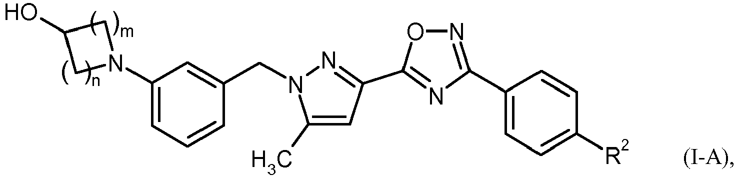 in welcher m, n und R² jeweils die oben angegebenen Bedeutungen haben,
nach üblichen Methoden mit einer Verbindung der Formel (VIII)

R^{PD}-OH (VIII)

oder einer aktivierten Form dieser Verbindung, in welcher R^{PD} die oben angegebene Bedeutung hat,
verestert
und die so erhaltenen Verbindungen der Formel (I-PD) gegebenenfalls in ihre Enantiomere und/ oder Diastereomere trennt und/oder mit den entsprechenden (i) Lösungsmitteln und/oder (ii) Säuren in ihre Solvate, Salze und/oder Solvate der Salze überführt.

Aktivierte Formen der Verbindung (VIII), welche sich für die Einführung der Prodrug-Gruppe R^{PD} eignen, sind beispielsweise entsprechende Chloride oder Anhydride, einschließlich gemischter Anhydride, oder auch bestimmte Ester- oder Amid-Derivate. Eine im Rest R^{PD} vorhandene freie AminoGruppe liegt hierbei zweckmäßigerweise in temporär geschützter Form vor und wird dann am Ende der Veresterungsreaktion nach geläufigen Methoden wieder freigesetzt. Als ein solcher Schutz wird vorzugsweise die *tert*.-Butoxycarbonyl-Gruppe eingesetzt, die durch Behandlung mit einer starken Säure, wie Chlorwasserstoff oder Trifluoressigsäure, abgespalten werden kann [vgl. z.B. M. Bodanszky und A. Bodanszky, The Practice of Peptide Synthesis, Springer-Verlag, Berlin, 1984; M. Bodanszky, Principles of Peptide Synthesis, Springer-Verlag, Berlin, 1993; T.W. Greene und P.G.M. Wuts, Protective Groups in Organic Synthesis, Wiley, New York, 1999].

Die Verbindungen der Formel (I-A) selbst sind über die zuvor beschriebene Kupplungsreaktion (II) + (III) → (I) zugänglich.

Die Verbindungen der Formeln (III), (IV), (V), (VII) und (VIII) sind kommerziell erhältlich oder als solche in der Literatur beschrieben, oder sie können auf für den Fachmann offenkundigem Wege in Analogie zu in der Literatur publizierten Methoden hergestellt werden. Zahlreiche detaillierte Vorschriften sowie Literaturangaben zur Herstellung der Ausgangsmaterialien befinden sich auch im Experimentellen Teil im Abschnitt zur Herstellung der Ausgangsverbindungen und Intermediate.

Die Herstellung der erfindungsgemäßen Verbindungen kann durch die folgenden Reaktionsschemata beispielhaft veranschaulicht werden:

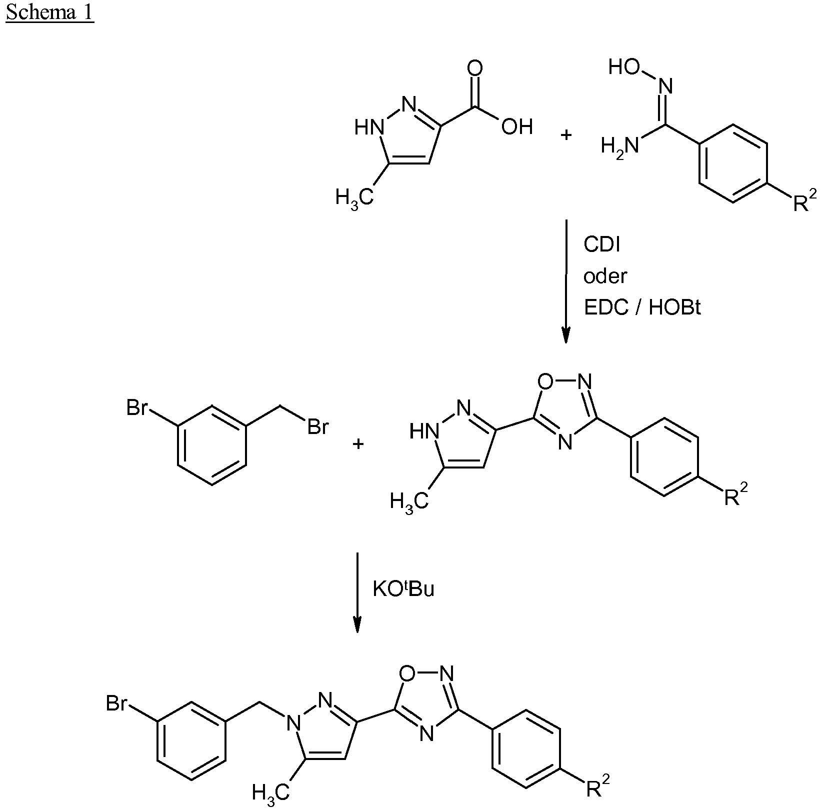

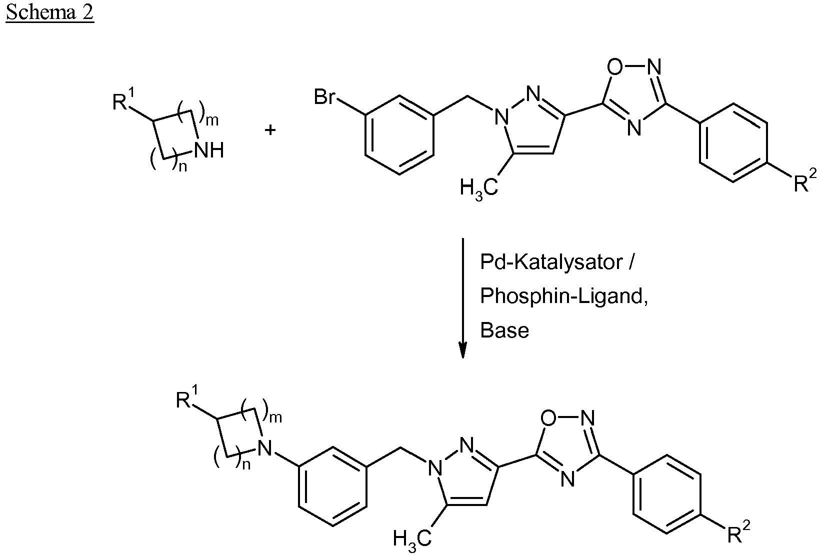

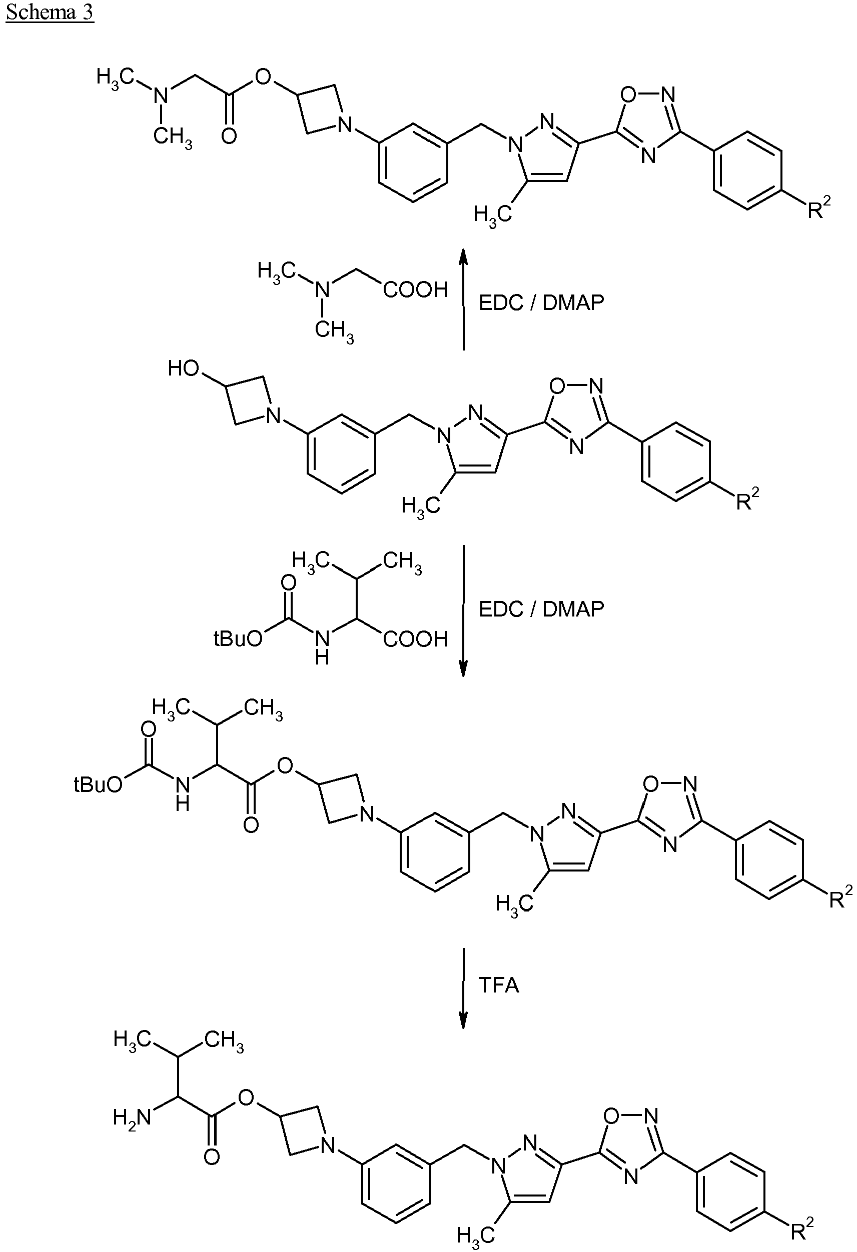

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Verbindungen stellen hochpotente Inhibitoren des HIF-Regulationsweges dar. Darüber hinaus weisen die erfindungsgemäßen Verbindungen vorteilhafte pharmakokinetische Eigenschaften bezüglich ihres Verteilungsvolumens und/oder ihrer Clearance und der daraus abgeleiteten Halbwertszeit auf, was sie für eine wiederholte einmal tägliche Gabe geeignet macht.

Die erfindungsgemäßen Verbindungen eignen sich aufgrund ihres Wirkprofils insbesondere zur Behandlung von hyperproliferativen Erkrankungen beim Menschen und bei Säugetieren allgemein. Die Verbindungen können die Zellproliferation und Zellteilung hemmen, blockieren, verringern oder senken und andererseits die Apoptose verstärken.

Zu den hyperproliferativen Erkrankungen, zu deren Behandlung die erfindungsgemäßen Verbindungen eingesetzt werden können, zählen unter anderem Psoriasis, Keloide, Narbenbildungen und andere proliferative Erkrankungen der Haut, benigne Erkrankungen wie die benigne Prostatahyperplasie (BPH), sowie insbesondere die Gruppe der Tumorerkrankungen. Hierunter werden im Rahmen der vorliegenden Erfindung insbesondere die folgenden Erkrankungen verstanden, ohne jedoch auf sie beschränkt zu sein: Brustkarzinome und Brusttumore (ductale und lobuläre Formen, auch *in situ*), Atemwegstumore (kleinzelliges und nicht-kleinzelliges Karzinom, Bronchialkarzinom), Hirntumore (z.B. des Hirnstamms und des Hypothalamus, Astrocytoma, Medulloblastoma, Ependymoma sowie neuro-ectodermale und pineale Tumore), Tumore der Verdauungsorgane (Speiseröhre, Magen, Gallenblase, Dünndarm, Dickdarm, Rektum), Lebertumore (u.a. hepatozelluläres Karzinom, Cholangiokarzinom und gemischt-hepatozelluläres Cholangiokarzinom), Tumore des Kopf- und Halsbereiches (Larynx, Hypopharynx, Nasopharynx, Oropharynx, Lippen und Mundhöhle), Hauttumore (Plattenepithelkarzinom, Kaposi-Sarkom, malignes Melanom, Merkelzell-Hautkrebs und nicht-melanomartiger Hautkrebs), Tumore der Weichteile (u.a. Weichteilsarkome, Osteosarkome, maligne fibröse Histiozytome, Lymphosarkome und Rhabdomyosarkome), Tumore der Augen (u.a. intraokuläres Melanom und Retinoblastom), Tumore der endokrinen und exokrinen Drüsen (z.B. thyroide und parathyroide Drüsen, Bauchspeicheldrüse und Speicheldrüse), Tumore des Harntrakts (Blasen-, Penis-, Nieren-, Nierenbecken- und Harnleitertumore) sowie Tumore der reproduktiven Organe (Endometrium-, Zervix-, Ovarial-, Vaginal-, Vulva- und Uteruskarzinome der Frau sowie Prostata- und Hodenkarzinome des Mannes). Dazu gehören auch proliferative Bluterkrankungen in solider Form und als zirkulierende Blutzellen, wie Lymphome, Leukämien und myeloproliferative Erkrankungen, z.B. akute myeloide, akute lymphoblastische, chronisch-lymphozytische, chronischmyelogene und Haarzell-Leukämie, sowie AIDS-korrelierte Lymphome, Hodgkin-Lymphome, Non-Hodgkin-Lymphome, kutane T-Zell-Lymphome, Burkitt-Lymphome und Lymphome im zentralen Nervensystem.

Diese gut beschriebenen Krankheiten des Menschen können mit vergleichbarer Ätiologie auch in anderen Säugetieren vorkommen und dort mit den Verbindungen der vorliegenden Erfindung behandelt werden.

Der Begriff "Behandlung" oder "behandeln" wird im Rahmen dieser Erfindung konventionell verwendet und bedeutet die Versorgung, Pflege und Betreuung eines Patienten mit dem Ziel, eine Krankheit oder gesundheitliche Abweichung zu bekämpfen, zu verringern, abzuschwächen oder zu erleichtern und die Lebensbedingungen zu verbessern, die durch diese Krankheit beeinträchtigt werden, wie beispielsweise bei einer Krebserkrankung.

Die erfindungsgemäßen Verbindungen wirken als Modulatoren des HIF-Regulationsweges und eignen sich daher auch zur Behandlung von Erkrankungen, welche mit einer schädlichen Expression des HIF-Transkriptionsfaktors assoziiert sind. Dies betrifft insbesondere die Transkriptionsfaktoren HIF-1α und HIF-2a. Der Begriff "schädliche Expression von HIF" bedeutet hierbei ein nicht-normalphysiologisches Vorhandensein von HIF-Protein. Dies kann bedingt sein durch übermäßige Synthese des Proteins (mRNA- oder translationsbedingt), durch verringerten Abbau oder durch unzureichende Gegenregulation bei der Funktion des Transkriptionsfaktors.

HIF-1α und HIF-2α regulieren mehr als 100 Gene. Dies betrifft Proteine, die bei der Angiogenese eine Rolle spielen und daher direkt tumorrelevant sind, und auch solche, die den Glukose-, Aminosäure- und Lipid-Stoffwechsel sowie Zellmigration, Metastase und DNA-Reparatur beeinflussen oder durch Unterdrückung der Apoptose das Überleben der Tumorzellen verbessern. Andere wirken eher indirekt über die Hemmung der Immunreaktion und Hochregulierung von angiogenen Faktoren in Entzündungszellen. Eine wichtige Rolle spielt HIF auch bei den Stammzellen, hier insbesondere den Tumorstammzellen, von denen berichtet wird, dass sie erhöhte HIF-Spiegel aufweisen. Durch die Hemmung des HIF-Regulationsweges durch die Verbindungen der vorliegenden Erfindung werden damit auch Tumorstammzellen therapeutisch beeinflusst, die keine hohe Proliferationsrate aufweisen und daher von zytotoxischen Substanzen nur unzureichend betroffen sind (vgl. Semenza, 2007; Weidemann und Johnson, 2008).

Veränderungen des Zellmetabolismus durch HIF sind nicht exklusiv für Tumore, sondern treten auch bei anderen hypoxischen pathophysiologischen Prozessen auf, mögen sie chronisch oder transient sein. HIF-Inhibitoren - wie die Verbindungen der vorliegenden Erfindung - sind in solchen Zusammenhängen therapeutisch hilfreich, in denen beispielsweise durch eine Adaptation von Zellen an hypoxische Situationen zusätzlicher Schaden entsteht, da geschädigte Zellen, wenn sie nicht wie vorgesehen funktionieren, weitere Schäden hervorrufen können. Ein Beispiel hierfür ist die Bildung von epileptischen Herden in partiell zerstörtem Gewebe nach Schlaganfällen. Ähnliches findet man bei Herz-Kreislauf-Erkrankungen, wenn als Folge von thromboembolischen Ereignissen, Entzündungen, Verwundungen, Intoxikationen oder anderen Ursachen ischämische Prozesse im Herzen oder im Gehirn auftreten. Diese können zu Schäden führen wie einem lokal verlangsamten Aktionspotential, welches seinerseits Arrhythmien oder ein chronisches Herzversagen nach sich ziehen kann. In transienter Form, z.B. durch Apnoe, kann es unter Umständen zu einer essentiellen Blutdruckerhöhung kommen, was zu bekannten Folgeerkrankungen wie beispielsweise Schlaganfall und Herzinfarkt führen kann.

Die Hemmung des HIF-Regulationsweges, wie sie durch die erfindungsgemäßen Verbindungen erreicht wird, kann daher auch bei Erkrankungen wie Herzinsuffizienz, Arrhythmie, Herzinfarkt, Apnoe-induzierte Hypertonie, pulmonale Hypertonie, Transplantationsischämie, Reperfusionsschäden, Schlaganfall und Makuladegeneration sowie zur Wiedergewinnung der Nervenfunktion nach traumatischer Schädigung oder Durchtrennung hilfreich sein.

Da HIF einer der Faktoren ist, welche den Übergang von einem epithelialen zu einem mesenchymalen Zelltyp steuern, was im Speziellen für die Lunge und die Niere von Bedeutung ist, können die erfindungsgemäßen Verbindungen auch eingesetzt werden, um mit HIF assoziierte Fibrosen von Lunge und Niere zu verhindern oder einzudämmen.

Weitere Erkrankungen, zu deren Behandlung die erfindungsgemäßen Verbindungen verwendet werden können, sind entzündliche Gelenkerkrankungen, wie verschiedene Formen der Arthritis, sowie entzündliche Darmerkrankungen, wie beispielsweise Morbus Crohn.

Die Chugwash-Polyzythämie wird durch HIF-2α-Aktivität während der Erythropoese unter anderem in der Milz vermittelt. Die erfindungsgemäßen Verbindungen, als Hemmstoffe des HIF-Regulationsweges, sind daher auch geeignet, hier die exzessive Erythrozytenbildung zu unterdrücken und damit die Auswirkungen dieser Erkrankung zu mildern.

Die Verbindungen der vorliegenden Erfindung können ferner verwendet werden zur Behandlung von Erkrankungen, die mit exzessiver oder anormaler Angiogenese verbunden sind. Dazu gehören unter anderem diabetische Retinopathie, ischämische Retinalvenenocclusion und Retinopathie bei Frühgeburt (vgl. Aiello *et al.,* 1994; Peer *et al.,* 1995), altersabhängige Makuladegeneration (AMD; vgl. Lopez *et al.,* 1996), neovaskuläres Glaukom, Psoriasis, retrolentale Fibroplasie, Angiofibrom, Entzündung, rheumatische Arthritis (RA), Restenose, in-stent-Restenose sowie Restenose nach Gefäßimplantation.

Eine gesteigerte Blutversorgung ist außerdem mit kanzerösem, neoplastischem Gewebe assoziiert und führt hier zu einem beschleunigten Tumorwachstum. Zudem erleichtert das Wachstum neuer Blut- und Lymphgefäße die Bildung von Metastasen und damit die Verbreitung des Tumors. Neue Lymph- und Blutgefäße sind auch schädlich für Allografts in immunprivilegierten Geweben, wie dem Auge, was zum Beispiel die Anfälligkeit für Abstoßungsreaktionen erhöht. Verbindungen der vorliegenden Erfindung können daher auch eingesetzt werden, um eine der vorgenannten Erkrankungen zu therapieren, z.B. durch eine Hemmung des Wachstums oder eine Verringerung der Anzahl von Blutgefäßen. Dies kann über eine Hemmung der Endothelzellproliferation oder andere Mechanismen zur Verhinderung oder Abschwächung der Gefäßbildung und über eine Reduktion von neoplastischen Zellen durch Apoptose erreicht werden.

Bei Adipositas kommt es zu einer Anreicherung von HIF-1α im Fettgewebe und dadurch zu einer HIF-vermittelten Verschiebung des Energiestoffwechsels in Richtung Glykolyse, so dass vermehrt Glukose als Energieträger verbraucht wird. Dies führt gleichzeitig zu einem verringerten Fettstoff-wechsel und somit zu einer Einlagerung von Fetten im Gewebe. Die erfindungsgemäßen Substanzen eignen sich daher auch zur Behandlung der HIF-1α-vermittelten Anreicherung von Fetten im Gewebe speziell bei der Adipositas-Erkrankung.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen.

Beispielsweise können die Verbindungen der vorliegenden Erfindung mit bekannten anti-hyperproliferativen, zytostatischen oder zytotoxischen Substanzen zur Behandlung von Krebserkrankungen kombiniert werden. Die Kombination der erfindungsgemäßen Verbindungen mit anderen für die Krebstherapie gebräuchlichen Substanzen oder auch mit der Strahlentherapie ist deshalb besonders angezeigt, da hypoxische Regionen eines Tumors nur wenig auf die genannten konventionellen Therapien ansprechen, wohingegen die Verbindungen der vorliegenden Erfindung insbesondere dort ihre Aktivität entfalten.

Als geeignete Kombinationswirkstoffe seien beispielhaft genannt:
Aldesleukin, Alendronsäure, Alfaferon, Alitretinoin, Allopurinol, Aloprim, Aloxi, Altretamin, Aminoglutethimid, Amifostin, Amrubicin, Amsacrin, Anastrozol, Anzmet, Aranesp, Arglabin, Arsentrioxid, Aromasin, 5-Azacytidin, Azathioprin, BCG oder tice-BCG, Bestatin, Betamethason-Acetat, Betamethason-Natriumphosphat, Bexaroten, Bleomycin-Sulfat, Broxuridin, Bortezomib, Busulfan, Calcitonin, Campath, Capecitabin, Carboplatin, Casodex, Cefeson, Celmoleukin, Cerubidin, Chlorambucil, Cisplatin, Cladribin, Clodronsäure, Cyclophosphamid, Cytarabin, Dacarbazin, Dactinomycin, DaunoXome, Decadron, Decadron-Phosphat, Delestrogen, Denileukin Diftitox, Depomedrol, Deslorelin, Dexrazoxan, Diethylstilbestrol, Diflucan, Docetaxel, Doxifluridin, Doxorubicin, Dronabinol, DW-166HC, Eligard, Elitek, Ellence, Emend, Epirubicin, Epoetin-alfa, Epogen, Eptaplatin, Ergamisol, Estrace, Estradiol, Estramustin-Natriumphosphat, Ethinylestradiol, Ethyol, Etidronsäure, Etopophos, Etoposid, Fadrozol, Farston, Filgrastim, Finasterid, Fligrastim, Floxuridin, Fluconazol, Fludarabin, 5-Fluordeoxyuridin-Monophosphat, 5-Fluoruracil (5-FU), Fluoxymesteron, Flutamid, Formestan, Fosteabin, Fotemustin, Fulvestrant, Gammagard, Gemcitabin, Gemtuzumab, Gleevec, Gliadel, Goserelin, Granisetron-Hydrochlorid, Histrelin, Hycamtin, Hydrocorton, erythro-Hydroxynonyladenin, Hydroxyhamstoff, Ibritumomab Tiuxetan, Idarubicin, Ifosfamid, Interferon-alpha, Interferon-alpha-2, Interferon-alpha-2α, Interferon-alpha-2β, Interferon-alpha-n1, Interferon-alpha-n3, Interferon-beta, Interferon-gamma-1α, Interleukin-2, Intron A, Iressa, Irinotecan, Kytril, Lentinan-Sulfat, Letrozol, Leucovorin, Leuprolid, Leuprolid-Acetat, Levamisol, Levofolinsäure-Calciumsalz, Levothroid, Levoxyl, Lomustin, Lonidamin, Marinol, Mechlorethamin, Mecobalamin, Medroxyprogesteron-Acetat, Megestrol-Acetat, Melphalan, Menest, 6-Mercaptopurin, Mesna, Methotrexat, Metvix, Miltefosin, Minocyclin, Mitomycin C, Mitotan, Mitoxantron, Modrenal, Myocet, Nedaplatin, Neulasta, Neumega, Neupogen, Nilutamid, Nolvadex, NSC-631570, OCT-43, Octreotid, Ondansetron-Hydrochlorid, Orapred, Oxaliplatin, Paclitaxel, Pediapred, Pegaspargase, Pegasys, Pentostatin, Picibanil, Pilocarpin-Hydrochlorid, Pirarubicin, Plicamycin, Porfimer-Natrium, Prednimustin, Prednisolon, Prednison, Premarin, Procarbazin, Procrit, Raltitrexed, Rebif, Rhenium-186-Etidronat, Rituximab, Roferon-A, Romurtid, Salagen, Sandostatin, Sargramostim, Semustin, Sizofiran, Sobuzoxan, Solu-Medrol, Streptozocin, Strontium-89-chlorid, Synthroid, Tamoxifen, Tamsulosin, Tasonermin, Tastolacton, Taxoter, Teceleukin, Temozolomid, Teniposid, Testosteron-Propionat, Testred, Thioguanin, Thiotepa, Thyrotropin, Tiludronsäure, Topotecan, Toremifen, Tositumomab, Tastuzumab, Teosulfan, Tretinoin, Trexall, Trimethylmelamin, Trimetrexat, Triptorelin-Acetat, Triptorelin-Pamoat, UFT, Uridin, Valrubicin, Vesnarinon, Vinblastin, Vincristin, Vindesin, Vinorelbin, Virulizin, Zinecard, Zinostatin-Stimalamer, Zofran; ABI-007, Acolbifen, Actimmun, Affinitak, Aminopterin, Arzoxifen, Asoprisnil, Atamestan, Atrasentan, Avastin, CCI-779, CDC-501, Celebrex, Cetuximab, Crisnatol, Cyproteron-Acetat, Decitabin, DN-101, Doxorubicin-MTC, dSLIM, Dutasterid, Edotecarin, Eflornithin, Exatecan, Fenretinid, Histamin-Dihydrochlorid, Histrelin-Hydrogel-Implant, Holmium-166-DOTMP, Ibandronsäure, Interferon-gamma, Intron-PEG, Ixabepilon, Keyhole Limpet-Hemocyanin, L-651582, Lanreotid, Lasofoxifen, Libra, Lonafarnib, Miproxifen, Minodronat, MS-209, liposomales MTP-PE, MX-6, Nafarelin, Nemorubicin, Neovastat, Nolatrexed, Oblimersen, Onko-TCS, Osidem, Paclitaxel-Polyglutamat, Pamidronat-Dinatrium, PN-401, QS-21, Quazepam, R-1549, Raloxifen, Ranpirnas, Regorafenib, 13-*cis*-Retinsäure, Satraplatin, Seocalcitol, Sorafenib, T-138067, Tarceva, Taxoprexin, Thymosin-alpha-1, Tiazofurin, Tipifarnib, Tirapazamin, TLK-286, Toremifen, TransMID-107R, Valspodar, Vapreotid, Vatalanib, Verteporfin, Vinflunin, Z-100, Zoledronsäure, sowie Kombinationen hiervon.

In einer bevorzugten Ausführungsform können die Verbindungen der vorliegenden Erfindung mit anti-hyperproliferativen Agentien kombiniert werden, welche beispielhaft - ohne dass diese Aufzählung abschließend wäre - sein können:
Aminoglutethimid, L-Asparaginase, Azathioprin, 5-Azacytidin, Bleomycin, Busulfan, Camptothecin, Carboplatin, Carmustin, Chlorambucil, Cisplatin, Colaspase, Cyclophosphamid, Cytarabin, Dacarbazin, Dactinomycin, Daunorubicin, Diethylstilbestrol, 2',2'-Difluordeoxycytidin, Docetaxel, Doxorubicin (Adriamycin), Epirubicin, Epothilon und seine Derivate, erythro-Hydroxynonyladenin, Ethinylestradiol, Etoposid, Fludarabin-Phosphat, 5-Fluordeoxyuridin, 5-Fluordeoxyuridin-Monophosphat, 5-Fluoruracil, Fluoxymesteron, Flutamid, Hexamethylmelamin, Hydroxyharnstoff, Hydroxyprogesteron-Caproat, Idarubicin, Ifosfamid, Interferon, Irinotecan, Leucovorin, Lomustin, Mechlorethamin, Medroxyprogesteron-Acetat, Megestrol-Acetat, Melphalan, 6-Mercaptopurin, Mesna, Methotrexat, Mitomycin C, Mitotan, Mitoxantron, Paclitaxel, Pentostatin, *N*-Phosphono-acetyl-L-aspartat (PALA), Plicamycin, Prednisolon, Prednison, Procarbazin, Raloxifen, Semustin, Streptozocin, Tamoxifen, Teniposid, Testosteron-Propionat, Thioguanin, Thiotepa, Topotecan, Tri-methylmelamin, Uridin, Vinblastin, Vincristin, Vindesin und Vinorelbin.

In viel versprechender Weise lassen sich die erfindungsgemäßen Verbindungen auch mit biologischen Therapeutika wie Antikörpern (z.B. Avastin, Rituxan, Erbitux, Herceptin) und rekombinanten Proteinen kombinieren, welche additiv oder synergistisch die Effekte der Hemmung der HIF-Signalwegsübertragung verstärken.

Inhibitoren des HIF-Regulationsweges wie die erfindungsgemäßen Verbindungen können auch in Kombination mit anderen, gegen die Angiogenese gerichteten Therapien positive Effekte erzielen, wie zum Beispiel mit Avastin, Axitinib, Recentin, Regorafenib, Sorafenib oder Sunitinib. Kombinationen mit Inhibitoren des Proteasoms und von mTOR sowie Antihormone und steroidale metabolische Enzyminhibitoren sind wegen ihres günstigen Nebenwirkungsprofils besonders geeignet.

Generell können mit der Kombination von Verbindungen der vorliegenden Erfindung mit anderen, zytostatisch oder zytotoxisch wirksamen Agentien folgende Ziele verfolgt werden:
- eine verbesserte Wirksamkeit bei der Verlangsamung des Wachstums eines Tumors, bei der Reduktion seiner Größe oder sogar bei seiner völligen Eliminierung im Vergleich zu einer Behandlung mit einem einzelnen Wirkstoff;
- die Möglichkeit, die verwendeten Chemotherapeutika in geringerer Dosierung als bei der Monotherapie einzusetzen;
- die Möglichkeit einer verträglicheren Therapie mit weniger Nebeneffekten im Vergleich zur Einzelgabe;
- die Möglichkeit zur Behandlung eines breiteren Spektrums von Tumorerkrankungen;
- das Erreichen einer höheren Ansprechrate auf die Therapie;
- eine längere Überlebenszeit der Patienten im Vergleich zur heutigen Standardtherapie.

Darüber hinaus können die erfindungsgemäßen Verbindungen auch in Verbindung mit einer Strahlentherapie und/oder einer chirurgischen Intervention eingesetzt werden.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut
- Ac: Acetyl
- aq.: wässrig
- Bsp.: Beispiel
- Bu: Butyl
- ca.: *circa,* ungefähr
- CDI: 1,1'-Carbonyldiimidazol
- CI: chemische Ionisation (bei MS)
- d: Dublett (bei NMR)
- d: Tag(e)
- DAST: Diethylaminoschwefeltrifluorid
- DC: Dünnschichtchromatographie
- DCI: direkte chemische Ionisation (bei MS)
- dd: Dublett von Dublett (bei NMR)
- DMAP: 4-*N*,*N-*Dimethylaminopyridin
- DME: 1,2-Dimethoxyethan
- DMF: *N,N*-Dimethylformamid
- DMSO: Dimethylsulfoxid
- dt: Dublett von Triplett (bei NMR)
- d. Th.: der Theorie (bei chemischer Ausbeute)
- EDC: *N'*-(3-Dimethylaminopropyl)-*N*-ethylcarbodiimid-Hydrochlorid
- ee: Enantiomerenüberschuss
- EI: Elektronenstoß-Ionisation (bei MS)
- eq.: Äquivalent(e)
- ESI: Elektrospray-Ionisation (bei MS)
- Et: Ethyl
- GC: Gaschromatographie
- h: Stunde(n)
- HOBt: 1-Hydroxy-1*H*-benzotriazol-Hydrat
- HPLC: Hochdruck-, Hochleistungsflüssigchromatographie
- ⁱPr: Isopropyl
- LC-MS: Flüssigchromatographie-gekoppelte Massenspektrometrie
- Lit.: Literatur(stelle)
- m: Multiplett (bei NMR)
- Me: Methyl
- min: Minute(n)
- MPLC: Mitteldruckflüssigchromatographie (über Kieselgel; auch "flash-Chromatographie" genannt)
- Ms: Methansulfonyl (Mesyl)
- MS: Massenspektrometrie
- NMP: *N*-Methyl-2-pyrrolidinon
- NMR: Kernresonanzspektrometrie
- Pd/C: Palladium auf Aktivkohle
- PEG: Polyethylenglykol
- Pr: Propyl
- quart: Quartett (bei NMR)
- quint: Quintett (bei NMR)
- R_{f}: Retentionsindex (bei DC)
- RT: Raumtemperatur
- Rₜ: Retentionszeit (bei HPLC)
- s: Singulett (bei NMR)
- sept: Septett (bei NMR)
- t: Triplett (bei NMR)
- TBAF: Tetra-*n*-butylammoniumfluorid
- ^{t}Bu: *tert*.-Butyl
- Tf: Trifluormethylsulfonyl (Triflyl)
- TFA: Trifluoressigsäure
- THF: Tetrahydrofuran
- UV: Ultraviolett-Spektrometrie
- v/v: Volumen zu Volumen-Verhältnis (einer Lösung)
- zus.: zusammen

### HPLC-, LC/MS- und GC/MS-Methoden:

### Methode 1 (LC/MS):

Gerätetyp MS: Micromass ZQ; Gerätetyp HPLC: HP 1100 Series; UV DAD; Säule: Phenomenex Gemini 3µ 30 mm x 3.00 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 2 (LC/MS):

Instrument: Micromass Quattro Micro MS mit HPLC Agilent Serie 1100; Säule: Thermo Hypersil GOLD 3µ 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 3 (LC/MS):

Gerätetyp MS: Micromass ZQ; Gerätetyp HPLC: Waters Alliance 2795; Säule: Phenomenex Synergi 2.5µ MAX-RP 100A Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

### Methode 4 (LC/MS):

Instrument: Micromass Quattro Premier mit Waters UPLC Acquity; Säule: Thermo Hypersil GOLD 1.9µ 50 mm x 1 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 5 (LC/MS):

Instrument: Micromass Quattro LCZ mit HPLC Agilent Serie 1100; Säule: Phenomenex Synergi 2.5µ MAX-RP 100A Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 0.1 min 90% A → 3.0 min 5% A → 4.0 min 5% A → 4.1 min 90% A; Fluss: 2 ml/min; Ofen: 50°C; UV-Detektion: 208-400 nm.

### Methode 6 (LC/MS):

Instrument: Waters Acquity SQD UPLC System; Säule: Waters Acquity UPLC HSS T3 1.8µ 50 mm x 1 mm; Eluent A: 11 Wasser + 0.25 ml 99%-ige Ameisensäure, Eluent B: 11 Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 210-400 nm.

### Methode 7 (LC/MS):

Gerätetyp MS: Waters ZQ; Gerätetyp HPLC: Agilent 1100 Series; UV DAD; Säule: Thermo Hypersil GOLD 3µ 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 100% A → 3.0 min 10% A → 4.0 min 10% A → 4.1 min 100% A (Fluss 2.5 ml/min); Ofen: 55°C; Fluss: 2 ml/min; UV-Detektion: 210 nm.

### Methode 8 (LC/MS):

Gerätetyp MS: Micromass ZQ; Gerätetyp HPLC: HP 1100 Series; UV DAD; Säule: Phenomenex Gemini 3µ 30 mm x 3.00 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 9 (GC/MS):

Instrument: Micromass GCT, GC 6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Methode 10 (analytische HPLC):

Instrument: HP 1100 mit DAD-Detektion; Säule: Kromasil 100 RP-18, 60 mm x 2.1 mm, 3.5 µm; Eluent A: 5 ml Perchlorsäure (70%-ig) / 1 Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

### Methode 11 (präparative HPLC):

Säule: Grom-Sil 120 ODS-4 HE, 10 µm 250 mm x 30 mm; Laufmittel und Gradientenprogramm: Acetonitril/0.1% aq. Ameisensäure 10:90 (0-3 min), Acetonitril/0.1% aq. Ameisensäure 10:90 → 95:5 (3-27 min), Acetonitril/0.1% aq. Ameisensäure 95:5 (27-34 min), Acetonitril/0.1% aq. Ameisensäure 10:90 (34-38 min); Fluss: 50 ml/min; Temperatur: 22°C; UV-Detektion: 254 nm.

### Methode 12 (präparative HPLC):

Säule: Daiso C18 Bio Spring Column, 10 µm 300 mm x 100 mm; Laufmittel und Gradientenprogramm: Wasser/Methanol 80:20 (0-6 min), Wasser/Methanol 80:20 → 20:80 (6-60 min), Wasser/ Methanol 20:80 (60-95 min), Wasser/Methanol 10:90 (95-105 min), Wasser/Methanol 80:20 (105-113 min); Fluss: 250 ml/min; Temperatur: 25°C; UV-Detektion: 240 nm.

### Methode 13 (präparative HPLC):

Säule: Reprosil-Pur C18, 10 µm 250 mm x 30 mm; Laufmittel und Gradientenprogramm: Aceto-nitril/0.1% aq. Ameisensäure 10:90 (0-3 min), Acetonitril/0.1% aq. Ameisensäure 10:90 → 95:5 (3-27 min), Acetonitril/0.1% aq. Ameisensäure 95:5 (27-34 min), Acetonitril/0.1% aq. Ameisensäure 10:90 (34-38 min); Fluss: 50 ml/min; Temperatur: 22°C; UV-Detektion: 254 nm.

### Methode 14 (LC/MS):

Instrument: Waters Acquity SQD UPLC System; Säule: Waters Acquity UPLC HSS T3 1.8µ 30 mm x 2 mm; Eluent A: 11 Wasser + 0.25 ml 99%-ige Ameisensäure, Eluent B: 11 Acetonitril + 0.25 ml 99%-ige Ameisensäure; Gradient: 0.0 min 90% A → 1.2 min 5% A → 2.0 min 5% A; Fluss: 0.60 ml/min; Ofen: 50°C; UV-Detektion: 208-400 nm.

Für alle Reaktanden oder Reagenzien, deren Herstellung im Folgenden nicht explizit beschrieben ist, gilt, dass sie von allgemein zugänglichen Quellen kommerziell bezogen wurden. Für alle übrigen Reaktanden oder Reagenzien, deren Herstellung im Folgenden ebenfalls nicht beschrieben ist und die nicht kommerziell erhältlich waren oder von Quellen bezogen wurden, die nicht allgemein zugänglich sind, ist ein Verweis auf die veröffentlichte Literatur angegeben, in der ihre Herstellung beschrieben ist.

### Ausgangsverbindungen und Intermediate:

### Beispiel 1A

### 5-[1-(3-Brombenzyl)-5-methyl-1H-pyrazol-3-yl]-3-[4-(trifluormethoxy)phenyl]-1,2,4-oxadiazol

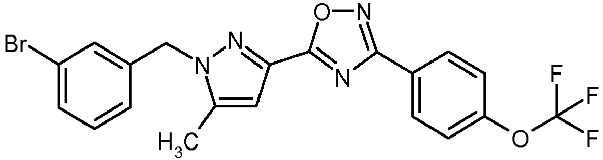

### Schritt 1: 5-(5-Methyl-1H-pyrazol-3-yl)-3-[4-(trifluormethoxy)phenyl]-1,2,4-oxadiazol

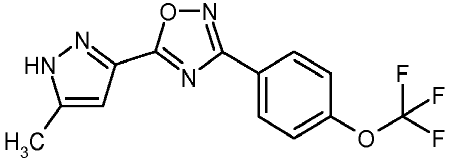

Zu einer Suspension von 20.25 g (125 mmol) 1,1'-Carbonyldiimidazol in 75 ml wasserfreiem DMF wurde bei RT innerhalb von 15 min eine Lösung von 15.0 g (119 mmol) 5-Methyl-1*H* pyrazol-3-carbonsäure in 75 ml wasserfreiem DMF hinzugetropft. Nachdem das Gemisch 1 h 45 min bei RT gerührt worden war, wurden 26.19 g (119 mmol) *N'*-Hydroxy-4-(trifluormethoxy)benzolcarboximidamid hinzugefügt. Anschließend wurde das Reaktionsgemisch 4 h auf 110°C erhitzt. Nach dem Abkühlen wurde der größte Teil des Lösungsmittels am Rotationsverdampfer entfernt. Der Rückstand wurde mit 800 ml Wasser versetzt und einige Minuten gerührt. Das ungelöste Produkt wurde abgesaugt und mit Diethylether gewaschen. Vom Filtrat wurde die Etherphase abgetrennt und die wässrige Phase noch einmal mit Diethylether extrahiert. Die vereinigten Etherextrakte wurden mit 25 ml Methanol versetzt und das zuvor abgesaugte Produkt darin suspendiert. Nach einigen Minuten Rühren wurde erneut abgesaugt. Der Rückstand wurde im Hochvakuum getrocknet und ergab eine erste Fraktion der Titelverbindung (8.79 g). Das Filtrat wurde mit dem gleichen Volumen Wasser versetzt und ausgeschüttelt. Nach Phasentrennung wurde die organische Phase mit gesättigter Natriumchlorid-Lösung gewaschen, über wasserfreiem Magnesiumsulfat getrocknet und schließlich am Rotationsverdampfer vom Lösungsmittel befreit. Nach Trocknen des Rückstands im Hochvakuum wurde auf diese Weise eine zweite Fraktion der Titelverbindung gewonnen (24.43 g). Insgesamt wurden so 33.32 g (90% d. Th.) der Titelverbindung erhalten.
¹H-NMR (400 MHz, CDCl₃, δ/ppm): 10.64 (breit, 1H), 8.23 (d, 2H), 7.34 (d, 2H), 6.82 (s, 1H), 2.46 (s, 3H).
LC/MS (Methode 4, ESIpos): Rₜ = 1.28 min, m/z = 311 [M+H]⁺.

### Schritt 2: 5-[1-(3-Brombenzyl)-5-methyl-1H-pyrazol-3-yl]-3-[4-(trifluormethoxy)phenyl]-1,2,4-oxadiazol

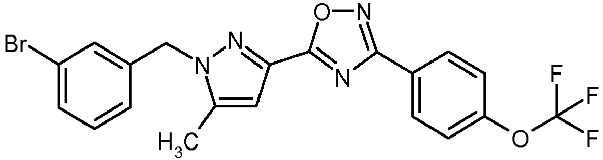

Eine Lösung von 14.0 g (45.1 mmol) der Verbindung aus Beispiel 1A / Schritt 1 und 13.54 g (54.2 mmol) 3-Brombenzylbromid in 450 ml wasserfreiem Dioxan wurde bei RT mit 6.58 g (58.7 mmol) festem Kalium-*tert*.-butylat versetzt. Das Reaktionsgemisch wurde zunächst 16 h bei RT gerührt und dann noch weitere 2 h bei 45°C, um den Umsatz zu vervollständigen. Anschließend wurde mit 500 ml Wasser versetzt und dreimal mit je ca. 300 ml Ethylacetat extrahiert. Die vereinigten organischen Extrakte wurden nacheinander mit Wasser und gesättigter Kochsalz-Lösung gewaschen, über wasserfreiem Magnesiumsulfat getrocknet und schließlich am Rotationsverdampfer vom Lösungsmittel befreit. Das erhaltene Rohprodukt wurde durch Verrühren mit einem Gemisch aus 525 ml Pentan und 35 ml Diisopropylether gereinigt. Nach Absaugen und Trocknen des Feststoffs wurden 19.08 g (84% d. Th., 95% Reinheit) der Titelverbindung erhalten.
¹H-NMR (400 MHz, CDCl₃, δ/ppm): 8.25 (d, 2H), 7.44 (d, 1H), 7.33 (d, 2H), 7.32 (s, 1H), 7.22 (t, 1H), 7.08 (d, 1H), 6.83 (s, 1H), 5.43 (s, 2H), 2.30 (s, 3H).
LC/MS (Methode 6, ESIpos): Rₜ = 1.45 min, m/z = 479/481 [M+H]⁺.

### Beispiel 2A

### 5-[1-(3-Brombenzyl)-5-methyl-1H-pyrazol-3-yl]-3-{4-[1-(trifluormethyl)cyclopropyl]phenyl}-1,2,4-oxadiazol

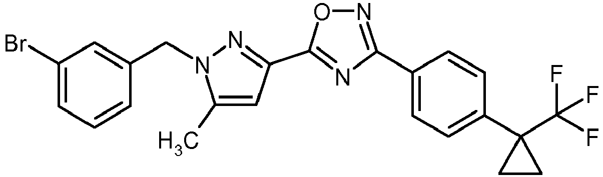

### Schritt 1: 1-Brom-4-[1-(trifluormethyl)cyclopropyl]benzol

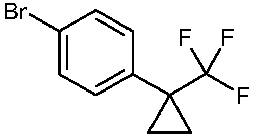

Zunächst wurde aktiviertes Zinkbromid auf Montmorillonit wie folgt dargestellt: 7.0 g (31.1 mmol) Zinkbromid wurden in einem 1 Liter-Kolben in 225 ml Methanol vorgelegt und mit 28.2 g Montmorillonit K10 versetzt. Anschließend wurde die Suspension 1 h bei RT gerührt. Dann wurde am Rotationsverdampfer bis zur Trockene eingedampft. Das verbleibende feine Pulver wurde unter schwachem Vakuum (ca. 500 mbar) 1 h lang in einem Sandbad auf 200°C Badtemperatur erhitzt und anschließend unter Argon abkühlen gelassen.

Die Titelverbindung wurde anschließend wie folgt dargestellt: 49.63 g (267 mmol) 1-Phenyl-1-(trifluormethyl)cyclopropan wurden in 1.25 Liter Pentan vorgelegt und mit dem oben erhaltenen aktivierten Zinkbromid auf Montmorillonit versetzt. Dann wurde das Reaktionsgefäß außen mit Aluminiumfolie verpackt, um den Lichteinfall zu reduzieren. Es wurden 137 ml (2.67 mol) Brom unter Rühren langsam zugetropft. Anschließend wurde das Reaktionsgemisch 16 h in der Dunkelheit bei RT gerührt. Dann wurde unter Eiskühlung 1 Liter gesättigte wässrige Natriumsulfit-Lösung hinzugetropft. Die Feststoffe wurden abgesaugt und zweimal mit Pentan nachgewaschen. Nach Phasentrennung wurde das Filtrat noch zweimal mit je 1 Liter Pentan extrahiert. Die vereinigten organischen Extrakte wurden über wasserfreiem Natriumsulfat getrocknet, filtriert und am Rotationsverdampfer unter nur schwachem Vakuum vom Lösungsmittel befreit. Es wurden 77.18 g (92% Reinheit, 100% d. Th.) der Titelverbindung erhalten.
¹H-NMR (400 MHz, CDCl₃, δ/ppm): 7.47 (d, 2H), 7.33 (s, 2H), 1.37-1.34 (m, 2H), 1.03-0.98 (m, 2H).
GC/MS (Methode 9, ESIpos): Rₜ = 3.43 min, m/z = 264/266 [M]⁺.

### Schritt 2: 4-[1-(Trifluormethyl)cyclopropyl]benzonitril

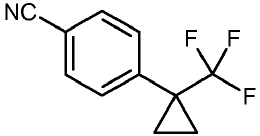

Eine Lösung von 75.0 g (283 mmol) der Verbindung aus Beispiel 2A / Schritt 1 in einem Gemisch aus 990 ml DMF und 10 ml Wasser wurde durch wiederholtes Anlegen eines schwachen Vakuums und Begasen mit Argon von Sauerstoff befreit. Dann wurde mit 37.87 g (322 mmol) Zinkcyanid und 32.69 g (28.3 mmol) Tetrakis(triphenylphosphin)palladium(0) versetzt. Das Reaktionsgemisch wurde anschließend 5 h auf 120°C erhitzt. Nach dem Erkalten auf RT wurde vom Ungelösten abfiltriert und der Rückstand mit wenig DMF nachgewaschen. Das Filtrat wurde anschließend am Rotationsverdampfer vom Lösungsmittel befreit. Das erhaltene Rohprodukt wurde in 1.5 Liter Ethylacetat gelöst und zweimal mit je 500 ml gesättigter Ammoniumchlorid-Lösung und einmal mit 500 ml gesättigter Natriumchlorid-Lösung gewaschen. Nach Trocknen der organischen Phase über wasserfreiem Magnesiumsulfat wurde filtriert und das Filtrat am Rotationsverdampfer eingeengt. Das erhaltene Öl wurde mittels Saugfiltration über 175 g Kieselgel mit Cyclohexan/Ethylacetat 40:1 als Laufmittel gereinigt. Nach Eindampfen der Produktfraktionen und Trocknen im Hochvakuum wurden 49.7 g (83% d. Th.) der Titelverbindung erhalten.
¹H-NMR (400 MHz, CDCl₃, δ/ppm): 7.65 (d, 2H), 7.57 (d, 2H), 1.46-1.42 (m, 2H), 1.09-1.03 (m, 2H).
GC/MS (Methode 9, ESIpos): Rₜ = 3.79 min, m/z = 211 [M]⁺.

### Schritt 3: N'-Hydroxy-4-[1-(trifluormethyl)cyclopropyl]benzolcarboximidamid

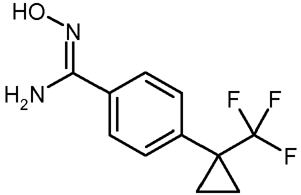

Eine Lösung von 20.0 g (94.7 mmol) der Verbindung aus Beispiel 2A / Schritt 2 in 500 ml Ethanol wurde mit 14.48 g (208 mmol) Hydroxylammoniumchlorid und 29 ml (208 mmol) Triethylamin versetzt. Das Reaktionsgemisch wurde 2 h unter Rückfluss erhitzt. Anschließend wurde ungefähr die Hälfte des Lösungsmittels am Rotationsverdampfer entfernt. Der verbliebene Rest wurde mit 1.5 Liter Wasser versetzt, und die entstandene Suspension wurde 20 min bei RT gerührt. Dann wurde der Feststoff abgesaugt, mit wenig kaltem Wasser gewaschen und im Hochvakuum getrocknet. Zur weiteren Reinigung wurde mit einem Gemisch aus 120 ml Pentan und 30 ml Dichlormethan verrührt. Nach erneutem Absaugen und Trocknen des Feststoffs wurden 15.79 g (68% d. Th.) der Titelverbindung erhalten.
¹H-NMR (400 MHz, DMSO-d₆, δ/ppm): 9.68 (s, 1H), 7.67 (d, 2H), 7.46 (d, 2H), 5.83 (s, breit, 2H), 1.36-1.32 (m, 2H), 1.15-1.11 (m, 2H).
LC/MS (Methode 4, ESIpos): Rₜ = 0.80 min, m/z = 245 [M+H]⁺.

### Schritt 4: 5-(5-Methyl-1H-pyrazol-3-yl)-3-{4-[1-(trifluormethyl)cyclopropyl]phenyl}-1,2,4-oxadiazol

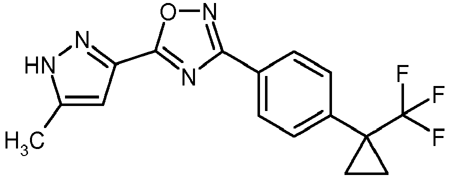

Zu einer Suspension von 6.75 g (41.6 mmol) 1,1'-Carbonyldiimidazol in 25 ml wasserfreiem DMF wurde bei RT innerhalb von 15 min eine Lösung von 5.0 g (36.6 mmol) 5-Methyl-1*H-* pyrazol-3-carbonsäure in 25 ml wasserfreiem DMF hinzugetropft. Nachdem das Gemisch 1 h 45 min bei RT gerührt worden war, wurden 9.68 g (39.6 mmol) der Verbindung aus Beispiel 2A / Schritt 3 hinzugefügt. Anschließend wurde das Reaktionsgemisch 2.5 h auf 110°C erhitzt. Nach dem Abkühlen auf RT wurden langsam unter starkem Rühren 800 ml Wasser hinzugefügt, wodurch das Produkt ausfiel. Der Feststoff wurde abgesaugt und mit wenig kaltem Wasser gewaschen. Das noch feuchte Rohprodukt wurde aus einem siedenden Gemisch aus 300 ml Ethanol und 350 ml Wasser umkristallisiert. Es wurden 11.03 g (83% d. Th.) der Titelverbindung erhalten.
¹H-NMR (400 MHz, CDCl₃, δ/ppm): 11.04 (s, breit, 1H), 8.16 (d, 2H), 7.60 (d, 2H), 6.82 (s, 1H), 2.45 (s, 3H), 1.43-1.40 (m, 2H), 1.11-1.07 (m, 2H).
LC/MS (Methode 6, ESIpos): Rₜ = 1.14 min, m/z = 335 [M+H]⁺.

### Schritt 5: 5-[1-(3-Brombenzyl)-5-methyl-1H-pyrazol-3-yl]-3-{4-[1-(trifluormethyl)cyclopropyl]-phenyl}-1,2,4-oxadiazol

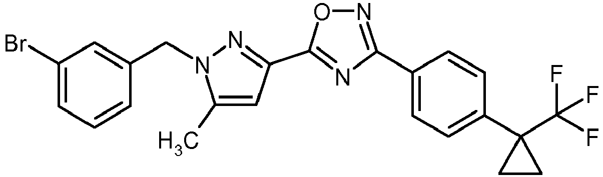

Eine Lösung von 1.0 g (2.99 mmol) der Verbindung aus Beispiel 2A / Schritt 4 und 897 mg (3.59 mmol) 3-Brombenzylbromid in 30 ml wasserfreiem Dioxan wurde bei 0°C mit 436 mg (3.89 mmol) festem Kalium-*tert*.-butylat versetzt. Das Reaktionsgemisch wurde anschließend 16 h bei RT gerührt. Dann wurde mit ca. 200 ml Wasser versetzt und dreimal mit je ca. 100 ml Ethylacetat extrahiert. Die vereinigten organischen Extrakte wurden nacheinander mit Wasser und gesättigter Kochsalz-Lösung gewaschen, über wasserfreiem Magnesiumsulfat getrocknet und schließlich am Rotationsverdampfer vom Lösungsmittel befreit. Das erhaltene Rohprodukt wurde zunächst mittels Saugfiltration über Kieselgel mit Cyclohexan/Ethylacetat 10:1 als Laufmittel vorgereinigt. Die weitere Reinigung des Produkts erfolgte durch Verrühren mit einem Gemisch aus 50 ml Pentan und 2 ml Diethylether. Nach Absaugen und Trocknen des Feststoffs wurden 1.34 g (88% d. Th.) der Titelverbindung erhalten.
¹H-NMR (400 MHz, CDCl₃, δ/ppm): 8.19 (d, 2H), 7.59 (d, 2H), 7.44 (d, 1H), 7.31 (s, 1H), 7.22 (t, 1H), 7.08 (d, 1H), 6.83 (s, 1H), 5.43 (s, 2H), 2.28 (s, 3H), 1.42-1.39 (m, 2H), 1.11-1.07 (m, 2H).
LC/MS (Methode 6, ESIpos): Rₜ = 1.48 min, m/z = 503/505 [M+H]⁺.

### Beispiel 3A

### 5-[1-(3-Brombenzyl)-5-methyl-1H-pyrazol-3-yl]-3-[4-(1,1,1-trifluor-2-methylpropan-2-yl)phenyl]-1,2,4-oxadiazol

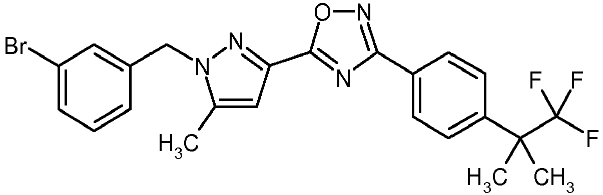

### Schritt 1: 2-(4-Bromphenyl)-1,1,1-trifluorpropan-2-ol

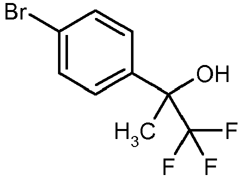

Zunächst wurde eine Suspension von Dichlor(dimethyl)titan in einem Heptan/Dichlormethan-Gemisch wie folgt hergestellt: Man kühlte 100 ml (100 mmol) einer 1 M Lösung von Titantetrachlorid in Dichlormethan auf -30°C, tropfte 100 ml (100 mmol) einer 1 M Lösung von Dimethylzink in Heptan hinzu und rührte 30 min bei -30°C nach. Anschließend wurde diese Suspension auf -40°C abgekühlt und eine Lösung von 10 g (39.5 mmol) 1-(4-Bromphenyl)-2,2,2-trifluorethanon in 50 ml Dichlormethan hinzugegeben. Man rührte 5 min bei -40°C nach, ließ dann die Temperatur auf RT kommen und rührte weitere 2 h bei RT. Unter Eiskühlung ließ man langsam 50 ml Wasser hinzutropfen und verdünnte anschließend mit weiteren 300 ml Wasser. Man extrahierte zweimal mit Dichlormethan, wusch die vereinigten Dichlormethan-Phasen einmal mit Wasser, trocknete über wasserfreiem Magnesiumsulfat, filtrierte und entfernte das Lösungsmittel am Rotationsverdampfer. Der Rückstand wurde säulenchromatographisch an Kieselgel gereinigt (Laufmittel: Cyclohexan/Ethylacetat 85:15). Es wurden 10.5 g (100% d. Th.) der Titelverbindung erhalten, welche laut ¹H-NMR noch Reste von Lösungsmittel enthielt.
¹H-NMR (400 MHz, CDCl₃, δ/ppm): 7.52 (d, 2H), 7.47 (d, 2H), 1.76 (s, 3H).
LC/MS (Methode 1, ESIpos): Rₜ = 2.27 min, m/z = 268 [M+H]⁺.

### Schritt 2: 2-(4-Bromphenyl)-1,1,1 -trifluorpropan-2-ylmethansulfonat

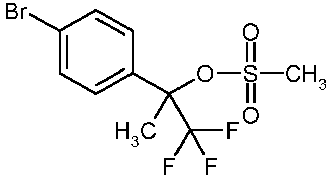

Man legte 3.12 g (78.05 mmol, 60%-ig in Mineralöl) Natriumhydrid in 45 ml THF unter Argon vor und tropfte eine Lösung von 10.5 g (39.03 mmol) der in Beispiel 3A / Schritt 1 erhaltenen Verbindung in 20 ml THF bei RT hinzu. Nachdem man 1 h bei RT und 30 min bei 40°C gerührt hatte, wurde eine Lösung von 8.94 g (78.05 mmol) Methansulfonylchlorid in 45 ml THF hinzugetropft und das Reaktionsgemisch weitere 60 min bei 40°C gerührt. Anschließend tropfte man langsam 50 ml Wasser zum Gemisch hinzu, verdünnte mit gesättigter wässriger Natriumhydrogencarbonat-Lösung und extrahierte zweimal mit Ethylacetat. Man trocknete die vereinigten Ethylacetat-Phasen über wasserfreiem Magnesiumsulfat, filtrierte und entfernte das Lösungsmittel am Rotationsverdampfer. Der Rückstand wurde in Hexan verrührt und der erhaltene Feststoff abfiltriert und im Vakuum getrocknet. Es wurden 12.4 g (92% d. Th.) der Titelverbindung erhalten.
¹H-NMR (400 MHz, CDCl₃, δ/ppm): 7.58 (d, 2H), 7.43 (d, 2H), 3.16 (s, 3H), 2.28 (s, 3H).
LC/MS (Methode 2, ESIpos): Rₜ = 2.32 min, m/z = 364 [M+NH₄]⁺.

### Schritt 3: 1-Brom-4-(1,1,1-trifluor-2-methylpropan-2-yl)benzol

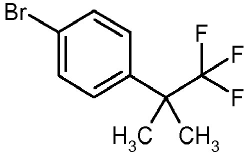

Man legte 12.4 g (35.72 mmol) der in Beispiel 3A / Schritt 2 erhaltenen Verbindung in 250 ml Dichlormethan vor und kühlte auf 0°C ab. Dann tropfte man langsam unter Rühren 35.7 ml (71.44 mmol) einer 2 M Lösung von Trimethylaluminium bei 0°C hinzu, ließ das Gemisch anschließend auf RT kommen und rührte weitere 1.5 h bei RT nach. Zu dem Gemisch tropfte man langsam 120 ml gesättigte wässrige Natriumhydrogencarbonat-Lösung und danach 40 ml gesättigte wässrige Natriumchlorid-Lösung hinzu. Man filtrierte über Kieselgur und wusch das Kieselgur zweimal mit Dichlormethan nach. Man wusch die vereinigten Dichlormethan-Phasen einmal mit gesättigter wässriger Natriumchlorid-Lösung, trocknete über wasserfreiem Magnesiumsulfat und entfernte das Lösungsmittel am Rotationsverdampfer. Es wurden 8.69 g (87% d. Th.) der Titelverbindung in 95%-iger Reinheit erhalten.
¹H-NMR (400 MHz, CDCl₃, δ/ppm): 7.49 (d, 2H), 7.33 (d, 2H), 1.55 (s, 6H).
GC/MS (Methode 9, EIpos): Rₜ = 3.48 min, m/z = 266 [M]⁺.

### Schritt 4: 4-(1,1,1-Trifluor-2-methylpropan-2-yl)benzolcarbonitril

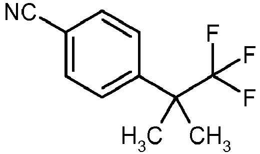

Man legte 3.34 g (12.50 mmol) der in Beispiel 3A / Schritt 3 erhaltenen Verbindung in 2.5 ml entgastem DMF unter Argon vor, gab 881 mg (7.50 mmol) Zinkcyanid sowie 867 mg (0.75 mmol) Tetrakis(triphenylphosphin)palladium(0) hinzu und rührte über Nacht bei 80°C. Nach Abkühlen auf RT verdünnte man das Reaktionsgemisch mit Ethylacetat und filtrierte feste Bestandteile ab. Das Filtrat wurde zweimal mit 2 N wässriger Ammoniak-Lösung und einmal mit gesättigter wässriger Natriumchlorid-Lösung gewaschen, über wasserfreiem Magnesiumsulfat getrocknet und am Rotationsverdampfer vom Lösungsmittel befreit. Der Rückstand wurde säulenchromatographisch an Kieselgel gereinigt (Laufmittel: Cyclohexan/Ethylacetat 85:15). Es wurden 2.08 g (78% d. Th.) der Titelverbindung erhalten.
¹H-NMR (400 MHz, CDCl₃, δ/ppm): 7.68 (d, 2H), 7.62 (d, 2H), 1.60 (s, 6H).
GC/MS (Methode 9, EIpos): Rₜ = 3.83 min, m/z = 213 [M]⁺.

### Schritt 5: N'-Hydroxy-4-(1,1,1-trifluor-2-methylpropan-2-yl)benzolcarboximidamid

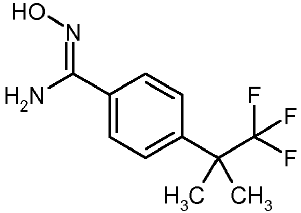

Ein Gemisch aus 2.40 g (11.26 mmol) der Verbindung aus Beispiel 3A / Schritt 4, 1.72 g (24.77 mmol) Hydroxylamin-Hydrochlorid und 3.45 ml (24.77 mmol) Triethylamin in 60 ml Ethanol wurde 1 h unter Rückfluss gerührt. Nach Abkühlen auf RT wurde das Lösungsmittel am Rotationsverdampfer entfernt. Man versetzte den Rückstand mit Ethylacetat und filtrierte den vorhandenen Feststoff ab. Die Ethylacetat-Lösung wurde nacheinander mit Wasser und gesättigter wässriger Natriumchlorid-Lösung gewaschen, über wasserfreiem Magnesiumsulfat getrocknet und filtriert. Nach Entfernen des Lösungsmittels wurde das erhaltene Öl mit Petrolether verrieben. Nach Absaugen des resultierenden Feststoffs und Trocknen im Hochvakuum wurden 2.65 g (96% d. Th.) der Titelverbindung erhalten.
¹H-NMR (400 MHz, CDCl₃, δ/ppm): 8.0 (s, breit, 1H), 7.62 (d, 2H), 7.52 (d, 2H), 4.88 (s, breit, 2H), 1.60 (s, 6H).
LC/MS (Methode 2, ESIpos): Rₜ = 1.34 min, m/z = 247 [M+H]⁺.

### Schritt 6: 5-(5-Methyl-1H-pyrazol-3-yl)-3-[4-(1,1,1-trifluor-2-methylpropan-2-yl)phenyl]-1,2,4-oxadiazol

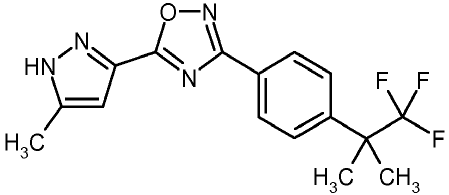

Eine Lösung von 7.57 g (60.0 mmol) 5-Methyl-1*H*-pyrazol-3-carbonsäure in 300 ml wasserfreiem DMF wurde bei RT nacheinander mit 11.6 g (60.0 mmol) EDC, 8.13 g (60.0 mmol) HOBt und 14.8 g (60.0 mmol) der Verbindung aus Beispiel 3A / Schritt 5 versetzt. Das Gemisch wurde zunächst 2 h bei RT und anschließend 5 h bei 140°C gerührt. Nach dem Abkühlen wurde das Reaktionsgemisch in 900 ml Eiswasser eingerührt. Das dabei ausgefallene Produkt wurde abgesaugt, mit kaltem Wasser gewaschen und anschließend im Hochvakuum getrocknet. Es wurden so 14.7 g (73% d. Th.) der Titelverbindung erhalten.
¹H-NMR (400 MHz, DMSO-d₆, δ/ppm): 11.80 (s, breit, 1H), 8.17 (d, 2H), 7.63 (d, 2H), 6.83 (s, 1H), 2.46 (s, 3H), 1.63 (s, 6H).
LC/MS (Methode 4, ESIpos): Rₜ = 1.34 min, m/z = 337 [M+H]⁺.

### Schritt 7: 5-[1-(3-Brombenzyl)-5-methyl-1H-pyrazol-3-yl]-3-[4-(1,1,1-trifluor-2-methylpropan-2-yl)phenyl]-1,2,4-oxadiazol

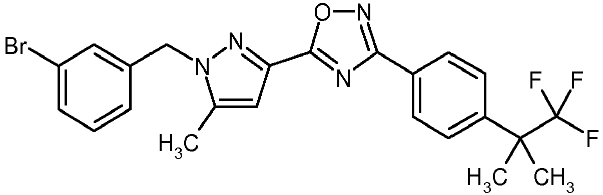

Analog zu dem unter Beispiel 2A / Schritt 5 beschriebenen Verfahren wurden aus 750 mg (2.23 mmol) der Verbindung aus Beispiel 3A / Schritt 6 und 669 mg (2.68 mmol) 3-Brombenzylbromid 1.02 g (84% d. Th., 94% Reinheit) der Titelverbindung hergestellt.
¹H-NMR (400 MHz, CDCl₃, δ/ppm): 8.20 (d, 2H), 7.62 (d, 2H), 7.44 (d, 1H), 7.32 (s, 1H), 7.22 (t, 1H), 7.08 (d, 1H), 6.83 (s, 1H), 5.43 (s, 2H), 2.29 (s, 3H), 1.63 (s, 6H).
LC/MS (Methode 6, ESIpos): Rₜ = 1.49 min, m/z = 505/507 [M+H]⁺.

### Beispiel 4A

### 5-[1-(3-Brombenzyl)-5-methyl-1H-pyrazol-3-yl]-3-[4-(tetrahydro-2H-pyran-4-yl)phenyl]-1,2,4-oxadiazol

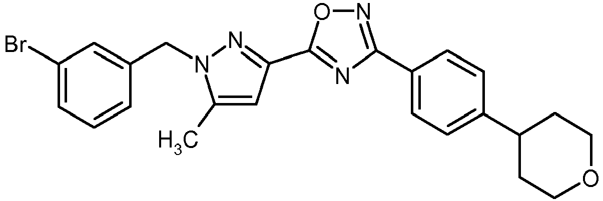

### Schritt 1: 4-(Tetrahydro-2H-pyran-4-yl)benzonitril

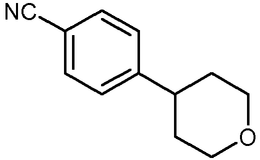

Eine Lösung von 2.91 g (19.8 mmol) 4-Cyanophenylboronsäure [M. Nishimura et al., Tetrahedron 2002, 58 (29), 5779-5788] in 20 ml Isopropanol wurde mit 186 mg (0.594 mmol) Nickel(II)iodid, 90 mg (0.594 mmol) *trans*-2-Aminocyclohexanol-Hydrochlorid und 3.63 g (19.8 mmol) Natriumhexamethyldisilazid versetzt. Die so erhaltene Suspension wurde 5 min bei RT unter einer Argonatmosphäre gerührt. Dann wurden 2.1 g (9.90 mmol) 4-Iodtetrahydropyran [Heuberger et al., J. Chem. Soc. 1952, 910] zugefügt. Nachdem das Reaktionsgemisch 15 h bei einer Temperatur von 75°C gerührt worden war, wurde es auf RT abgekühlt und mit Dichlormethan durch Filtration über ca. 50 g Kieselgel von anorganischen Salzen weitgehend befreit. Das Rohprodukt wurde durch MPLC (Kieselgel, Laufmittel: Dichlormethan) gereinigt. Es wurden so 986 mg (53% d. Th.) der Titelverbindung erhalten.
¹H-NMR (400 MHz, CDCl₃, δ/ppm): 7.60 (d, 2H), 7.32 (d, 2H), 4.12-4.07 (m, 2H), 3.56-3.50 (m, 2H), 2.87-2.79 (m, 1H), 1.86-1.73 (m, 4H).
GC/MS (Methode 9, EIpos): Rₜ = 5.97 min, m/z = 187 [M]⁺.

### Schritt 2: N'-Hydroxy-4-(tetrahydro-2H-pyran-4-yl)benzolcarboximidamid

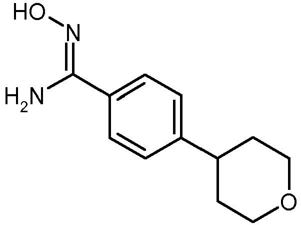

480 mg (2.56 mmol) der Verbindung aus Beispiel 4A / Schritt 1, 392 mg (5.64 mmol) Hydroxylammoniumchlorid und 786 µl (5.64 mmol) Triethylamin wurden in 18 ml Ethanol 16 h unter Rückfluss erhitzt. Anschließend wurde der größte Teil der flüchtigen Komponenten am Rotationsverdampfer entfernt. Der verbliebene Rückstand wurde mit 50 ml Wasser versetzt und einige Minuten bei RT gerührt. Dann wurde der Feststoff abgesaugt, mit wenig kaltem Wasser gewaschen und schließlich im Hochvakuum getrocknet. Es wurden 525 mg (93% d. Th.) der Titelverbindung erhalten.
¹H-NMR (400 MHz, CDCl₃, δ/ppm): 7.58 (d, 2H), 7.26 (d, 2H), 6.79 (breit, 1H), 4.82 (s, breit, 2H), 4.11-4.05 (m, 2H), 3.57-3.50 (m, 2H), 2.83-2.74 (m, 1H), 1.87-1.73 (m, 4H).
LC/MS (Methode 2, ESIpos): Rₜ = 0.92 min, m/z = 221 [M+H]⁺.

### Schritt 3: 5-(5-Methyl-1H-pyrazol-3-yl)-3-[4-(tetrahydro-2H-pyran-4-yl)phenyl]-1,2,4-oxadiazol

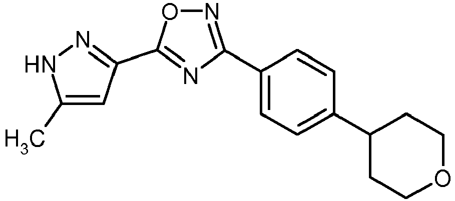

Eine Lösung von 469 mg (3.72 mmol) 5-Methyl-1*H*-pyrazol-3-carbonsäure in 15 ml wasserfreiem DMF wurde bei RT nacheinander mit 785 mg (4.10 mmol) EDC, 627 mg (4.10 mmol) HOBt und 820 mg (3.72 mmol) der Verbindung aus Beispiel 4A / Schritt 2 versetzt. Das Gemisch wurde zunächst 16 h bei RT und anschließend 20 min bei 140°C gerührt. Nach dem Abkühlen wurde das Reaktionsgemisch mit 100 ml Wasser versetzt und dreimal mit je ca. 100 ml Ethylacetat extrahiert. Die vereinigten organischen Extrakte wurden mit gesättigter Natriumchlorid-Lösung gewaschen, über wasserfreiem Magnesiumsulfat getrocknet und schließlich am Rotationsverdampfer bis zur Trockene eingedampft. Aus dem verbliebenen Rückstand wurden 450 mg der Titelverbindung durch Ausrühren aus Acetonitril und weitere 97 mg der Titelverbindung nach Aufreinigung der Mutterlauge durch präparative HPLC (Methode 13) erhalten (Ausbeute insgesamt 47% d. Th.).
¹H-NMR (400 MHz, DMSO-d₆, δ/ppm): 13.52 (s, 1H), 8.01 (d, 2H), 7.49 (d, 2H), 6.79 (s, 1H), 3.99-3.95 (m, 2H), 3.49-3.42 (m, 2H), 2.92-2.84 (m, 1H), 2.34 (s, 3H), 1.77-1.65 (m, 4H).
LC/MS (Methode 6, ESIpos): Rₜ = 0.98 min, m/z = 311 [M+H]⁺.

### Schritt 4: 5-[1-(3-Brombenzyl)-5-methyl-1H-pyrazol-3-yl]-3-[4-(tetrahydro-2H-pyran-4-yl)-phenyl]-1,2,4-oxadiazol

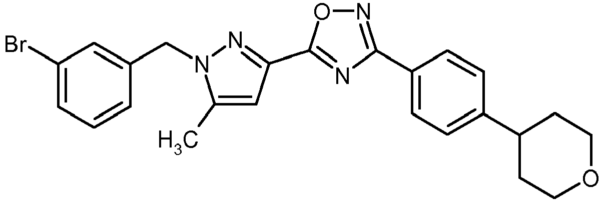

Analog zu dem unter Beispiel 1A / Schritt 2 beschriebenen Verfahren wurden aus 250 mg (0.806 mmol) der Verbindung aus Beispiel 4A / Schritt 3 und 242 mg (0.967 mmol) 3-Brombenzylbromid 338 mg (87% d. Th.) der Titelverbindung hergestellt. Die abschließende Reinigung des Produkts erfolgte hier durch Ausrühren aus 10 ml Pentan/Diisopropylether (5:1), dem einige Tropfen Dichlormethan zugesetzt waren.
¹H-NMR (400 MHz, CDCl₃, δ/ppm): 8.17 (d, 2H), 7.44 (d, 1H), 7.37 (d, 2H), 7.32 (s, 1H), 7.22 (t, 1H), 7.08 (d, 1H), 6.83 (s, 1H), 5.43 (s, 2H), 4.13-4.08 (m, 2H), 2.54 (dt, 2H), 2.88-2.79 (m, 1H), 2.28 (s, 3H), 1.92-1.78 (m, 4H).
LC/MS (Methode 4, ESIpos): Rₜ = 1.52 min, m/z = 479/481 [M+H]⁺.

### Beispiel 5A

### 5-[1-(3-Brombenzyl)-5-methyl-1H-pyrazol-3-yl]-3-[4-(4-fluortetrahydro-2H-pyran-4-yl)phenyl]-1,2,4-oxadiazol

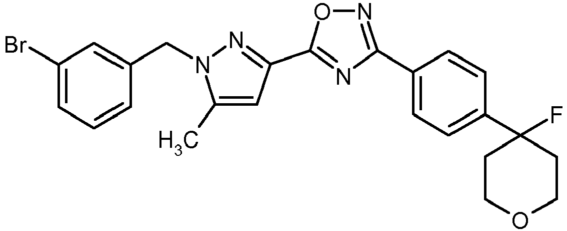

### Schritt 1: 4-(4-Hydroxytetrahydro-2H-pyran-4-yl)benzolcarbonitril

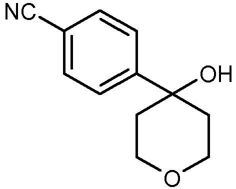

Unter inerten Bedingungen wurde bei -40°C eine Lösung von 50.0 g (218 mmol) 4-Iodbenzonitril in 1000 ml wasserfreiem THF tropfenweise mit 109 ml (218 mmol) einer 2 M Lösung von Isopropylmagnesiumchlorid in Diethylether versetzt. Nachdem das Gemisch 1.5 h bei derselben Temperatur gerührt worden war, wurde eine Lösung von 32.8 g (327 mmol) Tetrahydro-4*H-*pyran-4-on in 250 ml wasserfreiem THF hinzugefügt. Nach beendeter Zugabe wurde das Reaktionsgemisch zunächst 10 min bei -40°C, dann 30 min bei 0°C und schließlich 60 min bei RT gerührt. Es wurde dann bei - 20°C mit ca. 20 ml gesättigter wässriger Ammoniumchlorid-Lösung versetzt. Anschließend wurde das Lösungsmittel am Rotationsverdampfer weitgehend entfernt. Der verbliebene Rückstand wurde mit 1000 ml Wasser versetzt und dreimal mit je ca. 500 ml Dichlormethan extrahiert. Die vereinigten organischen Extrakte wurden nacheinander mit Wasser und gesättigter Kochsalz-Lösung gewaschen. Nach Trocknen über wasserfreiem Magnesiumsulfat wurde filtriert und das Lösungsmittel am Rotationsverdampfer entfernt. Das erhaltene Rohprodukt wurde durch Verrühren mit Cyclohexan/Ethylacetat 10:1 gereinigt. Es wurden 19.3 g (43% d. Th.) der Titelverbindung erhalten.
¹H-NMR (400 MHz, DMSO-d₆, δ/ppm): 7.80 (d, 2H), 7.70 (d, 2H), 5.30 (s, 1H), 3.81-3.70 (m, 4H), 2.02-1.94 (m, 2H), 1.51-1.48 (m, 2H).
LC/MS (Methode 4, ESIpos): Rₜ = 0.71 min, m/z = 186 [M-H₂O+H]⁺, 204 [M+H]⁺.

### Schritt 2: 4-(4-Fluortetrahydro-2H-pyran-4-yl)benzolcarbonitril

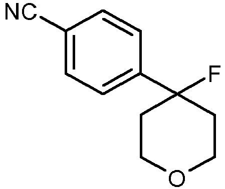

Unter inerten Bedingungen wurde bei -78°C eine Suspension von 6.5 g (31.98 mmol) der Verbindung aus Beispiel 5A / Schritt 1 in 800 ml Dichlormethan tropfenweise mit einer Lösung von 6.19 g (38.4 mmol) Diethylaminoschwefeltrifluorid (DAST) in 58 ml Dichlormethan versetzt. Nach 30 min bei -78°C wurde das Reaktionsgemisch mit Hilfe eines Eis/Wasser-Bades sehr schnell auf -20°C erwärmt. Nach ca. 30 Sekunden wurden 200 ml 1 M Natronlauge zugesetzt, und man ließ das Gemisch auf RT erwärmen. Nach Verdünnen mit 500 ml Wasser wurde dreimal mit je ca. 200 ml Diethylether extrahiert. Die vereinigten organischen Extrakte wurden über wasserfreiem Magnesiumsulfat getrocknet, und nach Filtration wurde das Lösungsmittel am Rotationsverdampfer entfernt. Das Rohprodukt wurde mittels MPLC gereinigt (Kieselgel, Laufmittel: Cyclohexan/Ethyl-acetat 10:1 → 5:1 → 2:1 → 1:1). Es wurden 3.73 g (57% d. Th.) der Titelverbindung erhalten.
¹H-NMR (400 MHz, CDCl₃, δ/ppm): 7.68 (d, 2H), 7.50 (d, 2H), 3.98-3.83 (m, 4H), 2.23-2.05 (m, 2H), 1.91-1.85 (m, 2H).
GC/MS (Methode 9, EIpos): Rₜ = 5.82 min, m/z = 205 [M]⁺.

### Schritt 3: 4-(4-Fluortetrahydro-2H-pyran-4-yl)-N'-hydroxybenzolcarboximidamid

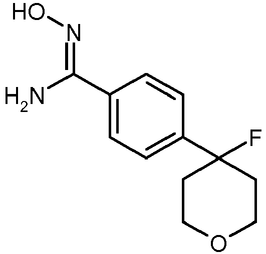

Nach dem unter Beispiel 4A / Schritt 2 beschriebenen Verfahren wurden aus 3.5 g (17.05 mmol) der Verbindung aus Beispiel 5A / Schritt 2 3.57 g (88% d. Th.) der Titelverbindung erhalten. Die Reaktionszeit betrug in diesem Fall 2 h.
¹H-NMR (500 MHz, DMSO-d₆, δ/ppm): 9.64 (s, 1H), 7.70 (d, 2H), 7.44 (d, 2H), 5.81 (s, 2H), 3.88-3.83 (m, 2H), 3.73-3.67 (m, 2H), 2.23-2.06 (m, 2H), 1.87-1.81 (m, 2H).
LC/MS (Methode 4, ESIpos): Rₜ = 0.40 min, m/z = 239 [M+H]⁺.

### Schritt 4: 3-[4-(4-Fluortetrahydro-2H-pyran-4-yl)phenyl]-5-(5-methyl-1H-pyrazol-3-yl)-1,2,4-oxadiazol

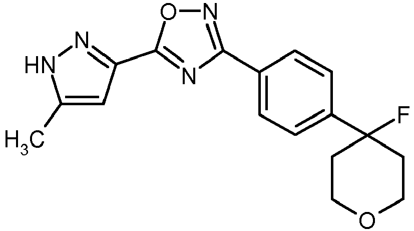

Eine Lösung von 4.30 g (34.1 mmol) 5-Methyl-1*H*-pyrazol-3-carbonsäure in 20 ml wasserfreiem DMF wurde bei RT innerhalb von ca. 15 min zu einer Suspension von 5.81 g (35.8 mmol) 1,1'-Carbonyldiimidazol (CDI) in 25 ml wasserfreiem DMF getropft. Nachdem das Gemisch 105 min bei RT gerührt worden war, wurden 8.12 g (34.1 mmol) der Verbindung aus Beispiel 5A / Schritt 3 hinzugefügt. Anschließend wurde das Reaktionsgemisch 5 h auf 110°C erhitzt. Nach dem Abkühlen auf RT wurde der Ansatz langsam in 800 ml Wasser eingerührt. Dabei fiel das Produkt aus. Es wurde abgesaugt und mit Wasser gewaschen. Anschließend wurde das feuchte Rohprodukt aus 430 ml Ethanol umkristallisiert. Nach Filtration und Trocknen des Feststoffs wurden 8.31 g (74% d. Th.) der Titelverbindung erhalten. Eine weitere Fraktion wurde durch Aufkonzentrieren der Mutterlauge gewonnen (1.69 g mit 85% Reinheit, 13% d. Th.).
¹H-NMR (400 MHz, CDCl₃, δ/ppm): 10.73 (breit, 1H), 8.20 (d, 2H), 7.52 (d, 2H), 6.81 (s, 1H), 4.00-3.88 (m, 4H), 2.45 (s, 3H), 2.30-2.11 (m, 2H), 1.98-1.91 (m, 2H).
LC/MS (Methode 1, ESIpos): Rₜ = 4.24 min, m/z = 329 [M+H]⁺.

### Schritt 5: 5-[1-(3-Brombenzyl)-5-methyl-1H-pyrazol-3-yl]-3-[4-(4-fluortetrahydro-2H-pyran-4-yl)-phenyl]-1,2,4-oxadiazol

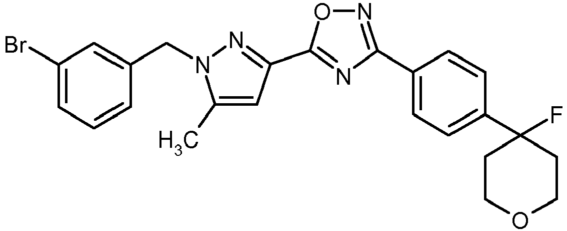

Analog zu dem unter Beispiel 1A / Schritt 2 beschriebenen Verfahren wurden aus 250 mg (0.761 mmol) der Verbindung aus Beispiel 5A / Schritt 4 und 228 mg (0.914 mmol) 3-Brombenzylbromid 355 mg (94% d. Th.) der Titelverbindung hergestellt. Auf ein Erwärmen des Reaktionsgemisches auf 45°C konnte hier verzichtet werden. Die abschließende Reinigung des Produkts erfolgte durch Ausrühren aus 10 ml Pentan/Diisopropylether (5:1), dem einige Tropfen Dichlormethan zugesetzt waren.
¹H-NMR (400 MHz, CDCl₃, δ/ppm): 8.23 (d, 2H), 7.53 (d, 2H), 7.44 (d, 1H), 7.32 (s, 1H), 7.23 (t, 1H), 7.08 (d, 1H), 6.84 (s, 1H), 5.43 (s, 2H), 4.00-3.87 (m, 4H), 2.29 (s, 3H), 2.30-2.11 (m, 2H), 1.98-1.91 (m, 2H).
LC/MS (Methode 4, ESIpos): Rₜ = 1.52 min, m/z = 497/499 [M+H]⁺.

### Beispiel 6A

### 5-[1-(3-Brombenzyl)-5-methyl-1H-pyrazol-3-yl]-3-[4-(3-fluoroxetan-3-yl)phenyl]-1,2,4-oxadiazol

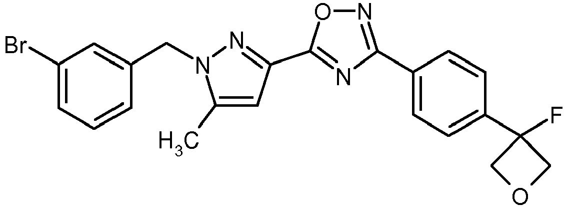

### Schritt 1: 4-(3-Hydroxyoxetan-3-yl)benzolcarbonitril

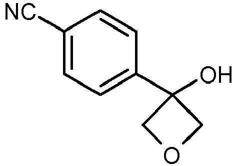

Unter inerten Bedingungen wurde bei -40°C eine Lösung von 5.0 g (21.8 mmol) 4-Iodbenzonitril in 100 ml wasserfreiem THF tropfenweise mit 11 ml (21.8 mmol) einer 2 M Lösung von Isopropylmagnesiumchlorid in Diethylether versetzt. Nachdem das Gemisch 1.5 h bei derselben Temperatur gerührt worden war, wurde es auf -78°C heruntergekühlt und mit Hilfe einer Kanüle zu einer ebenfalls auf -78°C gekühlten Lösung von 2.95 g (32.7 mmol, 80% in Dichlormethan) 3-Oxooxetan [G. Wuitschik et al., Angew. Chem. Int. Ed. Engl. 2006, 45 (46), 7736-7739] in 100 ml wasserfreiem THF langsam hinzugefügt. Nach beendeter Zugabe wurde das Reaktionsgemisch zunächst 10 min bei -78°C, dann 2 h bei 0°C und schließlich 30 min bei RT gerührt. Es wurde dann mit einigen ml gesättigter wässriger Ammoniumchlorid-Lösung versetzt. Anschließend wurde das Lösungsmittel am Rotationsverdampfer weitgehend entfernt. Der erhaltene Rückstand wurde mit 200 ml Wasser verdünnt und dreimal mit je ca. 200 ml Ethylacetat extrahiert. Die vereinigten organischen Extrakte wurden nacheinander mit Wasser und gesättigter Kochsalz-Lösung gewaschen. Nach Trocknen über wasserfreiem Magnesiumsulfat wurde filtriert und das Lösungsmittel am Rotationsverdampfer entfernt. Das erhaltene Rohprodukt wurde durch Kristallisation aus Cyclohexan/Ethylacetat 10:1 gereinigt. Es wurden 2.42 g (63% d. Th.) der Titelverbindung erhalten.
¹H-NMR (400 MHz, DMSO-d₆, δ/ppm): 7.88 (d, 2H), 7.80 (d, 2H), 6.63 (s, 1H), 4.79 (d, 2H), 4.65 (d, 2H).
HPLC (Methode 10): Rₜ = 3.09 min.
MS (DCI, NH₃): m/z = 193 [M+NH₄]⁺.

### Schritt 2: 4-(3-Fluoroxetan-3-yl)benzolcarbonitril

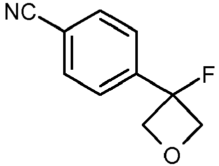

Analog zu dem unter Beispiel 5A / Schritt 2 beschriebenen Verfahren wurden aus 600 mg (3.43 mmol) der Verbindung aus Beispiel 6A / Schritt 1 und 662 mg (4.11 mmol) Diethylaminoschwefeltrifluorid (DAST) 495 mg (82% d. Th.) der Titelverbindung hergestellt. Für die MPLC-Reinigung wurde hier Cyclohexan/Ethylacetat 8:1 als Laufmittel verwendet.
¹H-NMR (400 MHz, CDCl₃, δ/ppm): 7.76 (d, 2H), 7.73 (d, 2H), 5.15 (dd, 2H), 4.81 (dd, 2H).
LC/MS (Methode 2, ESIpos): Rₜ = 1.59 min, m/z = 178 [M+H]⁺.

### Schritt 3: 4-(3-Fluoroxetan-3-yl)-N'-hydroxybenzolcarboximidamid

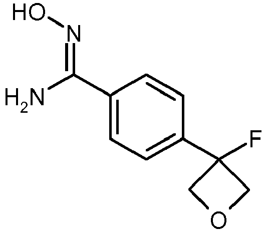

Nach dem unter Beispiel 4A / Schritt 2 beschriebenen Verfahren wurden aus 450 mg (2.54 mmol) der Verbindung aus Beispiel 6A / Schritt 2 470 mg (86% d. Th.) der Titelverbindung erhalten.
¹H-NMR (400 MHz, DMSO-d₆, δ/ppm): 9.71 (s, 1H), 7.77 (d, 2H), 7.54 (d, 2H), 5.87 (s, breit, 2H), 4.97 (dd, 2H), 4.91 (dd, 2H).
LC/MS (Methode 2, ESIpos): Rₜ = 0.80 min, m/z = 211 [M+H]⁺.

### Schritt 4: 3-[4-(3-Fluoroxetan-3-yl)phenyl]-5-(5-methyl-1H-pyrazol-3-yl)-1,2,4-oxadiazol

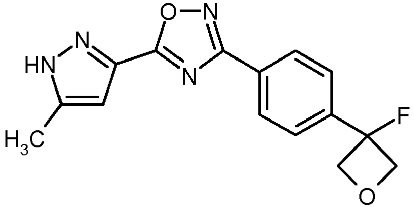

Eine Lösung von 300 mg (2.38 mmol) 5-Methyl-1*H*-pyrazol-3-carbonsäure in 10 ml wasserfreiem DMF wurde bei RT nacheinander mit 502 mg (2.62 mmol) EDC, 401 mg (2.62 mmol) HOBt und 500 mg (2.38 mmol) der Verbindung aus Beispiel 6A / Schritt 3 versetzt. Das Gemisch wurde zunächst 16 h bei RT und anschließend 45 min bei 140°C gerührt. Nach dem Abkühlen wurde das Lösungsmittel am Rotationsverdampfer entfernt. Der verbliebene Rückstand wurde mit 120 ml Wasser versetzt und dreimal mit je ca. 100 ml Diethylether extrahiert. Die vereinigten organischen Extrakte wurden mit gesättigter Natriumchlorid-Lösung gewaschen, über wasserfreiem Magnesiumsulfat getrocknet, filtriert und schließlich am Rotationsverdampfer eingedampft. Das Rohprodukt wurde bei RT 1 h lang mit 5 ml Ethanol verrührt. Nach Filtration und Trocknen des ungelösten Feststoffs im Hochvakuum wurde eine erste Fraktion von 204 mg der Titelverbindung erhalten. Die Mutterlauge wurde zur Trockene eingedampft. Anschließend wurde aus dem Rückstand mittels präparativer HPLC (Methode 13) eine weitere Fraktion von 29 mg der Titelverbindung isoliert. Insgesamt wurden somit 233 mg (33% d. Th.) der Titelverbindung erhalten.
¹H-NMR (400 MHz, CDCl₃, δ/ppm): 8.27 (d, 2H), 7.72 (d, 2H), 6.81 (s, 1H), 5.05 (dd, 2H), 5.01 (dd, 2H), 2.47 (s, 3H).
LC/MS (Methode 2, ESIpos): Rₜ = 1.93 min, m/z = 301 [M+H]⁺.

### Schritt 5: 5-[1-(3-Brombenzyl)-5-methyl-1H-pyrazol-3-yl]-3-[4-(3-fluoroxetan-3-yl)phenyl]-1,2,4-oxadiazol

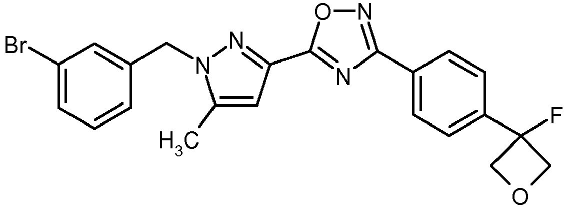

Analog zu dem unter Beispiel 1A / Schritt 2 beschriebenen Verfahren wurden aus 250 mg (0.833 mmol) der Verbindung aus Beispiel 6A / Schritt 4 und 250 mg (0.999 mmol) 3-Brombenzylbromid 347 mg (89% d. Th.) der Titelverbindung hergestellt. Auf ein Erwärmen des Reaktionsgemisches auf 45°C konnte hier verzichtet werden. Die abschließende Reinigung des Produkts erfolgte durch Ausrühren aus 10 ml Pentan/Diisopropylether (5:1), dem einige Tropfen Dichlormethan zugesetzt waren.
¹H-NMR (400 MHz, CDCl₃, δ/ppm): 8.30 (d, 2H), 7.72 (d, 2H), 7.45 (d, 1H), 7.33 (s, 1H), 7.22 (t, 1H), 7.09 (d, 1H), 6.85 (s, 1H), 5.43 (s, 2H), 5.04 (dd, 2H), 5.00 (dd, 2H), 2.29 (s, 3H).
LC/MS (Methode 4, ESIpos): Rₜ = 1.46 min, m/z = 469/471 [M+H]⁺.

### Beispiel 7A

### 5-[1-(3-Brombenzyl)-5-methyl-1H-pyrazol-3-yl]-3-[4-(1-methoxycyclobutyl)phenyl]-1,2,4-oxadiazol

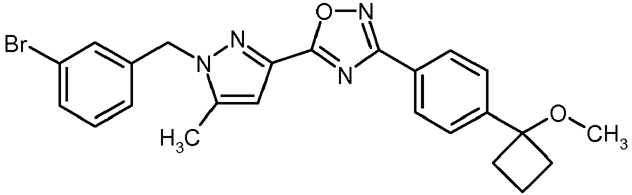

### Schritt 1: 4-(1-Hydroxycyclobutyl)benzonitril

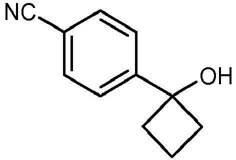

Analog zu dem unter Beispiel 5A / Schritt 1 beschriebenen Verfahren wurden aus 32.67 g (143 mmol) 4-Iodbenzonitril, 75 ml (150 mmol) 2 M Isopropylmagnesiumchlorid-Lösung in Diethylether und 15.0 g (214 mmol) Cyclobutanon 13.31 g (78% d. Th.) der Titelverbindung hergestellt. Die Reaktionszeit bei RT betrug in diesem Fall 16 h, und nach der wässrigen Aufarbeitung wurde das Rohprodukt mittels Saugfiltration über 150 g Kieselgel mit Cyclohexan/Ethylacetat 10:1 → 4:1 als Laufmittel gereinigt.
¹H-NMR (400 MHz, CDCl₃, δ/ppm): 7.67 (d, 2H), 7.62 (d, 2H), 2.58-2.51 (m, 2H), 2.44-2.37 (m, 2H), 2.23-2.04 (m, 2H), 1.83-1.72 (m, 1H).
HPLC (Methode 10): Rₜ = 3.47 min.
MS (DCI, NH₃): m/z = 191 [M+NH₄]⁺.

### Schritt 2: 4-(1-Methoxycyclobutyl)benzonitril

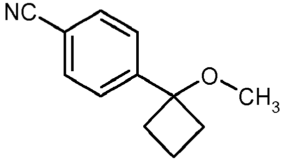

Eine Lösung von 2.0 g (11.5 mmol) der Verbindung aus Beispiel 7A / Schritt 1 in 40 ml wasserfreiem DMF wurde bei 0°C mit 508 mg (12.7 mmol) Natriumhydrid (60%-ige Suspension in Mineralöl) versetzt. Nach 1 h wurde mit 863 µl (13.9 mmol) Iodmethan versetzt und das Eis/ Wasser-Bad entfernt. Nach 16 h Rühren bei RT wurde das Reaktionsgemisch auf 120 ml Wasser gegossen und dreimal mit je ca. 100 ml Diethylether extrahiert. Die vereinigten organischen Extrakte wurden nacheinander mit Wasser und gesättigter Natriumchlorid-Lösung gewaschen, über wasserfreiem Magnesiumsulfat getrocknet, filtriert und schließlich am Rotationsverdampfer vom Lösungsmittel befreit. Der verbliebene Rückstand wurde mittels Saugfiltration über ca. 100 g Kieselgel mit Cyclohexan/Ethylacetat 20:1 → 4:1 als Laufmittel gereinigt. Es wurden 1.27 g (59% d. Th.) der Titelverbindung erhalten.
¹H-NMR (400 MHz, CDCl₃, δ/ppm): 7.68 (d, 2H), 7.54 (d, 2H), 2.95 (s, 3H), 2.46-2.32 (m, 4H), 2.03-1.93 (m, 1H), 1.76-1.63 (m, 1H).
MS (DCI, NH₃): m/z = 205 [M+NH₄]⁺.

### Schritt 3: N'-Hydroxy-4-(1-methoxycyclobutyl)benzolcarboximidamid

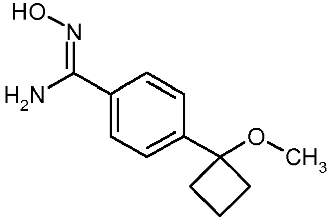

Analog zu dem unter Beispiel 3A / Schritt 5 beschriebenen Verfahren wurden aus 1.10 g (5.88 mmol) der Verbindung aus Beispiel 7A / Schritt 2 und 612 mg (8.81 mmol) Hydroxylammoniumchlorid 1.28 g (93% d. Th., 95% Reinheit) der Titelverbindung hergestellt. Die Reaktionszeit betrug in diesem Fall 16 h.
¹H-NMR (400 MHz, DMSO-d₆, δ/ppm): 9.62 (s, 1H), 7.68 (d, 2H), 7.40 (d, 2H), 5.80 (s, breit, 2H), 2.83 (s, 3H), 2.37-2.24 (m, 4H), 1.91-1.81 (m, 1H), 1.65-1.53 (m, 1H).
HPLC (Methode 10): Rₜ = 3.02 min.
MS (DCI, NH₃): m/z = 221 [M+H]⁺.

### Schritt 4: 3-[4-(1-Methoxycyclobutyl)phenyl]-5-(5-methyl-1H-pyrazol-3-yl)-1,2,4-oxadiazol

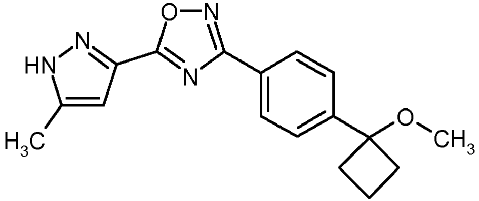

Zu einer Suspension von 491 mg (3.03 mmol) 1,1'-Carbonyldiimidazol (CDI) in 2 ml wasserfreiem DMF wurde bei RT innerhalb von 15 min eine Lösung von 364 mg (2.88 mmol) 5-Methyl-1*H-*pyrazol-3-carbonsäure in 2 ml wasserfreiem DMF hinzugetropft. Nachdem das Gemisch 2 h bei RT gerührt worden war, wurden 635 mg (2.88 mmol) der Verbindung aus Beispiel 7A / Schritt 3 hinzugefügt. Anschließend wurde das Reaktionsgemisch 4.5 h auf 110°C erhitzt. Nach dem Abkühlen auf RT wurde das Reaktionsgemisch langsam unter starkem Rühren in 60 ml Wasser eingerührt, wobei das Produkt ausfiel. Der Feststoff wurde abgesaugt und mit wenig kaltem Wasser gewaschen. Nach Trocknen im Hochvakuum wurden 640 mg (68% d. Th., 95% Reinheit) der Titelverbindung erhalten.
¹H-NMR (400 MHz, CDCl₃, δ/ppm): 10.85 (breit, 1H), 8.19 (d, 2H), 7.57 (d, 2H), 6.82 (s, 1H), 2.98 (s, 3H), 2.45 (s, 3H), 2.45-2.37 (m, 4H), 2.03-1.93 (m, 1H), 1.78-1.67 (m, 1H).
LC/MS (Methode 6, ESIpos): Rₜ = 1.06 min, m/z = 311 [M+H]⁺.

### Schritt 5: 5-[1-(3-Brombenzyl)-5-methyl-1H-pyrazol-3-yl]-3-[4-(1-methoxycyclobutyl)phenyl]-1,2,4-oxadiazol

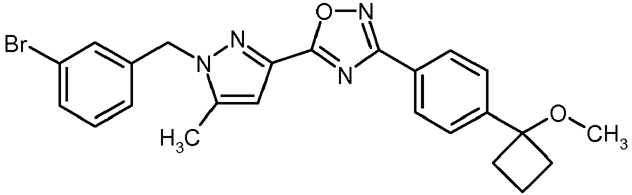

Analog zu dem unter Beispiel 1A / Schritt 2 beschriebenen Verfahren wurden aus 250 mg (0.806 mmol) der Verbindung aus Beispiel 7A / Schritt 4 und 242 mg (0.967 mmol) 3-Brombenzylbromid 326 mg (84% d. Th.) der Titelverbindung hergestellt. Auf ein Erwärmen der Reaktionsmischung auf 45°C konnte hier verzichtet werden.
¹H-NMR (400 MHz, CDCl₃, δ/ppm): 8.21 (d, 2H), 7.56 (d, 2H), 7.44 (d, 1H), 7.32 (s, 1H), 7.22 (t, 1H), 7.09 (d, 1H), 6.83 (s, 1H), 5.43 (s, 2H), 2.97 (s, 3H), 2.44-2.40 (m, 4H), 2.29 (s, 3H), 2.03-1.93 (m, 1H), 1.78-1.67 (m, 1H).
LC/MS (Methode 6, ESIpos): Rₜ = 1.44 min, m/z = 479/481 [M+H]⁺.

### Beispiel 8A

### 3- {[tert.-Butyl(diphenyl)silyl]oxy}azetidin

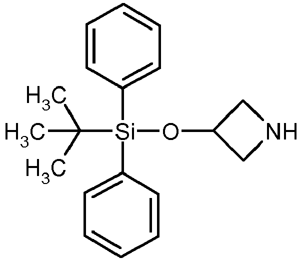

### Schritt 1: tert.-Butyl-3-{[tert.-butyl(diphenyl)silyl]oxy}azetidin-1-carboxylat

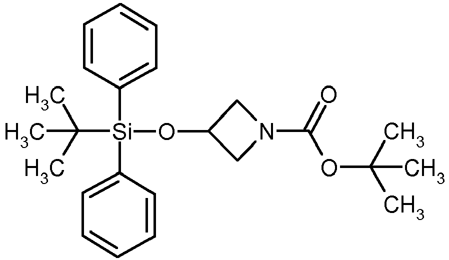

20.0 g (115 mmol) tert.-Butyl-3-hydroxyazetidin-1-carboxylat und 9.43 g (139 mmol) Imidazol wurden in 200 ml wasserfreiem DMF vorgelegt und bei RT mit 34.91 g (127 mmol) *tert*.-Butyl-(diphenyl)silylchlorid versetzt. Nachdem das Reaktionsgemisch 18 h bei RT gerührt worden war, wurde es in 3.2 Liter Wasser gegossen und anschließend dreimal mit je ca. 1 Liter Diethylether extrahiert. Die vereinigten organischen Extrakte wurden nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und das Lösungsmittel am Rotationsverdampfer entfernt. Der verbliebene Rückstand wurde mit 100 ml Pentan einige Minuten verrührt. Anschließend wurde der Feststoff abgesaugt und im Hochvakuum getrocknet. Es wurden 29.18 g (61% d. Th.) der Titelverbindung erhalten.
¹H-NMR (400 MHz, CDCl₃, δ/ppm): 7.60 (d, 4H), 7.46-7.37 (m, 6H), 4.53-4.49 (m, 1H), 3.93 (dd, 2H), 3.87 (dd, 2H), 1.41 (s, 9H), 1.04 (s, 9H).
LC/MS (Methode 6, ESIpos): Rₜ = 1.65 min, m/z = 412 [M+H]⁺.

### Schritt 2: 3-{[tert.-Butyl(diphenyl)silyl]oxy}azetidin

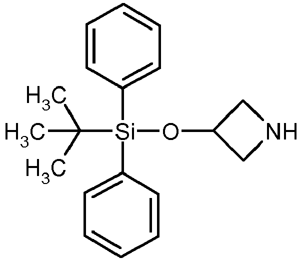

Eine Lösung von 20.0 g (48.6 mmol) der Verbindung aus Beispiel 8A / Schritt 1 in 70 ml Dichlormethan wurde bei RT tropfenweise mit 70 ml Trifluoressigsäure (TFA) versetzt. Nachdem das Reaktionsgemisch 30 min bei RT gerührt worden war, wurden alle flüchtigen Komponenten am Rotationsverdampfer entfernt. Der verbliebene Rückstand wurde mit 1 Liter 1 M Natronlauge versetzt, und es wurde dreimal mit je ca. 200 ml Dichlormethan extrahiert. Die vereinigten organischen Extrakte wurden über wasserfreiem Magnesiumsulfat getrocknet, filtriert und schließlich am Rotationsverdampfer zur Trockene eingedampft. Nach Trocknen des Rückstands im Hochvakuum wurden 14.85 g (98% d. Th.) der Titelverbindung erhalten.
¹H-NMR (400 MHz, CDCl₃, δ/ppm): 7.61 (d, 4H), 7.45-7.36 (m, 6H), 4.64-4.58 (m, 1H), 3.68 (dd, 2H), 3.53 (dd, 2H), 2.19 (breit, 1H), 1.03 (s, 9H).
LC/MS (Methode 6, ESIpos): Rₜ = 0.90 min, m/z = 312 [M+H]⁺.

### Beispiel 9A

### 4- {[tert.-Butyl(diphenyl)silyl]oxy}piperidin

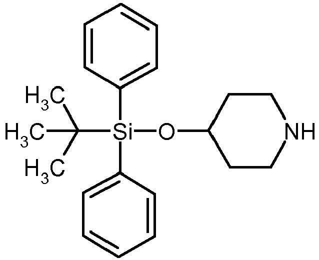

### Schritt 1: tert.-Butyl-4-{[tert.-butyl(diphenyl)silyl]oxy}piperidin-1-carboxylat

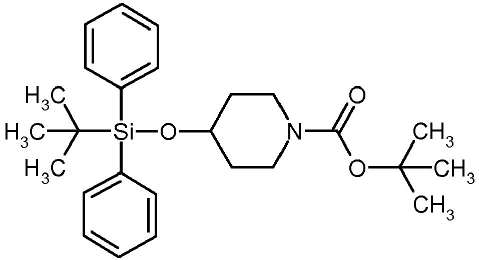

10.0 g (49.7 mmol) tert.-Butyl-4-hydroxypiperidin-1-carboxylat und 4.06 g (59.7 mmol) Imidazol wurden in 100 ml wasserfreiem DMF vorgelegt und bei 0°C mit 15.02 g (54.7 mmol) tert.-Butyl-(diphenyl)silylchlorid versetzt. Nachdem das Reaktionsgemisch 48 h bei RT gerührt worden war, wurde es in 1.6 Liter Wasser gegossen und anschließend dreimal mit je ca. 500 ml Diethylether extrahiert. Die vereinigten organischen Extrakte wurden nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und das Lösungsmittel am Rotationsverdampfer entfernt. Der verbliebene Rückstand wurde mittels Saugfiltration grob gereinigt (ca. 300 g Kieselgel, Laufmittel: Cyclohexan → Cyclohexan/Ethylacetat 2:1). Es wurden 22.21 g (91% d. Th. bei ca. 90% Reinheit) der Titelverbindung erhalten.
¹H-NMR (400 MHz, CDCl₃, δ/ppm): 7.67 (d, 4H), 7.43-7.37 (m, 6H), 3.93-3.87 (m, 1H), 3.68-3.60 (m, 2H), 3.22-3.14 (m, 2H), 1.63-1.48 (m, 4H), 1.43 (s, 9H), 1.07 (s, 9H).
LC/MS (Methode 6, ESIpos): Rₜ = 1.68 min, m/z = 440 [M+H]⁺.

### Schritt 2: 4-{[tert.-Butyl(diphenyl)silyl]oxy}piperidin

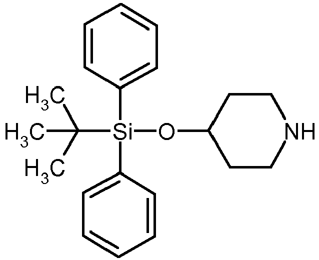

Analog zu dem unter Beispiel 8A / Schritt 2 beschriebenen Verfahren wurden aus 2.5 g (5.12 mmol, 90% Reinheit) der Verbindung aus Beispiel 9A / Schritt 1 1.45 g (83% d. Th.) der Titelverbindung erhalten. Das Produkt wurde in diesem Falle mittels MPLC gereinigt (ca. 50 g Kieselgel, Laufmittel: Ethylacetat → Ethylacetat/Triethylamin 9:1).
¹H-NMR (400 MHz, CDCl₃, δ/ppm): 7.68 (d, 4H), 7.45-7.35 (m, 6H), 3.83-3.77 (m, 1H), 3.07-3.01 (m, 2H), 2.52-2.47 (m, 2H), 1.72-1.66 (m, 2H), 1.53-1.45 (m, 2H), 1.07 (s, 9H).
LC/MS (Methode 14, ESIpos): Rₜ = 0.87 min, m/z = 340 [M+H]⁺.

### Beispiel 10A

### 5-[1-(3-Brombenzyl)-5-methyl-1H-pyrazol-3-yl]-3-{4-[(trifluormethyl)sulfanyl]phenyl}-1,2,4-oxadiazol

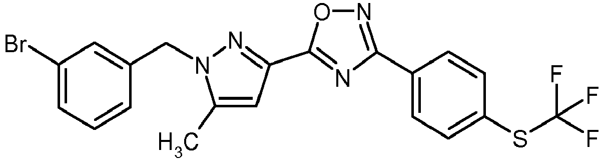

### Schritt 1: N'-Hydroxy-4-[(trifluormethyl)sulfanyl]benzolcarboximidamid

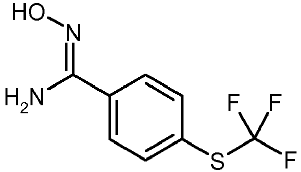

Eine Lösung von 113 g (500 mmol) 4-[(Trifluormethyl)sulfanyl]benzolcarbonitril und 147 ml (1.05 mol) Triethylamin in 1.4 Litern Ethanol wurde mit 73 g (1.05 mol) Hydroxylammoniumchlorid versetzt und anschließend 30 min unter Rückfluss erhitzt. Nach dem Abkühlen auf RT wurde mit 1 Liter Wasser versetzt und dreimal mit insgesamt 1.4 Litern Ethylacetat extrahiert. Die vereinigten organischen Extrakte wurden nacheinander mit Wasser und gesättigter Kochsalz-Lösung gewaschen. Nach Trocknen über wasserfreiem Magnesiumsulfat wurde filtriert und das Lösungsmittel am Rotationsverdampfer entfernt. Der erhaltene Rückstand wurde in 1 Liter Cyclohexan aufgenommen, mit 40 ml Diisopropylether versetzt und 20 min bei RT verrührt. Der resultierende Feststoff wurde abgesaugt, und nach Trocknen im Hochvakuum wurde eine erste Portion von 78.6 g der Titelverbindung erhalten. Das Filtrat wurde eingeengt und der Rückstand anschließend in einem Gemisch aus 100 ml Cyclohexan und 5 ml Ethylacetat 30 min lang in der Siedehitze verrührt. Nach Abkühlen wurde der Feststoff abgesaugt und im Hochvakuum getrocknet. Dies ergab eine zweite Portion von 4.2 g der Titelverbindung. Insgesamt wurden so 82.8 g (70% d. Th.) der Titelverbindung erhalten.
¹H-NMR (400 MHz, DMSO-d₆, δ/ppm): 9.90 (s, 1H), 7.80 (d, 2H), 7.72 (d, 2H), 5.94 (s, 2H).
LC/MS (Methode 2, ESIpos): Rₜ = 1.42 min, m/z = 237 [M+H]⁺.

### Schritt 2: 5-(5-Methyl-1H-pyrazol-3-yl)-3-{4-[(trifluormethyl)sulfanyl]phenyl}-1,2,4-oxadiazol

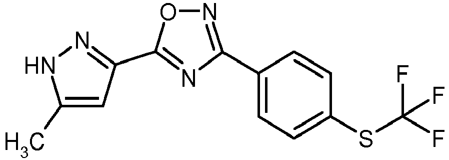

Zu einer Suspension von 63.45 g (391 mmol) 1,1'-Carbonyldiimidazol in 235 ml wasserfreiem DMF wurde bei RT innerhalb von 15 min eine Lösung von 47 g (373 mmol) 5-Methyl-1*H-*pyrazol-3-carbonsäure in 235 ml wasserfreiem DMF hinzugetropft. Nachdem das Gemisch 1 h 45 min bei RT gerührt worden war, wurden 88 g (373 mmol) der Verbindung aus Beispiel 10A / Schritt 1 hinzugefügt. Anschließend wurde das Reaktionsgemisch 4 h auf 110°C erhitzt. Nach dem Abkühlen auf RT wurden langsam unter starkem Rühren 3.5 Liter Wasser hinzugefügt, wodurch das Produkt ausfiel. Der Feststoff wurde abgesaugt und mit ca. 1 Liter kaltem Wasser gewaschen. Das Rohprodukt wurde durch Umkristallisation aus einem Lösungsmittelgemisch bestehend aus je 500 ml Acetonitril und Ethanol gereinigt. Nach Abkühlen, Filtration und Trocknen im Hochvakuum wurde eine erste Portion von 94.2 g der Titelverbindung erhalten. Aus der Mutterlauge wurden nach Eindampfen und erneuter Kristallisation aus 150 ml Ethanol weitere 6.2 g der Titelverbindung gewonnen. Insgesamt wurden so 100.4 g (83% d. Th.) der Titelverbindung erhalten.
¹H-NMR (400 MHz, CDCl₃, δ/ppm): 10.60 (breit, 1H), 8.23 (d, 2H), 7.79 (d, 2H), 6.81 (s, 1H), 2.47 (s, 3H).
LC/MS (Methode 6, ESIpos): Rₜ = 1.17 min, m/z = 327 [M+H]⁺.

### Schritt 3: 5-[1-(3-Brombenzyl)-5-methyl-1H-pyrazol-3-yl]-3-{4-[(trifluormethyl)sulfanyl]phenyl}-1,2,4-oxadiazol

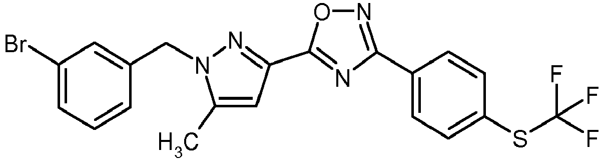

Eine Lösung von 5.0 g (15.3 mmol) der Verbindung aus Beispiel 10A / Schritt 2 und 4.60 g (18.4 mmol) 3-Brombenzylbromid in 150 ml wasserfreiem Dioxan wurde bei RT mit 2.24 g (19.9 mmol) festem Kalium-tert.-butylat versetzt. Das Reaktionsgemisch wurde 16 h bei RT gerührt. Anschließend wurden ca. 50-100 ml des Lösungsmittels am Rotationsverdampfer entfernt. Der verbliebene Rückstand wurde mit 900 ml Ethylacetat verdünnt und dreimal mit je 200 ml Wasser und abschließend einmal mit 200 ml gesättigter Kochsalz-Lösung gewaschen. Nach Trocknen über wasserfreiem Magnesiumsulfat und Filtration wurde das Lösungsmittel am Rotationsverdampfer entfernt. Das erhaltene Rohprodukt wurde aus Cyclohexan umkristallisiert. Nach Absaugen und Trocknen des Feststoffs wurden 5.03 g (66% d. Th.) der Titelverbindung erhalten.
¹H-NMR (400 MHz, CDCl₃, δ/ppm): 8.27 (d, 2H), 7.78 (d, 2H), 7.44 (d, 1H), 7.32 (s, 1H), 7.22 (t, 1H), 7.09 (d, 1H), 6.84 (s, 1H), 5.43 (s, 2H), 2.29 (s, 3H).
LC/MS (Methode 6, ESIpos): Rₜ = 1.49 min, m/z = 495/497 [M+H]⁺.

### Beispiel 11A

### (S)-1-{3-[(5-Methyl-3-{3-[4-(trifluormethoxy)phenyl]-1,2,4-oxadiazol-5-yl}-1H-pyrazol-1-yl)-methyl]phenyl}azetidin-3-yl-N-(tert.-butoxycarbonyl)-L-valinat

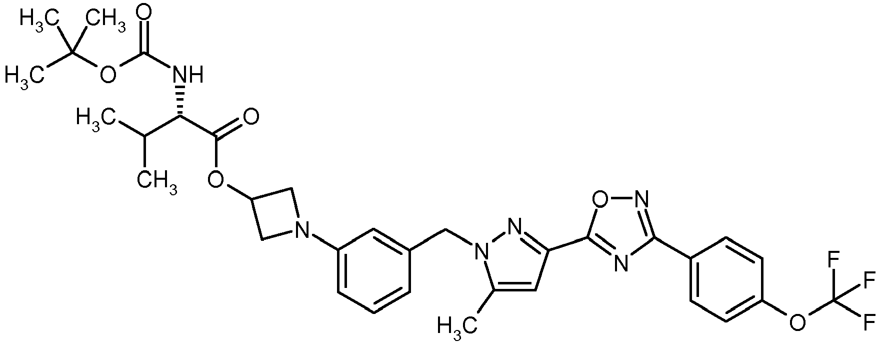

Eine Lösung von 750 mg (1.59 mmol) der Verbindung aus Beispiel 1 (siehe Abschnitt zu den Ausführungsbeispielen) und 691 mg (3.18 mmol) *(S)*-*N-*(*tert*.-Butoxycarbonyl)-L-valin in 30 ml Dichlormethan wurde mit 117 mg (0.955 mmol) 4-*N*,*N* Dimethylaminopyridin und 915 mg (4.77 mmol) EDC versetzt. Nachdem das Reaktionsgemisch 2 h bei RT gerührt worden war, wurde es mit 100 ml Dichlormethan verdünnt und zweimal mit je 100 ml Wasser und einmal mit 100 ml gesättigter Kochsalz-Lödung gewaschen. Nach Trocknen über wasserfreiem Magnesiumsulfat, Filtration und Einengen wurde das Produkt mittels MPLC isoliert (ca. 100 g Kieselgel, Laufmittel: Cyclohexan/Ethylacetat 4:1 → 3:1). Es wurden 671 mg (90% d. Th.) der Titelverbindung erhalten.
¹H-NMR (400 MHz, CDCl₃, δ/ppm): 8.27 (d, 2H), 7.33 (d, 2H), 7.19 (t, 1H), 6.81 (s, 1H), 6.56 (d, 1H), 6.38 (d, 1H), 6.24 (s, 1H), 5.39 (s, 2H), 5.37-5.31 (m, 1H), 4.95 (d, breit, 1H), 4.24-4.18 (m, 3H), 3.83-3.73 (m, 2H), 2.28 (s, 3H), 2.20-2.11 (m, 1H), 1.43 (s, 9H), 0.98 (d, 3H), 0.90 (d, 3H).
LC/MS (Methode 14, ESIpos): Rₜ = 1.53 min, m/z = 671 [M+H]⁺.

### Ausführungsbeispiele:

### Beispiel 1

### 1-{3-[(5-Methyl-3-{3-[4-(trifluormethoxy)phenyl]-1,2,4-oxadiazol-5-yl}-1H-pyrazol-1-yl)methyl]-phenyl} azetidin-3-ol

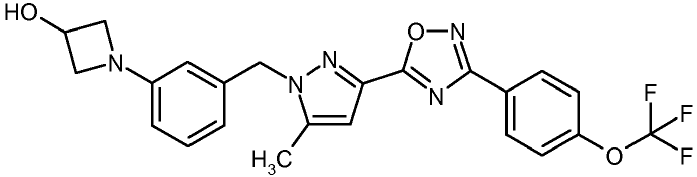

Ein Gemisch aus 2.0 g (4.17 mmol) der Verbindung aus Beispiel 1A, 1.95 g (6.26 mmol) der Verbindung aus Beispiel 8A, 256 mg (0.280 mmol) Tris(dibenzylidenaceton)dipalladium(0), 398 mg (0.835 mmol) 2-Dicyclohexylphosphino-2',4',6'-triisopropylbiphenyl (X-Phos) und 2.72 g (8.35 mmol) Cäsiumcarbonat in 20 ml wasserfreiem DMF wurde unter Argon in einem Mikrowellenofen (Biotage Initiator, dynamische Steuerung der Einstrahlleistung) 2.5 h lang auf 120°C erhitzt. Nach dem Abkühlen auf RT wurde das Reaktionsgemisch mit 100 ml Wasser versetzt, und es wurde dreimal mit je ca. 100 ml Ethylacetat extrahiert. Die vereinigten organischen Extrakte wurden nacheinander mit Wasser und gesättigter Natriumchlorid-Lösung gewaschen, über wasserfreiem Magnesiumsulfat getrocknet, filtriert und schließlich am Rotationsverdampfer vom Lösungsmittel befreit. Der verbliebene Rückstand wurde in 40 ml THF gelöst und mit 6.3 ml (6.26 mmol) einer 1 M Lösung von Tetra-*n*-butylammoniumfluorid (TBAF) in THF versetzt. Nachdem dieses Gemisch 1 h bei RT gerührt worden war, wurde auf die gleiche Weise wässrig aufgearbeitet wie oben beschrieben. Der verbliebene Rückstand wurde durch Saugfiltration über 200 g Kieselgel mit Cyclohexan/Ethylacetat 2:1 (3 Liter) → 1:1 (3 Liter) als Laufmittel gereinigt. Nach Eindampfen und Trocknen im Hochvakuum wurden 1.56 g (79% d. Th.) der Titelverbindung erhalten.
¹H-NMR (400 MHz, CDCl₃, δ/ppm): 8.25 (d, 2H), 7.33 (d, 2H), 7.17 (t, 1H), 6.80 (s, 1H), 6.53 (d, 1H), 6.39 (d, 1H), 6.27 (s, 1H), 5.38 (s, 2H), 4.77-4.70 (m, 1H), 4.13 (dd, 2H), 3.63 (dd, 2H), 2.27 (s, 3H), 2.12 (d, 1H).
LC/MS (Methode 6, ESIpos): Rₜ = 1.20 min, m/z = 472 [M+H]⁺.

### Beispiel 2

### 1-(3-{[5-Methyl-3-(3-{4-[1-(trifluormethyl)cyclopropyl]phenyl}-1,2,4-oxadiazol-5-yl)-1H-pyrazol-1-yl]methyl}phenyl)azetidin-3-ol

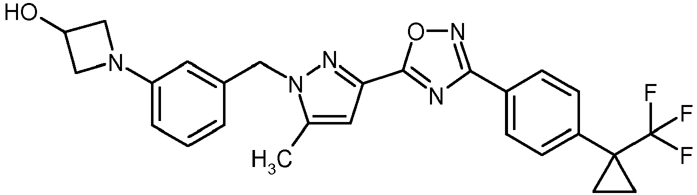

Analog zu dem unter Beispiel 1 beschriebenen Verfahren wurden aus 500 mg (0.993 mmol) der Verbindung aus Beispiel 2A und 464 mg (1.49 mmol) der Verbindung aus Beispiel 8A 289 mg (58% d. Th.) der Titelverbindung erhalten. Auf den zweiten Teilschritt des Verfahrens (Silylether-Spaltung mit TBAF) konnte hier verzichtet werden, da die Schutzgruppe bereits im Verlauf des ersten Teilschritts abgespalten wurde. Die Reinigung des Rohproduktes erfolgte mittels MPLC (Kieselgel, Laufmittel: Cyclohexan/Ethylacetat 1:1) und anschließendem Ausrühren in einem Gemisch aus 25 ml Pentan, 1 ml Diisopropylether und 125 µl Dichlormethan.
¹H-NMR (400 MHz, CDCl₃, δ/ppm): 8.19 (d, 2H), 7.58 (d, 2H), 7.17 (t, 1H), 6.80 (s, 1H), 6.53 (d, 1H), 6.39 (d, 1H), 6.27 (s, 1H), 5.38 (s, 2H), 4.77-4.70 (m, 1H), 4.13 (dd, 2H), 3.63 (dd, 2H), 2.27 (s, 3H), 2.01 (d, 1H), 1.42-1.39 (m, 2H), 1.10-1.07 (m, 2H).
LC/MS (Methode 6, ESIpos): Rₜ = 1.26 min, m/z = 496 [M+H]⁺.

### Beispiel 3

### 1-{3-[(5-Methyl-3-{3-[4-(1,1,1-trifluor-2-methylpropan-2-yl)phenyl]-1,2,4-oxadiazol-5-yl}-1H-pyrazol-1-yl)methyl]phenyl}azetidin-3-ol

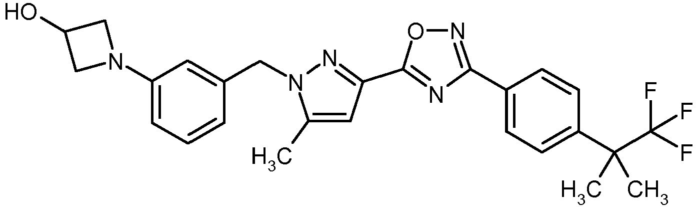

Analog zu dem unter Beispiel 1 beschriebenen Verfahren wurden aus 150 mg (0.297 mmol) der Verbindung aus Beispiel 3A und 139 mg (0.445 mmol) der Verbindung aus Beispiel 8A 68 mg (46% d. Th.) der Titelverbindung erhalten. Die Reaktionszeit im ersten Teilschritt des Verfahrens betrug hier 1 h. Die Aufreinigung des Rohprodukts erfolgte mittels präparativer HPLC (Methode 13). Aus dem dabei angefallenen Formiat-Salz der Titelverbindung wurde die freie Base durch Perkolation einer methanolischen Lösung des Salzes über eine Hydrogencarbonat-Kartusche erhalten (Fa. Polymerlabs, Stratospheres SPE, PL-HCO₃ MP SPE, Kapazität 0.9 mmol). Die abschließende Reinigung erfolgte durch Ausrühren in einem Gemisch aus 3 ml Pentan und einigen Tropfen Diisopropylether.
¹H-NMR (400 MHz, CDCl₃, δ/ppm): 8.20 (d, 2H), 7.63 (d, 2H), 7.17 (t, 1H), 6.80 (s, 1H), 6.53 (d, 1H), 6.39 (d, 1H), 6.27 (s, 1H), 5.38 (s, 2H), 4.77-4.70 (m, 1H), 4.13 (dd, 2H), 3.63 (dd, 2H), 2.27 (s, 3H), 2.18 (d, 1H), 1.62 (s, 6H).
LC/MS (Methode 6, ESIpos): Rₜ = 1.27 min, m/z = 498 [M+H]⁺.

### Beispiel 4

### 1-{3-[(5-Methyl-3-{3-[4-(tetrahydro-2H-pyran-4-yl)phenyl]-1,2,4-oxadiazol-5-yl}-1H-pyrazol-1-yl)methyl]phenyl}azetidin-3-ol

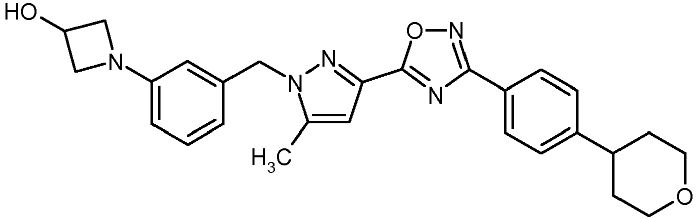

Analog zu dem unter Beispiel 3 beschriebenen Verfahren wurden aus 150 mg (0.313 mmol) der Verbindung aus Beispiel 4A und 146 mg (0.469 mmol) der Verbindung aus Beispiel 8A 77 mg (52% d. Th.) der Titelverbindung erhalten.
¹H-NMR (400 MHz, CDCl₃, δ/ppm): 8.17 (d, 2H), 7.36 (d, 2H), 7.17 (t, 1H), 6.80 (s, 1H), 6.53 (d, 1H), 6.39 (d, 1H), 6.27 (s, 1H), 5.38 (s, 2H), 4.77-4.70 (m, 1H), 4.15-4.07 (m, 4H), 3.63 (dd, 2H), 3.55 (dt, 2H), 2.87-2.78 (m, 1H), 2.26 (s, breit, 4H), 1.91-1.78 (m, 4H).
LC/MS (Methode 4, ESIpos): Rₜ = 1.27 min, m/z = 472 [M+H]⁺.

### Beispiel 5

### 1-{3-[(3-{3-[4-(4-Fluortetrahydro-2H-pyran-4-yl)phenyl]-1,2,4-oxadiazol-5-yl}-5-methyl-1H pyrazol-1-yl)methyl]phenyl}azetidin-3-ol

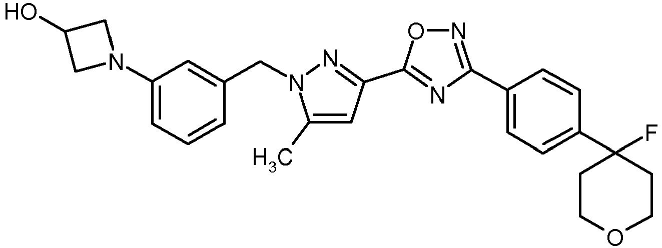

Analog zu dem unter Beispiel 3 beschriebenen Verfahren wurden aus 150 mg (0.302 mmol) der Verbindung aus Beispiel 5A und 141 mg (0.452 mmol) der Verbindung aus Beispiel 8A 62 mg (42% d. Th.) der Titelverbindung erhalten.
¹H-NMR (400 MHz, CDCl₃, δ/ppm): 8.23 (d, 2H), 7.53 (d, 2H), 7.17 (t, 1H), 6.80 (s, 1H), 6.53 (d, 1H), 6.39 (d, 1H), 6.27 (s, 1H), 5.38 (s, 2H), 4.77-4.70 (m, 1H), 4.13 (dd, 2H), 4.00-3.87 (m, 4H), 3.63 (dd, 2H), 2.27 (d, 1H), 2.26 (s, 3H), 2.29-2.12 (m, 2H), 1.99-1.90 (m, 2H).
LC/MS (Methode 4, ESIpos): Rₜ = 1.28 min, m/z = 490 [M+H]⁺.

### Beispiel 6

### 1-{3-[(3-{3-[4-(3-Fluoroxetan-3-yl)phenyl]-1,2,4-oxadiazol-5-yl}-5-methyl-1H-pyrazol-1-yl)-methyl]phenyl}azetidin-3-ol

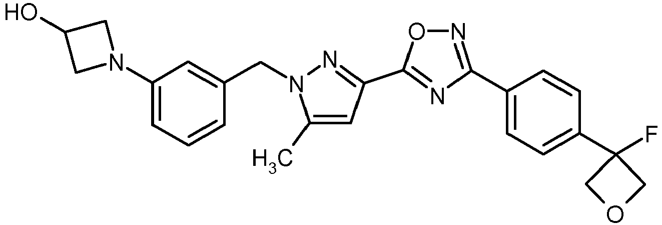

Analog zu dem unter Beispiel 3 beschriebenen Verfahren wurden aus 150 mg (0.320 mmol) der Verbindung aus Beispiel 6A und 149 mg (0.479 mmol) der Verbindung aus Beispiel 8A 60 mg (39% d. Th.) der Titelverbindung erhalten.
¹H-NMR (400 MHz, CDCl₃, δ/ppm): 8.29 (d, 2H), 7.71 (d, 2H), 7.17 (t, 1H), 6.81 (s, 1H), 6.53 (d, 1H), 6.39 (d, 1H), 6.27 (s, 1H), 5.39 (s, 2H), 5.06 (dd, 2H), 5.00 (dd, 2H), 4.77-4.70 (m, 1H), 4.13 (dd, 2H), 3.63 (dd, 2H), 2.29 (d, 1H), 2.27 (s, 3H).
LC/MS (Methode 4, ESIpos): Rₜ = 1.22 min, m/z = 462 [M+H]⁺.

### Beispiel 7

### 1-{3-[(3-{3-[4-(1-Methoxycyclobutyl)phenyl]-1,2,4-oxadiazol-5-yl}-5-methyl-1H-pyrazol-1-yl)-methyl]phenyl}azetidin-3-ol

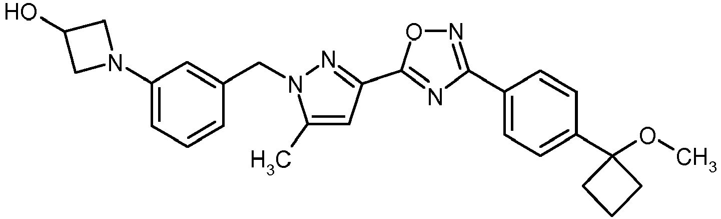

Analog zu dem unter Beispiel 3 beschriebenen Verfahren wurden aus 150 mg (0.313 mmol) der Verbindung aus Beispiel 7A und 146 mg (0.469 mmol) der Verbindung aus Beispiel 8A 53 mg (36% d. Th.) der Titelverbindung erhalten.
¹H-NMR (400 MHz, CDCl₃, δ/ppm): 8.22 (d, 2H), 7.56 (d, 2H), 7.17 (t, 1H), 6.80 (s, 1H), 6.53 (d, 1H), 6.39 (d, 1H), 6.27 (s, 1H), 5.38 (s, 2H), 4.77-4.70 (m, 1H), 4.13 (dd, 2H), 3.63 (dd, 2H), 2.97 (s, 3H), 2.46-2.39 (m, 4H), 2.27 (s, 3H), 2.21 (d, 1H), 2.03-1.93 (m, 1H), 1.78-1.67 (m, 1H).
LC/MS (Methode 6, ESIpos): Rₜ = 1.21 min, m/z = 472 [M+H]⁺.

### Beispiel 8

### 1-{3-[(5-Methyl-3-{3-[4-(trifluormethoxy)phenyl]-1,2,4-oxadiazol-5-yl}-1H-pyrazol-1-yl)methyl]-phenyl}piperidin-4-ol

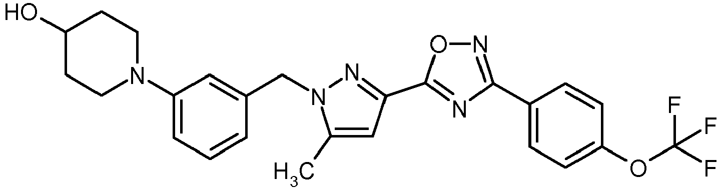

Ein Gemisch aus 150 mg (0.313 mmol) der Verbindung aus Beispiel 1A, 63 mg (0.626 mmol) 4-Hydroxypiperidin, 19 mg (0.021 mmol) Tris(dibenzylidenaceton)dipalladium(0), 30 mg (0.063 mmol) 2-Dicyclohexylphosphino-2',4',6'-triisopropylbiphenyl (X-Phos) und 254 mg (0.782 mmol) Cäsiumcarbonat in 3 ml wasserfreiem DMF wurde unter Argon 12 h lang bei 80°C gerührt. Nach dem Abkühlen auf RT wurde das Reaktionsgemisch über wenig Celite filtriert und das Filtrat anschließend mittels präparativer HPLC (Methode 11) in seine Komponenten aufgetrennt. Nach Eindampfen der Produktfraktionen und Trocknen im Hochvakuum wurden 36 mg (23% d. Th.) der Titelverbindung erhalten.
¹H-NMR (400 MHz, CDCl₃, δ/ppm): 8.27 (d, 2H), 7.34 (d, 2H), 7.21 (t, 1H), 6.86 (d, 1H), 6.81 (s, 1H), 6.75 (s, 1H), 6.61 (d, 1H), 5.40 (s, 2H), 3.87-3.81 (m, 1H), 3.53-3.49 (m, 2H), 2.90 (dt, 2H), 2.28 (s, 3H), 2.01-1.95 (m, 2H), 1.70-1.61 (m, 3H).
LC/MS (Methode 6, ESIpos): Rₜ = 1.18 min, m/z = 500 [M+H]⁺.

### Beispiel 9

### 1-(3-{[5-Methyl-3-(3-{4-[1-(trifluormethyl)cyclopropyl]phenyl}-1,2,4-oxadiazol-5-yl)-1H-pyrazol-1-yl]methyl}phenyl)piperidin-4-ol

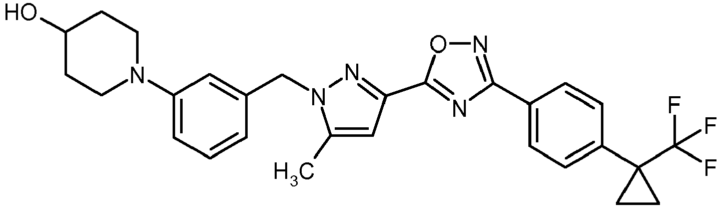

Analog zu dem unter Beispiel 3 beschriebenen Verfahren wurden aus 125 mg (0.248 mmol) der Verbindung aus Beispiel 2A und 50 mg (0.497 mmol) 4-Hydroxypiperidin 20 mg (16% d. Th.) der Titelverbindung erhalten. Der erste Teilschritt des Verfahrens erfolgte hier bei einer Reaktionstemperatur von 130°C. Die präparative HPLC-Reinigung wurde nach Methode 11 durchgeführt.
¹H-NMR (400 MHz, CDCl₃, δ/ppm): 8.18 (d, 2H), 7.59 (d, 2H), 7.20 (t, 1H), 6.86 (d, 1H), 6.80 (s, 1H), 6.75 (s, 1H), 6.61 (d, 1H), 5.40 (s, 2H), 3.87-3.81 (m, 1H), 3.53-3.49 (m, 2H), 2.90 (dt, 2H), 2.27 (s, 3H), 2.01-1.94 (m, 2H), 1.70-1.60 (m, 3H), 1.43-1.39 (m, 2H), 1.10-1.07 (m, 2H).
LC/MS (Methode 4, ESIpos): Rₜ = 1.39 min, m/z = 524 [M+H]⁺.

### Beispiel 10

### 1-{3-[(5-Methyl-3-{3-[4-(1,1,1-trifluor-2-methylpropan-2-yl)phenyl]-1,2,4-oxadiazol-5-yl}-1H-pyrazol-1-yl)methyl]phenyl}piperidin-4-ol

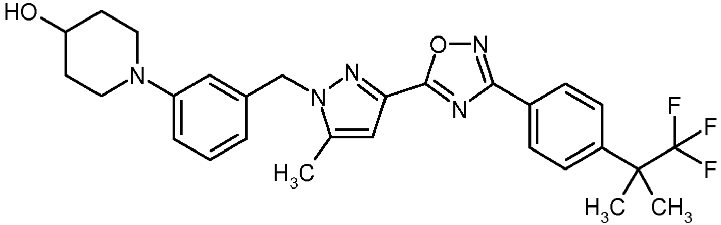

Analog zu dem unter Beispiel 9 beschriebenen Verfahren wurden aus 125 mg (0.247 mmol) der Verbindung aus Beispiel 3A und 50 mg (0.495 mmol) 4-Hydroxypiperidin 16 mg (12% d. Th.) der Titelverbindung erhalten.
¹H-NMR (400 MHz, CDCl₃, δ/ppm): 8.20 (d, 2H), 7.62 (d, 2H), 7.20 (t, 1H), 6.86 (d, 1H), 6.80 (s, 1H), 6.75 (s, 1H), 6.61 (d, 1H), 5.40 (s, 2H), 3.88-3.80 (m, 1H), 3.54-3.48 (m, 2H), 2.90 (dt, 2H), 2.27 (s, 3H), 2.01-1.94 (m, 2H), 1.69-1.64 (m, 2H), 1.62 (s, 6H), 1.43 (d, 1H).
LC/MS (Methode 6, ESIpos): Rₜ = 1.20 min, m/z = 526 [M+H]⁺.

### Beispiel 11

### 1-{3-[(5-Methyl-3-{3-[4-(trifluormethoxy)phenyl]-1,2,4-oxadiazol-5-yl}-1H-pyrazol-1-yl)methyl]-phenyl}piperidin-4-carbonitril

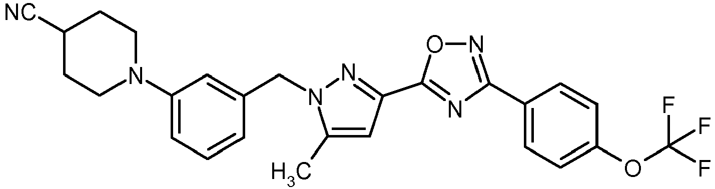

Analog zu dem unter Beispiel 8 beschriebenen Verfahren wurden aus 150 mg (0.313 mmol) der Verbindung aus Beispiel 1A und 69 mg (0.626 mmol) 4-Cyanopiperidin 68 mg (43% d. Th.) der Titelverbindung erhalten.
¹H-NMR (400 MHz, CDCl₃, δ/ppm): 8.26 (d, 2H), 7.34 (d, 2H), 7.23 (t, 1H), 6.86 (d, 1H), 6.81 (s, 1H), 6.74 (s, 1H), 6.67 (d, 1H), 5.40 (s, 2H), 3.41-3.34 (m, 2H), 3.11-3.03 (m, 2H), 2.81-2.75 (m, 1H), 2.28 (s, 3H), 2.07-1.92 (m, 4H).
LC/MS (Methode 2, ESIpos): Rₜ = 2.79 min, m/z = 509 [M+H]⁺.

### Beispiel 12

### 1-(3-{[5-Methyl-3-(3-{4-[1-(trifluormethyl)cyclopropyl]phenyl}-1,2,4-oxadiazol-5-yl)-1H-pyrazol-1-yl]methyl}phenyl)piperidin-4-carbonitril

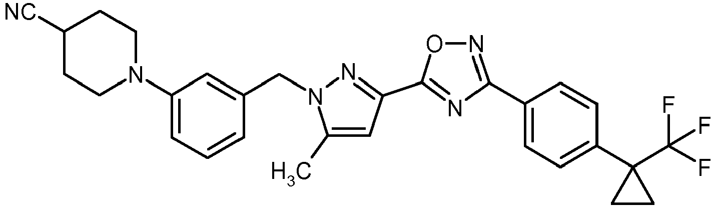

Analog zu dem unter Beispiel 9 beschriebenen Verfahren wurden aus 50 mg (0.099 mmol) der Verbindung aus Beispiel 2A und 22 mg (0.199 mmol) 4-Cyanopiperidin 23 mg (43% d. Th.) der Titelverbindung erhalten.
¹H-NMR (400 MHz, CDCl₃, δ/ppm): 8.19 (d, 2H), 7.58 (d, 2H), 7.22 (t, 1H), 6.84 (d, 1H), 6.81 (s, 1H), 6.73 (s, 1H), 6.67 (d, 1H), 5.40 (s, 2H), 3.41-3.34 (m, 2H), 3.10-3.03 (m, 2H), 2.80-2.74 (m, 1H), 2.28 (s, 3H), 2.07-1.92 (m, 4H), 1.42-1.39 (m, 2H), 1.11-1.07 (m, 2H).
LC/MS (Methode 6, ESIpos): Rₜ = 1.37 min, m/z = 533 [M+H]⁺.

### Beispiel 13

### 1-{3-[(5-Methyl-3-{3-[4-(1,1,1-trifluor-2-methylpropan-2-yl)phenyl]-1,2,4-oxadiazol-5-yl}-1H-pyrazol-1-yl)methyl]phenyl}piperidin-4-carbonitril

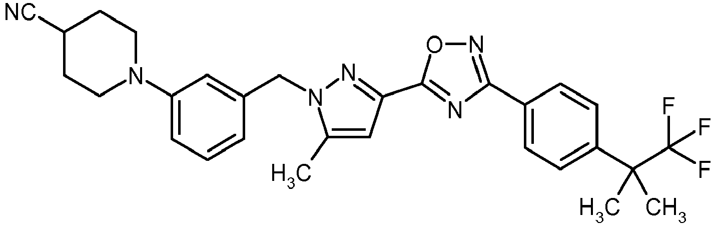

Analog zu dem unter Beispiel 9 beschriebenen Verfahren wurden aus 125 mg (0.247 mmol) der Verbindung aus Beispiel 3A und 55 mg (0.495 mmol) 4-Cyanopiperidin 66 mg (50% d. Th.) der Titelverbindung erhalten.
¹H-NMR (400 MHz, CDCl₃, δ/ppm): 8.20 (d, 2H), 7.62 (d, 2H), 7.22 (t, 1H), 6.84 (d, 1H), 6.81 (s, 1H), 6.73 (s, 1H), 6.67 (d, 1H), 5.40 (s, 2H), 3.40-3.34 (m, 2H), 3.10-3.03 (m, 2H), 2.80-2.74 (m, 1H), 2.28 (s, 3H), 2.07-1.91 (m, 4H), 1.63 (s, 6H).
LC/MS (Methode 6, ESIpos): Rₜ = 1.38 min, m/z = 535 [M+H]⁺.

### Beispiel 14

### 1-(3-{[5-Methyl-3-(3-{4-[(trifluormethyl)sulfanyl]phenyl}-1,2,4-oxadiazol-5-yl)-1H-pyrazol-1-yl]-methyl}phenyl)piperidin-4-ol

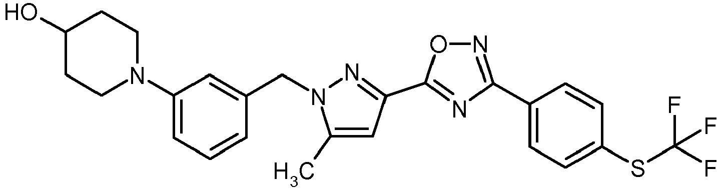

Ein Gemisch aus 500 mg (1.01 mmol) der Verbindung aus Beispiel 10A, 514 mg (1.51 mmol) der Verbindung aus Beispiel 9A, 92 mg (0.101 mmol) Tris(dibenzylidenaceton)dipalladium(0), 96 mg (0.202 mmol) 2-Dicyclohexylphosphino-2',4',6'-triisopropylbiphenyl (X-Phos) und 194 mg (2.02 mmol) Natrium-tert.-butylat in 10 ml Toluol wurde unter Argon in einem Mikrowellenofen (Biotage Initiator, dynamische Steuerung der Einstrahlleistung) 3 h lang auf 80°C erhitzt. Nach dem Abkühlen auf RT wurde das Reaktionsgemisch mit 100 ml Wasser versetzt, und es wurde dreimal mit je ca. 100 ml Ethylacetat extrahiert. Die vereinigten organischen Extrakte wurden nacheinander mit Wasser und gesättigter Natriumchlorid-Lösung gewaschen, über wasserfreiem Magnesiumsulfat getrocknet, filtriert und schließlich am Rotationsverdampfer vom Lösungsmittel befreit. Das erhaltene Rohprodukt, bei dem es sich um die entsprechende Silyl-geschützte Zwischenstufe handelte, wurde mittels MPLC gereinigt (ca. 70 g Kieselgel, Laufmittel: Cyclohexan → Cyclohexan/Ethylacetat 5:1). Anschließend wurde dieses Produkt in 3.5 ml THF gelöst und mit 3.5 ml (3.5 mmol) einer 1 M Lösung von Tetra-*n*-butylammoniumfluorid (TBAF) in THF versetzt. Nachdem dieses Gemisch 20 min bei RT gerührt worden war, wurde es am Rotationsverdampfer eingeengt. Die Isolierung der Titelverbindung erfolgte mittels MPLC (ca. 20 g Kieselgel, Laufmittel: Cyclohexan/Ethylacetat 2:1 → 1:3). Nach Eindampfen und Trocknen wurde das Produkt nochmals mit Pentan/Diethylether verrührt. Es wurden so 65 mg (12% d. Th.) der Titelverbindung erhalten.
¹H-NMR (400 MHz, CDCl₃, δ/ppm): 8.27 (d, 2H), 7.78 (d, 2H), 7.21 (t, 1H), 6.87 (d, 1H), 6.81 (s, 1H), 6.75 (s, 1H), 6.61 (d, 1H), 5.40 (s, 2H), 3.88-3.81 (m, 1H), 3.54-3.49 (m, 2H), 2.90 (dt, 2H), 2.29 (s, 3H), 2.01-1.94 (m, 2H), 1.69-1.61 (m, 3H).
LC/MS (Methode 6, ESIpos): Rₜ = 1.25 min, m/z = 516 [M+H]⁺.

### Beispiel 15

### 1-{3-[(5-Methyl-3-{3-[4-(trifluormethoxy)phenyl]-1,2,4-oxadiazol-5-yl}-1H-pyrazol-1-yl)methyl]-phenyl}azetidin-3-yl-N,N-dimethylglycinat

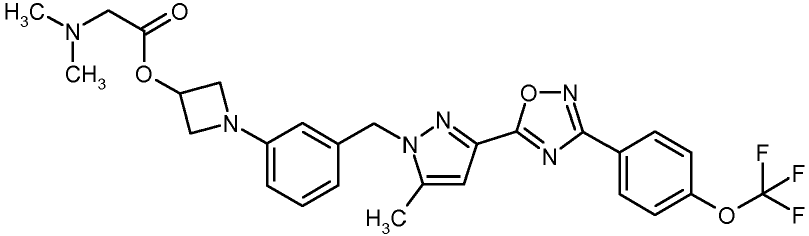

Eine Lösung von 550 mg (1.17 mmol) der Verbindung aus Beispiel 1 und 361 mg (3.50 mmol) *N,N-*Dimethylglycin in 11 ml Dichlormethan wurde mit 86 mg (0.70 mmol) 4-*N,N*-Dimethylaminopyridin und 671 mg (3.50 mmol) EDC versetzt. Nachdem das Reaktionsgemisch 8 h bei RT gerührt worden war, wurden alle flüchtigen Bestandteile am Rotationsverdampfer entfernt. Der erhaltene Rückstand wurde in 200 ml Ethylacetat aufgenommen und einmal mit ca. 100 ml Wasser gewaschen. Nach Trocknen über wasserfreiem Magnesiumsulfat, Filtration und Einengen wurde das Produkt mittels MPLC isoliert (ca. 30 g Kieselgel, Laufmittel: Cyclohexan/Ethylacetat/Triethylamin 25:25:1). Es wurde eine erste Fraktion von 328 mg der Titelverbindung erhalten. Aus einer zweiten, noch verunreinigten Fraktion wurden weitere 115 mg der Titelverbindung durch Umkristallisation aus Cyclopentylmethylether gewonnen. Insgesamt wurden so 443 mg (68% d. Th.) der Titelverbindung erhalten. Eine erneute Kristallisation aus Diisopropylether/Cyclopentylmethylether (2:1, 1.5 ml pro 100 mg) ergab die Titelverbindung mit einem Schmelzpunkt von 117°C.
¹H-NMR (400 MHz, CDCl₃, δ/ppm): 8.26 (d, 2H), 7.33 (d, 2H), 7.18 (t, 1H), 6.81 (s, 1H), 6.55 (d, 1H), 6.39 (d, 1H), 6.24 (s, 1H), 5.39 (s, 2H), 5.38-5.32 (m, 1H), 4.21 (dd, 2H), 3.79 (dd, 2H), 3.20 (s, 2H), 2.36 (s, 6H), 2.28 (s, 3H).
LC/MS (Methode 6, ESIpos): Rₜ = 0.95 min, m/z = 557 [M+H]⁺.

### Beispiel 16

### (S)-1-{3-[(5-Methyl-3-{3-[4-(trifluormethoxy)phenyl]-1,2,4-oxadiazol-5-yl}-1H-pyrazol-1-yl)-methyl]phenyl}azetidin-3-yl-L-valinat

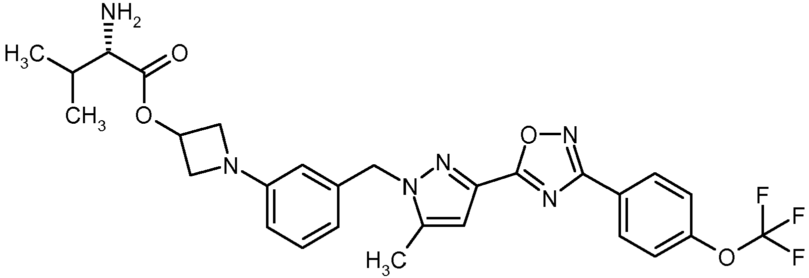

Eine Lösung von 965 mg (1.44 mmol) der Verbindung aus Beispiel 11A in 45 ml Dichlormethan wurde bei 0°C mit 25 ml Trifluoressigsäure (TFA) versetzt. Das Reaktionsgemisch wurde 1 h bei RT gerührt. Danach wurden alle flüchtigen Bestandteile am Rotationsverdampfer entfernt und der erhaltene Rückstand erneut in 500 ml Dichlormethan gelöst. Es wurde zweimal mit je ca. 100 ml gesättigter Natriumhydrogencarbonat-Lösung gewaschen, dann einmal mit 100 ml Wasser und abschließend einmal mit 100 ml gesättigter Kochsalz-Lösung. Nach Trocknen über wasserfreiem Magnesiumsulfat wurde filtriert und das Lösungsmittel am Rotationsverdampfer entfernt. Das erhaltene Rohprodukt (780 mg) wurde in einem Gemisch aus 40 ml Pentan, 2 ml Diisopropylether und einigen Tropfen Dichlormethan verrührt. Nach Absaugen des Feststoffs und Trocknen im Hochvakuum wurden so 659 mg (80% d. Th.) der Titelverbindung erhalten.
¹H-NMR (400 MHz, CDCl₃, δ/ppm): 8.27 (d, 2H), 7.33 (d, 2H), 7.19 (t, 1H), 6.80 (s, 1H), 6.57 (d, 1H), 6.39 (d, 1H), 6.25 (s, 1H), 5.39 (s, 2H), 5.35-5.30 (m, 1H), 4.24-4.20 (m, 2H), 3.80-3.74 (m, 2H), 3.32 (d, 1H), 2.28 (s, 3H), 2.12-2.00 (m, 1H), 0.98 (d, 3H), 0.91 (d, 3H).
LC/MS (Methode 14, ESIpos): Rₜ = 0.96 min, m/z = 571 [M+H]⁺.

### B. Bewertung der pharmakologischen Wirksamkeit

Die pharmakologische Aktivität der erfindungsgemäßen Verbindungen kann durch *in vitro-* und *in vivo*-Untersuchungen, wie sie dem Fachmann bekannt sind, nachgewiesen werden. Die Nützlichkeit der erfindungsgemäßen Substanzen kann beispielhaft illustriert werden durch *in vitro-*(Tumor-)Zellversuche und *in vivo*-Tumormodelle, wie sie weiter unten aufgeführt sind. Der Zusammenhang zwischen einer Hemmung der HIF-Transkriptionsaktivität und der Hemmung von Tumor-wachstum ist durch zahlreiche in der Literatur beschriebene Untersuchungen belegt (vgl. z.B. Warburg, 1956; Semenza, 2007).

### B-1. HIF-Luciferase-Assay:

HCT 116-Zellen wurden mit einem Plasmid stabil transfiziert, das einen Luciferase-Reporter unter der Kontrolle einer HIF-responsiven Sequenz enthielt. Diese Zellen wurden in Mikrotiterplatten ausgesät [20.000 Zellen/Kavität in RPMI 1640-Medium mit 10% fötalem Kälberserum (FKS) und 100 µg/ml Hygromycin]. Es wurde über Nacht unter Standardbedingungen inkubiert (5% CO₂, 21% O₂, 37°C, befeuchtet). Am anderen Morgen wurden die Zellen mit unterschiedlichen Konzentrationen der Testsubstanzen (0-10 µmol/L) in einer Hypoxiekammer (1% O₂) inkubiert. Nach 24 h wurde Bright Glo-Reagenz (Fa. Promega, Wisconsin, USA) entsprechend den Vorschriften des Herstellers zugefügt, und nach 5 min wurde die Lumineszenz gemessen. Zellen, die unter Normoxie inkubiert wurden, dienten als Hintergrundkontrollen.

In der folgenden Tabelle sind für repräsentative Ausführungsbeispiele die IC₅₀-Werte aus diesem Assay aufgeführt:

| **Beispiel Nr.** | **IC₅₀ [nmol/L]** |
|---|---|
| 1 | 0.7 |
| 2 | 0.6 |
| 3 | 0.6 |
| 8 | 0.6 |
| 9 | 0.5 |
| 10 | 0.4 |
| 11 | 0.4 |
| 12 | 0.3 |
| 13 | 0.3 |
| 14 | 0.5 |

Die als Example 1 in WO 2008/141731-A2 beschriebene Verbindung 5-[5-Methyl-1-(4-methylbenzyl)-1*H*-pyrazol-3-yl]-3-[4-(trifluormethoxy)phenyl]-1,2,4-oxadiazol, welche im Unterschied zu den erfindungsgemäßen Verbindungen keinen Heterocyclyl-Substituenten an der Benzyl-Kopfgruppe aufweist, zeigt in diesem Assay einen IC₅₀-Wert von 30 nmol/L.

### B-2. Suppression von HIF-Target-Genen in vitro:

Humane Bronchialkarzinom-Zellen (H460 und A549) wurden unter normoxischen Bedingungen sowie unter 1% Sauerstoffpartialdruck (siehe HIF-Luciferase-Assay) für 16 h mit variablen Konzentrationen der Testsubstanzen inkubiert (1 nM bis 10 µM). Aus den Zellen wurde die Gesamt-RNA isoliert, in cDNA umgeschrieben und in der Echtzeit-PCR die mRNA-Expression von HIF-Target-Genen analysiert. Bereits unter normoxischen Bedingungen, vor allem aber unter hypoxischen Bedingungen, erniedrigen aktive Testsubstanzen die mRNA-Expression der HIF-Target-Gene verglichen mit unbehandelten Zellen.

### B-3. Humane Xenograft-Tumormodelle:

Humane Tumor-Xenograftmodelle in immundefizienten Mäusen wurden zur Substanzbewertung herangezogen. Dazu wurden Tumorzellen *in vitro* kultiviert und subkutan implantiert, oder es wurden Tumor-Xenotransplantatstückchen subkutan weitertransplantiert. Die Behandlung der Tiere erfolgte durch orale, subkutane oder intraperitoneale Therapie nach der Etablierung des Tumors. Die Wirksamkeit von Testsubstanzen wurde in Monotherapie und in Kombinationstherapie mit anderen pharmakologischen Wirksubstanzen analysiert. Außerdem wurde die tumorinhibitorische Potenz von Testsubstanzen an Tumoren fortgeschrittener Größe (ca. 100 mm²) charakterisiert. Der Gesundheitszustand der Tiere wurde täglich überprüft, und die Behandlungen erfolgten entsprechend den Tierschutzbestimmungen. Die Tumorfläche wurde mit Schublehren gemessen (Länge L, Breite B = kleinere Ausdehnung). Das Tumorvolumen wurde nach der Formel (L x B²)/2 b erechnet. Die Hemmung des Tumorwachstums wurde am Ende des Versuches als T/C-Verhältnis der Tumorflächen bzw. Tumorgewichte und als TGI-Wert (tumor growth inhibition, berechnet nach der Formel [1-(T/C)] x 100) bestimmt (T = Tumorgröße der behandelten Gruppe; C = Tumorgröße der unbehandelten Kontrollgruppe).

Der Einfluss von Testsubstanzen auf die Tumor-Gefäßarchitektur und den Blutfluss innerhalb des Tumors wurde mit Hilfe von Mikro-Computertomographie- und Mikro-Ultraschall-Untersuchungen anhand von behandelten und unbehandelten tumortragenden Mäusen identifiziert.

### C. Bestimmung der pharmakokinetischen Kenngrößen

Die pharmakokinetischen Kenngrößen der erfindungsgemäßen Verbindungen nach intravenöser bzw. peroraler Gabe können wie folgt bestimmt werden:

Die zu untersuchende Substanz wurde Tieren (z.B. Mäusen oder Ratten) intravenös als Lösung appliziert (z.B. in entsprechendem Plasma mit geringem DMSO-Zusatz oder in einem PEG/ Ethanol/Wasser-Gemisch), die perorale Applikation erfolgte als Lösung (z.B. in Solutol/Ethanol/ Wasser- oder PEG/Ethanol/Wasser-Gemischen) oder als Suspension (z.B. in Tylose) jeweils über eine Schlundsonde. Nach Substanzgabe wurde den Tieren zu festgelegten Zeitpunkten Blut entnommen. Dieses wurde heparinisiert, anschließend wurde daraus durch Zentrifugation Plasma gewonnen. Die Substanz wurde im Plasma über LC-MS/MS analytisch quantifiziert. Aus den so ermittelten Plasmakonzentration-Zeit-Verläufen wurden unter Verwendung eines internen Standards und mit Hilfe eines validierten Rechenprogramms die pharmakokinetischen Kenngrößen berechnet, wie AUC (Fläche unter der Konzentration-Zeit-Kurve), Cₘₐₓ (maximale Plasmakonzentration), t_{1/2} (Halbwertszeit), V_{SS} (Verteilungsvolumen) und CL (Clearance) sowie die absolute und die relative Bioverfügbarkeit F bzw. Fᵣₑₗ (i.v./p.o.-Vergleich bzw. Vergleich von Suspension zu Lösung nach p.o.-Gabe).

Zur Bestimmung der Wirkstoff-Freisetzung aus einer Prodrug-Verbindung wurde das Prodrug entweder intravenös oder peroral, wie oben beschrieben, appliziert und im aufbereiteten Plasma die Konzentrationen sowohl des Prodrugs als auch des freigesetzten Wirkstoffs quantifiziert.

### C-1. Pharmakokinetische Kenngrößen nach intravenöser Applikation in der Ratte:

Die zu untersuchende Substanz wurde Ratten intravenös jeweils in Mengen zwischen 0.3 und 1.0 mg/kg als Lösung in Plasma, das bis zu 2% DMSO enthielt, appliziert. Exemplarisch für die erfindungsgemäßen Verbindungen sind nachfolgend die für die Ausführungsbeispiele 1 und 14 ermittelten kinetischen Kenngrößen dargestellt [CLₚₗₐₛₘₐ = Plasma-Clearance]:

| **Beispiel Nr.** | **t_{1/2} [h]** | **CLₚₗₐₛₘₐ [L/h/kg]** | **V_{SS} [L/kg]** |
|---|---|---|---|
| 1 | 6.8 | 1.6 | 12 |
| 14 | 8.2 | 0.72 | 7.8 |

Die als Example 1 in WO 2008/141731-A2 beschriebene Verbindung 5-[5-Methyl-1-(4-methylbenzyl)-1*H*-pyrazol-3-yl]-3-[4-(trifluormethoxy)phenyl]-1,2,4-oxadiazol, welche im Unterschied zu den erfindungsgemäßen Verbindungen keinen Heterocyclyl-Substituenten an der Benzyl-Kopfgruppe aufweist, zeigt nach intravenöser Applikation in der Ratte bezüglich dieser Parameter die folgenden Daten: t_{1/2} = 30 h, CLₚₗₐₛₘₐ = 0.4 L/h/kg, V_{SS} = 6.9 L/kg.

### C-2. Pharmakokinetische Kenngrößen nach peroraler Applikation in der Ratte:

Die zu untersuchende Substanz wurde Ratten peroral jeweils in Mengen zwischen 1 und 3 mg/kg als Lösung in Solutol/Ethanol/Wasser (40:10:50 oder 40:20:40) appliziert. Exemplarisch für die erfindungsgemäßen Verbindungen sind nachfolgend die für das Ausführungsbeispiel 1 ermittelten kinetischen Kenngrößen dargestellt [AUCₙₒᵣₘ = Dosis-normierte Exposition (Fläche unter der Konzentration-Zeit-Kurve)]:

| **Beispiel Nr.** | **t_{1/2} [h]** | **AUCₙₒᵣₘ [kg-h/L]** | **F [%]** |
|---|---|---|---|
| 1 | 12 | 0.43 | 68 |
| 1 aus 15 | 6 | 0.35 | 51 |

Die zweite Zeile in dieser Tabelle gibt die pharmakokinetischen Kenngrößen für das Beispiel 1 an, wie sie nach peroraler Applikation der Prodrug-Verbindung aus Beispiel 15 (1 mg/kg kristalline Substanz als Tylose-Suspension in Wasser) erhalten wurden. Die Prodrug-Verbindung selbst war zu keinem der untersuchten Zeitpunkte im Plasma der Ratten nachweisbar.

Die als Example 1 in WO 2008/141731-A2 beschriebene Verbindung 5-[5-Methyl-1-(4-methylbenzyl)-1*H*-pyrazol-3-yl]-3-[4-(trifluormethoxy)phenyl]-1,2,4-oxadiazol, welche im Unterschied zu den erfindungsgemäßen Verbindungen keinen Heterocyclyl-Substituenten an der Benzyl-Kopfgruppe aufweist, zeigt nach peroraler Applikation in der Ratte bezüglich dieser Parameter die folgenden Daten: t_{1/2} = 29 h, AUCₙₒᵣₘ = 1.9 kg·h/L, F = 74%.

### D. Bestimmung des Stabilitätsverhaltens

Die Stabilität der erfindungsgemäßen Prodrug-Verbindungen gegenüber einer unspezifischen Hydrolyse sowie in Plasma kann in den nachfolgend beschriebenen Tests bestimmt werden:

### D-1. Bestimmung der pH-abhängigen Hydrolysestabilität:

0.3 mg der Prüfsubstanz (Prodrug) wurden in einem 2 ml-HPLC-Gläschen eingewogen und mit 0.6 ml Acetonitril bzw. Acetonitril/DMSO-Gemisch (mit bis zu 20 Volumenprozent DMSO) versetzt. Zum Lösen der Substanz wurde das Probengefäß für ca. 10 Sekunden in ein Ultraschallbad gegeben. Anschließend wurden 1.0 ml der jeweiligen wässrigen Pufferlösung zugefügt (kommerziell erhältliche Pufferlösungen der pH-Werte 2, 4, 6.5, 8 und 10) und die Probe erneut im Ultraschallbad behandelt. Über einen Zeitraum von 24 Stunden bei 37°C wurden stündlich jeweils 5 µl der Probenlösung per HPLC auf ihren Gehalt an unverändertem Prodrug bzw. daraus durch Hydrolyse freigesetztem Wirkstoff analysiert. Quantifiziert wurde über die Flächenprozente der entsprechenden HPLC-Peaks.

Nachfolgend sind für das Ausführungsbeispiel 15 die Stabilitätswerte bei pH 6.5 aufgeführt:

| **Zeit [h]** | **% Prodrug Beispiel 15** | **% Wirkstoff Beispiel 1** |
|---|---|---|
| 0 | 94 | 3 |
| 2 | 91 | 7 |
| 4 | 87 | 10 |
| 6 | 84 | 13 |
| 12 | 73 | 22 |
| 24 | 56 | 35 |

In Abhängigkeit vom pH-Wert ergaben sich für das Ausführungsbeispiel 15 nach 12 h die folgenden Werte:

| **pH-Wert** | **% Prodrug Beispiel 15** | **% Wirkstoff Beispiel 1** |
|---|---|---|
| 2 | 89 | 6 |
| 4 | 89 | 9 |
| 6.5 | 73 | 22 |
| 8 | 64 | 30 |
| 10 | 0 | 78 |

### D-2. Bestimmung der Plasma-Stabilität in vitro:

1 mg der Prüfsubstanz (Prodrug) wurde in einem 2 ml-HPLC-Gläschen eingewogen und mit 1.5 ml DMSO und 1 ml Wasser versetzt. Zum Lösen der Substanz wurde das Probengefäß für ca. 10 Sekunden in ein Ultraschallbad gestellt. Zu 0.5 ml dieser Lösung wurden 0.5 ml 37°C warmes Ratten- bzw. Humanplasma gegeben. Die Probe wurde geschüttelt und für eine erste Analyse ca. 10 µl entnommen (Zeitpunkt t₀). Im Zeitraum bis zu 2 Stunden nach Inkubationsbeginn wurden 4-6 weitere Aliquote zur Quantifizierung entnommen. Die Probe wurde über die Prüfzeit bei 37°C gehalten. Charakterisierung und Quantifizierung erfolgten per HPLC.

Nachfolgend sind für das Ausführungsbeispiel 15 die Stabilitätswerte in Rattenplasma aufgeführt:

| **Zeit [h]** | **% Prodrug Beispiel 15** | **% Wirkstoff Beispiel 1** |
|---|---|---|
| 0 | 95 | 5 |
| 0.5 | 87 | 11 |
| 1 | 80 | 14 |
| 1.5 | 72 | 19 |
| 2 | 69 | 20 |
| 4 | 52 | 32 |

In Humanplasma ergaben sich für das Ausführungsbeispiel 15 die folgenden Werte:

| **Zeit [h]** | **% Prodrug Beispiel 15** | **% Wirkstoff Beispiel 1** |
|---|---|---|
| 0 | 97 | 3 |
| 0.5 | 96 | 6 |
| 1 | 93 | 7 |
| 1.5 | 91 | 9 |
| 2 | 89 | 11 |
| 4 | 81 | 18 |

### E. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

### F. Literaturangaben

- Globocan 2002 Report
   IARC International Agency for Research on Cancer: *Globocan* **2002,**
   http://www-dep.iarc.fr/globocan/downloads.htm
- American Cancer Society, Cancer Facts and Figures 2005
   American Cancer Society: *Cancer Facts and Figures* **2007,**
   http://www.cancer.org/docroot/STT/content/STT_1x_Cancer_Facts_Figures_2007.asp
- Gibbs JB, 2000
   Gibbs JB: Mechanism-based target identification and drug discovery in cancer research, Science 2000, 287 (5460), 1969-1973.
- Semenza und Wang, 1992
   Semenza GL, Wang GL: A nuclear factor induced by hypoxia via de novo protein synthesis binds to the human erythropoietin gene enhancer at a site required for transcriptional activation, Mol. Cell. Biol. 1992, 12 (12), 5447-5454.
- Wang und Semenza, 1995
   Wang GL, Semenza GL: Purification and characterization of hypoxia-inducible factor 1, J. Biol. Chem. 1995, 270 (3), 1230-1237.
- Wang, Jiang *et al.,* 1995
   Wang GL, Jiang BH, Rue EA, Semenza GL: Hypoxia-inducible factor 1 is a basic-helix-loop-helix-PAS heterodimer regulated by cellular O2 tension, PNAS 1995, 92 (12), 5510-5514.
- Jiang, Rue *et al.,* 1996
   Jiang BH, Rue E, Wang GL, Roe R, Semenza GL: Dimerization, DNA binding, and transactivation properties ofhypoxia-inducible factor 1, J. Biol. Chem. 1996, 271 (30), 17771-17778.
- Makino, Cao *et al.,* 2001
   Makino Y, Cao R, Svensson K, Bertilsson G, Asman M, Tanaka H, Cao Y, Poellinger L: Nature 2001, 414 (6863), 550-554.
- Jiang, Semenza *et al.,* 1996
   Jiang BH, Semenza GL, Bauer C, Marti HH: Hypoxia-inducible factor 1 levels vary exponentially over a physiologically relevant range of O2 tension, Am. J. Physiol. 1996, 271, 1172-1180.
- Maxwell, Wiesener *et al.,* 1999
   Maxwell PH, Wiesener MS, Chang GW, Clifford SC, Vaux EC, Cockman ME, Wykoff CC, Ratcliffe PJ: The tumour suppressor protein VHL targets hypoxia-inducible factors for oxygendependent proteolysis, Nature 1999, 399 (6733), 271-275.
- Hirota und Semenza, 2006
   Hirota K, Semenza GL: Regulation of angiogenesis by hypoxia-inducible factor 1, Crit. Rev. Oncol. Hematol. 2006, 59 (1), 15-26.
- Chen, Zhao *et al.,* 2003
   Chen J, Zhao S, Nakada K, Kuge Y, Tamaki N, Okada F, Wang J, Shindo M, Higashino F, Takeda K, Asaka M, Katoh H, Sugiyama T, Hosokawa M, Kobayashi M: Dominant-negative hypoxia-inducible factor-1 alpha reduces tumorigenicity of pancreatic cancer cells through the suppression of glucose metabolism, Am. J. Pathol. 2003, 162 (4), 1283-1291.
- Stoeltzing, McCarty *et al.,* 2004
   Stoeltzing O, McCarty MF, Wey JS, Fan F, Liu W, Belcheva A, Bucana CD, Semenza GL, Ellis LM: Role of hypoxia-inducible factor-1 alpha in gastric cancer cell growth, angiogenesis, and vessel maturation, J. Natl. Cancer Inst. 2004, 96 (12), 946-956.
- Li, Lin *et al.,* 2005
   Li L, Lin X, Staver M, Shoemaker A, Semizarov D, Fesik SW, Shen Y: Evaluating hypoxia-inducible factor-1 alpha as a cancer therapeutic target via inducible RNA interference in vivo, Cancer Res. 2005, 65 (16), 7249-7258.
- Mizukami, Jo *et al.,* 2005
   Mizukami Y, Jo WS, Duerr EM, Gala M, Li J, Zhang X, Zimmer MA, Iliopoulos O, Zukerberg LR, Kohgo Y, Lynch MP, Rueda BR, Chung DC: Induction of interleukin-8 preserves the angiogenic response in HIF-1alpha-deficient colon cancer cells, Nat. Med. 2005, 11 (9), 992-997.
- Li, Shi *et al.,* 2006
   Li J, Shi M, Cao Y, Yuan W, Pang T, Li B, Sun Z, Chen L, Zhao RC: Knockdown of hypoxia-inducible factor-1alpha in breast carcinoma MCF-7 cells results in reduced tumor growth and increased sensitivity to methotrexate, Biochem. Biophys. Res. Commun. 2006, 342, 1341-1351.
- Semenza, 2007
   Semenza GL: Drug Discov. Today 2007, 12 (19-20), 853-859*.*
- Weidemann und Johnson, 2008
   Weidemann A, Johnson RS: Cell Death and Differentiation 2008, 15, 621-627.
- Aiello *et al.,* 1994
   Aiello et al.: New Engl. J. Med. 1994, 331, 1480.
- Peer *et al.,* 1995
   Peer et al.: Lab. Invest. 1995, 72, 638.
- Lopez *et al.,* 1996
   Lopez et al.: Invest. Ophthalmol. Vis. Sci. 1996, 37, 855.
- Warburg, 1956
   Warburg O: Science 1956, 123 (3191), 309-314.

## Patentansprüche

1. Verbindung der Formel (I) 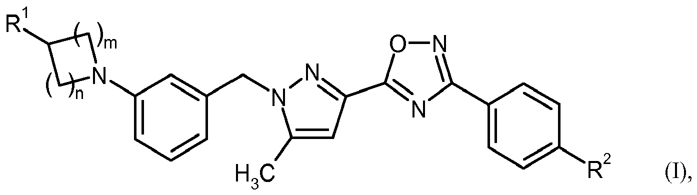 in welcher
m für die Zahl 1 oder 2 steht,
n für die Zahl 1, 2 oder 3 steht,
R¹ für Hydroxy oder Cyano steht,
und
R² für Trifluormethoxy, Trifluormethylsulfanyl, Trifluormethylsulfonyl, Pentafluorsulfanyl oder eine Gruppe der Formel 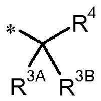 steht, worin
* die Verknüpfungsstelle mit dem Phenyl-Ring kennzeichnet,
R^{3A} und R^{3B} unabhängig voneinander Fluor oder Methyl bedeuten
oder
miteinander verknüpft sind und gemeinsam mit dem Kohlenstoffatom, an das sie gebunden sind, einen Cyclopropan-1,1-diyl-, Cyclobutan-1,1-diyl-, Cyclopentan-1,1-diyl-, Cyclohexan-1,1-diyl-, Oxetan-3,3-diyl- oder Tetra-hydro-2*H*-pyran-4,4-diyl-Ring bilden,
und
R⁴ Wasserstoff, Fluor, Methyl, Trifluormethyl oder Methoxy bedeutet,
sowie ihre Salze, Solvate und Solvate der Salze.

2. Verbindung der Formel (I) nach Anspruch 1, in welcher m und n unabhängig voneinander für die Zahl 1 oder 2 stehen,
R¹ für Hydroxy oder Cyano steht,
und
R² für Trifluormethyl, Trifluormethoxy, Trifluormethylsulfanyl oder eine Gruppe der Formel 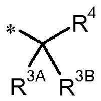 steht, worin
* die Verknüpfungsstelle mit dem Phenyl-Ring kennzeichnet,
R^{3A} und R^{3B} beide Methyl bedeuten oder miteinander verknüpft sind und gemeinsam mit dem Kohlenstoffatom, an das sie gebunden sind, einen Cyclopropan-1,1-diyl-, Cyclobutan-1,1-diyl-, Oxetan-3,3-diyl- oder Tetrahydro-2*H-*pyran-4,4-diyl-Ring bilden,
und
R⁴ Wasserstoff, Fluor, Methyl oder Trifluormethyl bedeutet,
sowie ihre Salze, Solvate und Solvate der Salze.

3. Verbindung der Formel (I) nach Anspruch 1 oder 2, in welcher m und n beide für die Zahl 1 oder die Zahl 2 stehen,
R¹ für Hydroxy oder Cyano steht,
und
R² für Trifluormethoxy oder eine Gruppe der Formel 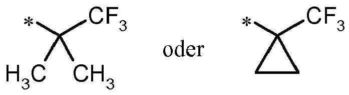 steht, worin
* die Verknüpfungsstelle mit dem Phenyl-Ring kennzeichnet,
sowie ihre Salze, Solvate und Solvate der Salze.

4. Verbindung der Formel (I) nach Anspruch 1, 2 oder 3, in welcher
R¹ für Hydroxy steht
und
m, n und R² jeweils die in Anspruch 1, 2 oder 3 angegebenen Bedeutungen haben, sowie ihre Salze, Solvate und Solvate der Salze.

5. Verbindung der Formel (I-PD) 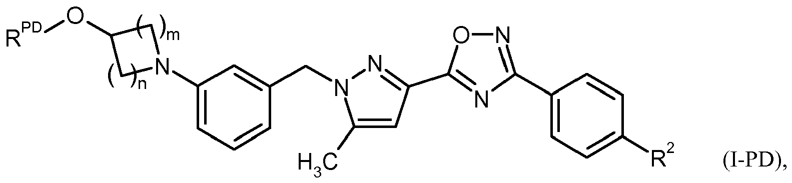 in welcher m, n und R² jeweils die in einem der Ansprüche 1 bis 4 aufgeführten Bedeutungen haben
und
R^{PD} für eine Prodrug-Gruppe der Formel 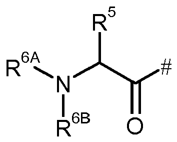 steht, worin
# die Verknüpfungsstelle mit dem Sauerstoffatom kennzeichnet,
R⁵ Wasserstoff oder (C₁-C₄)-Alkyl bedeutet,
und
R^{6A} und R^{6B} unabhängig voneinander Wasserstoff oder Methyl bedeuten,
sowie ihre Salze, Solvate und Solvate der Salze.

6. Verbindung der Formel (I-PD) nach Anspruch 5, in welcher
R^{PD} für eine Prodrug-Gruppe der Formel 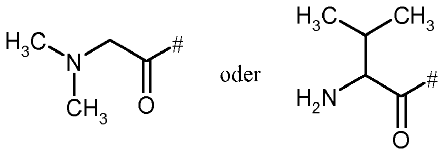 steht, worin
# die Verknüpfungsstelle mit dem Sauerstoffatom kennzeichnet,
sowie ihre Salze, Solvate und Solvate der Salze.

7. Verfahren zur Herstellung von Verbindungen der Formel (I), wie in den Ansprüchen 1 bis 4 definiert, **dadurch gekennzeichnet, dass** man eine Verbindung der Formel (II) 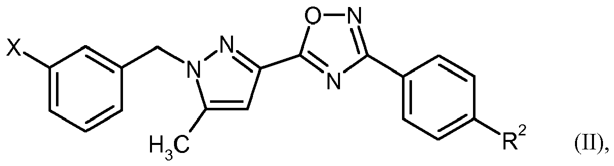 in welcher R² die in den Ansprüchen 1 bis 4 angegebene Bedeutung hat und
X für Brom oder Iod steht,
in Gegenwart eines geeigneten Palladium-Katalysators und einer Base mit einer Verbindung der Formel (III) 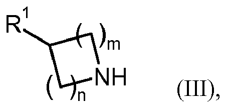 in welcher m, n und R¹ die in den Ansprüchen 1 bis 4 angegebenen Bedeutungen haben, kuppelt
und die so erhaltenen Verbindungen der Formel (I) gegebenenfalls in ihre Enantiomere und/oder Diastereomere trennt und/oder mit den entsprechenden (i) Lösungsmitteln und/ oder (ii) Säuren in ihre Solvate, Salze und/oder Solvate der Salze überführt.

8. Verfahren zur Herstellung von Verbindungen der Formel (I-PD), wie in den Ansprüchen 5 und 6 definiert, **dadurch gekennzeichnet, dass** man eine Verbindung der Formel (I-A) 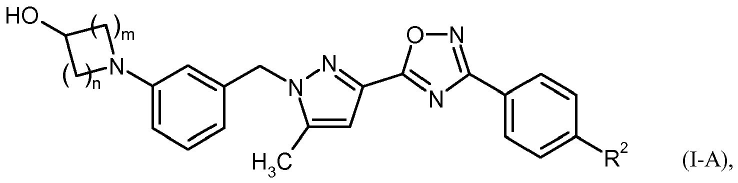 in welcher m, n und R² jeweils die in einem der Ansprüche 1 bis 4 aufgeführten Bedeutungen haben,
nach üblichen Methoden mit einer Verbindung der Formel (VIII)
R^{PD}-OH (VIII)
oder einer aktivierten Form dieser Verbindung, in welcher R^{PD} die in Anspruch 5 oder 6 angegebene Bedeutung hat,
verestert
und die so erhaltenen Verbindungen der Formel (I-PD) gegebenenfalls in ihre Enantiomere und/oder Diastereomere trennt und/oder mit den entsprechenden (i) Lösungsmitteln und/ oder (ii) Säuren in ihre Solvate, Salze und/oder Solvate der Salze überführt.

9. Verbindung, wie in einem der Ansprüche 1 bis 6 definiert, zur Behandlung und/oder Prävention von Krankheiten.

10. Verbindung, wie in einem der Ansprüche 1 bis 6 definiert, zur Verwendung in einem Verfahren zur Behandlung und/oder Prävention von Krebs- oder Tumorerkrankungen.

11. Verbindung, wie in einem der Ansprüche 1 bis 6 definiert, zur Verwendung in einem Verfahren zur Behandlung und/oder Prävention von ischämischen Herz-Kreislauf-Erkrankungen, Herzinsuffizienz, Herzinfarkt, Arrhythmie, Schlaganfall, pulmonaler Hypertonie, fibrotischen Erkrankungen von Niere und Lunge, Psoriasis, diabetischer Retinopathie, Makuladegeneration, rheumatischer Arthritis und der Chugwash-Polyzythämie.

12. Verwendung einer Verbindung, wie in einem der Ansprüche 1 bis 6 definiert, zur Herstellung eines Arzneimittels zur Behandlung und/oder Prävention von Krebs- oder Tumorerkrankungen.

13. Verwendung einer Verbindung, wie in einem der Ansprüche 1 bis 6 definiert, zur Herstellung eines Arzneimittels zur Behandlung und/oder Prävention von ischämischen Herz-Kreislauf-Erkrankungen, Herzinsuffizienz, Herzinfarkt, Arrhythmie, Schlaganfall, pulmonaler Hypertonie, fibrotischen Erkrankungen von Niere und Lunge, Psoriasis, diabetischer Retinopathie, Makuladegeneration, rheumatischer Arthritis und der Chugwash-Polyzyth-amie.

14. Arzneimittel enthaltend eine Verbindung, wie in einem der Ansprüche 1 bis 6 definiert, in Kombination mit einem oder mehreren inerten, nicht-toxischen, pharmazeutisch geeigneten Hilfsstoffen.

15. Arzneimittel enthaltend eine Verbindung, wie in einem der Ansprüche 1 bis 6 definiert, in Kombination mit einem oder mehreren weiteren Wirkstoffen.

16. Arzneimittel nach Anspruch 14 oder 15 zur Behandlung und/oder Prävention von Krebsoder Tumorerkrankungen.

17. Arzneimittel nach Anspruch 14 oder 15 zur Behandlung und/oder Prävention von ischämischen Herz-Kreislauf-Erkrankungen, Herzinsuffizienz, Herzinfarkt, Arrhythmie, Schlaganfall, pulmonaler Hypertonie, fibrotischen Erkrankungen von Niere und Lunge, Psoriasis, diabetischer Retinopathie, Makuladegeneration, rheumatischer Arthritis und der Chugwash-Polyzythämie.

## Claims

1. Compound of the formula (I) 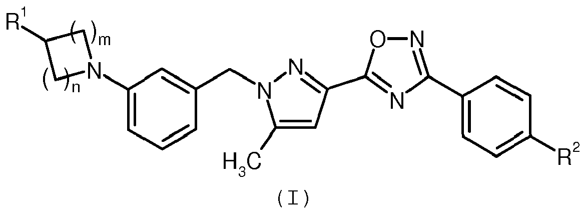 in which
m is 1 or 2,
n is 1, 2 or 3,
R¹ is hydroxyl or cyano,
and
R² is trifluoromethoxy, trifluoromethylsulphanyl, trifluoromethylsulphonyl, pentafluorosulphanyl or a group of the formula 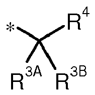 in which
* denotes the bonding site to the phenyl ring,
R^{3A} and R^{3B} are each independently fluorine or methyl
or
are joined to one another and, together with the carbon atom to which they are bonded, form a cyclopropane-1,1-diyl, cyclobutane-1,1-diyl, cyclopentane-1,1-diyl, cyclohexane-1,1-diyl, oxetane-3,3-diyl or tetrahydro-2*H*-pyran-4,4-diyl ring
and
R⁴ is hydrogen, fluorine, methyl, trifluoromethyl or methoxy,
and the salts, solvates and solvates of the salts thereof.

2. Compound of the formula (I) according to Claim 1, in which
m and n are each independently 1 or 2,
R¹ is hydroxyl or cyano,
and
R² is trifluoromethyl, trifluoromethoxy, trifluoromethylsulphanyl or a group of the formula 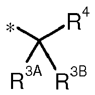 in which
* denotes the bonding site to the phenyl ring,
R^{3A} and R^{3B} are both methyl or are joined to one another and, together with the carbon atom to which they are bonded, form a cyclopropane-1,1-diyl, cyclobutane-1,1-diyl, oxetane-3,3-diyl or tetrahydro-2*H*-pyran-4,4-diyl ring
and
R⁴ is hydrogen, fluorine, methyl or trifluoromethyl,
and the salts, solvates and solvates of the salts thereof.

3. Compound of the formula (I) according to Claim 1 or 2, in which
m and n are both 1 or 2,
R¹ is hydroxyl or cyano,
and
R² is trifluoromethoxy or a group of the formula 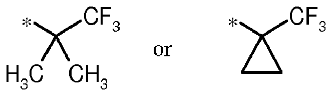 in which
* denotes the bonding site to the phenyl ring,
and the salts, solvates and solvates of the salts thereof.

4. Compound of the formula (I) according to Claim 1, 2 or 3, in which
R¹ is hydroxyl,
and
m, n and R² are each as defined in Claim 1, 2 or 3,
and the salts, solvates and solvates of the salts thereof.

5. Compound of the formula (I-PD) 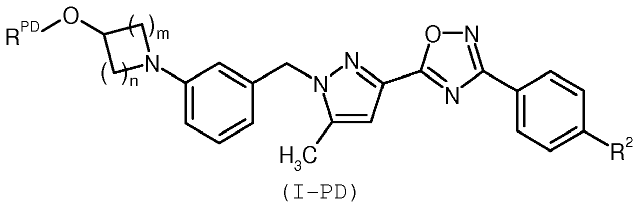 in which m, n and R² are each as defined in one of Claims 1 to 4
and
R^{PD} is a prodrug group of the formula 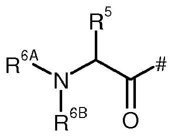 in which
# denotes the bonding site to the oxygen atom,
R⁵ is hydrogen or (C₁-C₄)-alkyl,
and
R^{6A} and R^{6B} are each independently hydrogen or methyl,
and the salts, solvates and solvates of the salts thereof.

6. Compound of the formula (I-PD) according to Claim 5, in which
R^{PD} is a prodrug group of the formula 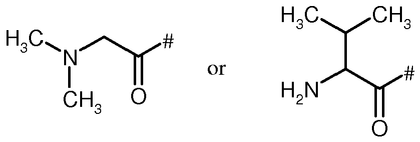 in which
# denotes the bonding site to the oxygen atom,
and the salts, solvates and solvates of the salts thereof.

7. Process for preparing compounds of the formula (I) as defined in Claims 1 to 4, **characterized in that** a compound of the formula (II) 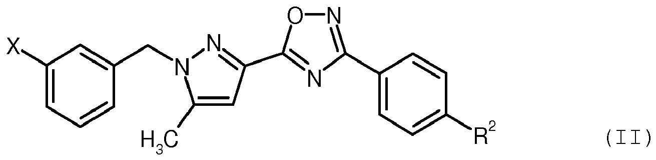
in which R² is as defined in Claims 1 to 4
and
X is bromine or iodine,
in the presence of a suitable palladium catalyst and of a base, is coupled with a compound of the formula (III) 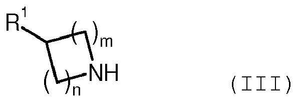
in which m, n and R¹ are each as defined in Claims 1 to 4,
and the resulting compounds of the formula (I) are optionally separated into the enantiomers and/or diastereomers thereof and/or converted using the appropriate (i) solvents and/or (ii) acids to the solvates, salts and/or solvates of the salts thereof.

8. Process for preparing compounds of the formula (I-PD) as defined in Claims 5 and 6, **characterized in that** a compound of the formula (I-A) 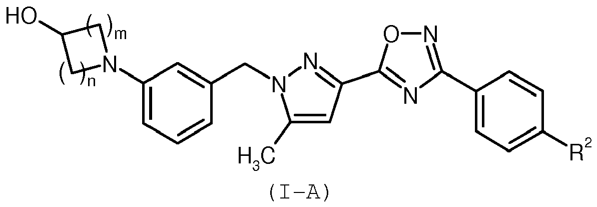
in which m, n and R² are each as defined in one of Claims 1 to 4
is esterified by customary methods with a compound of the formula (VIII)
R^{PD}-OH (VIII)
or an activated form of this compound in which R^{PD} is as defined in Claim 5 or 6,
and the resulting compounds of the formula (I-PD) are optionally separated into the enantiomers and/or diastereomers thereof and/or converted using the appropriate (i) solvents and/or (ii) acids to the solvates, salts and/or solvates of the salts thereof.

9. Compound as defined in any of Claims 1 to 6 for treatment and/or prevention of diseases.

10. Compound as defined in any of Claims 1 to 6 for use in a method for treatment and/or prevention of cancers or tumours.

11. Compound as defined in any of Claims 1 to 6, for use in a method for treatment and/or prevention of ischaemic cardiovascular diseases, heart failure, myocardial infarction, arrhythmia, stroke, pulmonary hypertension, fibrotic diseases of the kidney and lung, psoriasis, diabetic retinopathy, macular degeneration, rheumatic arthritis and Chuvash polycythaemia.

12. Use of a compound as defined in any of Claims 1 to 6 for production of a medicament for treatment and/or prevention of cancers or tumours.

13. Use of a compound as defined in any of Claims 1 to 6 for production of a medicament for treatment and/or prevention of ischaemic cardiovascular diseases, heart failure, myocardial infarction, arrhythmia, stroke, pulmonary hypertension, fibrotic diseases of the kidney and lung, psoriasis, diabetic retinopathy, macular degeneration, rheumatic arthritis and Chuvash polycythaemia.

14. Medicament comprising a compound as defined in any of Claims 1 to 6 in combination with one or more inert, nontoxic, pharmaceutically suitable excipients.

15. Medicament comprising a compound as defined in any of Claims 1 to 6 in combination with one or more further active ingredients.

16. Medicament according to Claim 14 or 15 for treatment and/or prevention of cancers or tumours.

17. Medicament according to Claim 14 or 15 for treatment and/or prevention of ischaemic cardiovascular diseases, heart failure, myocardial infarction, arrhythmia, stroke, pulmonary hypertension, fibrotic diseases of the kidney and lung, psoriasis, diabetic retinopathy, macular degeneration, rheumatic arthritis and Chuvash polycythaemia.

## Revendications

1. Composé de formule (I) 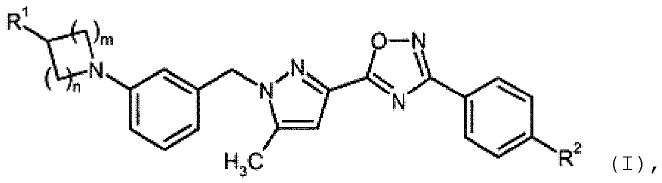 dans laquelle
m représente le nombre 1 ou 2,
n représente le nombre 1, 2 ou 3,
R¹ représente le groupe hydroxy ou cyano,
et
R² représente le groupe trifluorométhoxy, trifluorométhylsulfanyle, trifluorométhyl-sulfonyle, pentafluorosulfanyle ou un groupe de formule 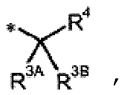 où
* désigne le point de liaison au cycle phényle,
R^{3A} et R^{3B} représentent indépendamment l'un de l'autre un atome de fluor ou le groupe méthyle
ou
sont liés l'un à l'autre et forment ensemble, avec l'atome de carbone auquel ils sont liés, un cycle cyclopropane-1,1-diyle, cyclobutane-1,1-diyle, cyclopentane-1,1-diyle, cyclohexane-1,1-diyle, oxétane-3,3-diyle ou tétrahydro-2*H*-pyrane-4,4-diyle,
et
R⁴ représente un atome d'hydrogène ou de fluor, le groupe méthyle, trifluorométhyle ou méthoxy,
ainsi que ses sels, produits de solvatation et produits de solvatation des sels.

2. Composé de formule (I) selon la revendication 1, dans lequel
m et n représentent indépendamment l'un de l'autre le nombre 1 ou 2,
R¹ représente le groupe hydroxy ou cyano,
et
R² représente le groupe trifluorométhyle, trifluorométhoxy, trifluorométhylsulfanyle ou un groupe de formule 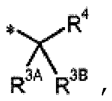 où
* désigne le point de liaison au cycle phényle,
R^{3A} et R^{3B} représentent l'un et l'autre le groupe méthyle ou sont liés l'un à l'autre et forment ensemble, avec l'atome de carbone auquel ils sont liés, un cycle cyclopropane-1,1-diyle, cyclobutane-1,1-diyle, oxétane-3,3-diyle ou tétrahydro-2*H*-pyrane-4,4-diyle,
et
R⁴ représente un atome d'hydrogène ou de fluor, le groupe méthyle ou trifluorométhyle,
ainsi que ses sels, produits de solvatation et produits de solvatation des sels.

3. Composé de formule (I) selon la revendication 1 ou 2, dans lequel
m et n représentent l'un et l'autre le nombre 1 ou le nombre 2
R¹ représente le groupe hydroxy ou cyano,
et
R² représente le groupe trifluorométhoxy ou un groupe de formule 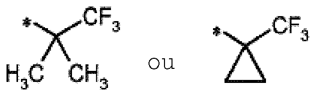 où
* désigne le point de liaison au cycle phényle,
ainsi que ses sels, produits de solvatation et produits de solvatation des sels.

4. Composé de formule (I) selon la revendication 1, 2 ou 3, dans lequel
R¹ représente le groupe hydroxy,
et
m, n et R² ont chacun les significations indiquées dans la revendication 1, 2 ou 3,
ainsi que ses sels, produits de solvatation et produits de solvatation des sels.

5. Composé de formule (I-PD) 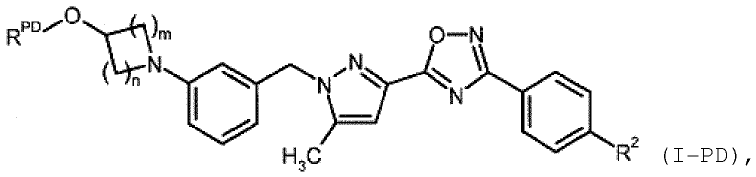 dans laquelle m, n et R² ont chacun les significations indiquées dans l'une quelconque des revendications 1 à 4
et
R^{PD} représente un groupe précurseur de médicament de formule 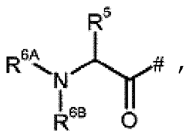 où
# désigne le point de liaison à l'atome d'oxygène,
R⁵ représente un atome d'hydrogène ou un groupe alkyle en C₁-C₄,
et
R^{6A} et R^{6B} représentent indépendamment l'un de l'autre un atome d'hydrogène ou le groupe méthyle,
ainsi que ses sels, produits de solvatation et produits de solvatation des sels.

6. Composé de formule (I-PD) selon la revendication 5, dans lequel
R^{PD} représente un groupe précurseur de médicament de formule 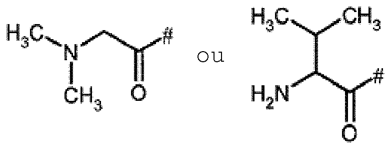 où
# désigne le point de liaison à l'atome d'oxygène,
ainsi que ses sels, produits de solvatation et produits de solvatation des sels.

7. Procédé pour la préparation des composés de formule (I), tels que définis dans les revendications 1 à 4, **caractérisé en ce qu'**on combine un composé de formule (II) 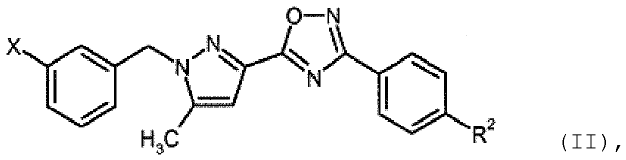 dans laquelle R² a la signification indiquée dans les revendications 1 à 4
et
X représente un atome de brome ou d'iode,
en présence d'un catalyseur au palladium approprié et d'une base, avec un composé de formule (III) 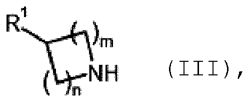 dans laquelle m, n et R¹ ont les significations indiquées dans les revendications 1 à 4,
et éventuellement on fractionne les composés de formule (I) ainsi obtenus en leurs énantiomères et/ou diastéréoisomères et/ou les convertit, à l'aide (i) des solvants et/ou (ii) acides correspondants en leurs produits de solvatation, sels et/ou produits de solvatation des sels.

8. Procédé pour la préparation des composés de formule (I-PD), tels que définis dans les revendications 5 et 6, **caractérisé en ce qu'**on estérifie un composé de formule (I-A) 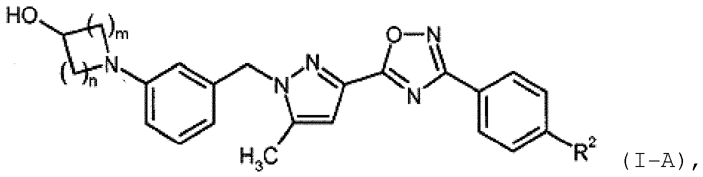 dans laquelle m, n et R² ont chacun les significations indiquées dans l'une quelconque des revendications 1 à 4,
selon des méthodes usuelles, avec un composé de formule (VIII)
R^{PD}-OH (VIII)
ou une forme activée de ce composé, formule dans laquelle R^{PD} a la signification indiquée dans la revendication 5 ou 6,
et éventuellement on fractionne les composés de formule (I-PD) ainsi obtenus en leurs énantiomères et/ou diastéréoisomères et/ou les convertit, à l'aide (i) des solvants et/ou (ii) acides correspondants en leurs produits de solvatation, sels et/ou produits de solvatation des sels.

9. Composé, tel que défini dans l'une quelconque des revendications 1 à 6, destiné au traitement et/ou à la prévention de maladies.

10. Composé, tel que défini dans l'une quelconque des revendications 1 à 6, destiné à l'utilisation dans un procédé pour le traitement et/ou la prévention de maladies cancéreuses ou tumorales.

11. Composé, tel que défini dans l'une quelconque des revendications 1 à 6, destiné à l'utilisation dans un procédé pour le traitement et/ou la prévention de maladies cardiovasculaires ischémiques, de l'insuffisance cardiaque, de l'infarctus du myocarde, de l'arythmie, de l'accident vasculaire cérébral, de l'hypertonie pulmonaire, de maladies fibreuses du rein et du poumon, du psoriasis, de la rétinopathie diabétique, de la dégénérescence maculaire, de la polyarthrite rhumatismale et de la polycythémie tchouvache.

12. Utilisation d'un composé, tel que défini dans l'une quelconque des revendications 1 à 6, pour la fabrication d'un médicament destiné au traitement et/ou à la prévention de maladies cancéreuses ou tumorales.

13. Utilisation d'un composé, tel que défini dans l'une quelconque des revendications 1 à 6, pour la fabrication d'un médicament destiné au traitement et/ou à la prévention de maladies cardiovasculaires ischémiques, de l'insuffisance cardiaque, de l'infarctus du myocarde, de l'arythmie, de l'accident vasculaire cérébral, de l'hypertonie pulmonaire, de maladies fibreuses du rein et du poumon, du psoriasis, de la rétinopathie diabétique, de la dégénérescence maculaire, de la polyarthrite rhumatismale et de la polycythémie tchouvache.

14. Médicament contenant un composé, tel que défini dans l'une quelconque des revendications 1 à 6, en association avec un ou plusieurs adjuvants inertes, non toxiques, pharmaceutiquement convenables.

15. Médicament contenant un composé, tel que défini dans l'une quelconque des revendications 1 à 6, en association avec une ou plusieurs autres substances actives.

16. Médicament selon la revendication 14 ou 15, destiné au traitement et/ou la prévention de maladies cancéreuses ou tumorales.

17. Médicament selon la revendication 14 ou 15, destiné au traitement et/ou la prévention de maladies cardiovasculaires ischémiques, de l'insuffisance cardiaque, de l'infarctus du myocarde, de l'arythmie, de l'accident vasculaire cérébral, de l'hypertonie pulmonaire, de maladies fibreuses du rein et du poumon, du psoriasis, de la rétinopathie diabétique, de la dégénérescence maculaire, de la polyarthrite rhumatismale et de la polycythémie tchouvache.
